(19) Europäisches Patentamt
European Patent Office
Office européen des brevets

(11) EP 1 149 575 A1

(12) **DEMANDE DE BREVET EUROPEEN**

(43) Date de publication:
**31.10.2001 Bulletin 2001/44**

(51) Int Cl.[7]: **A61K 7/13**

(21) Numéro de dépôt: **01400879.1**

(22) Date de dépôt: **05.04.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **18.04.2000 FR 0004991**

(71) Demandeur: **L'OREAL**
**75008 Paris (FR)**

(72) Inventeurs:
- **Kravtchenko, Sylvain**
**92600 Asnieres (FR)**
- **Lagrange, Alain**
**77700 Coupvray (FR)**

(74) Mandataire: **Miszputen, Laurent**
**L'OREAL-DPI**
**6 rue Bertrand Sincholle**
**92585 Clichy Cédex (FR)**

(54) **Composition de teinture d'oxydation des fibres kératiniques comprenant une 1-(4-aminophényl)-pyrrolidine et un colorant direct particulier**

(57) L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation choisie parmi les 1-(4-aminophényl)-pyrrolidines et leurs sels d'addition avec un acide, et au moins un colorant direct choisi parmi les colorants nitrés benzéniques et les colorants cationiques contenant un atome d'azote quaternisé et une liaison -Z=D- dans laquelle Z et D, identiques ou différents, désignent un atome d'azote ou un radical -CH- , ainsi que le procédé de teinture mettant en oeuvre cette composition.

EP 1 149 575 A1

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation choisie parmi les 1-(4-aminophényl)-pyrrolidines et leurs sels d'addition avec un acide, et au moins un colorant direct choisi parmi les colorants nitrés benzéniques et les colorants cationiques contenant un atome d'azote quaternisé et une liaison -Z=D- dans laquelle Z et D, identiques ou différents, désignent un atome d'azote ou un radical -CH-, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Ainsi, il est connu de longue date et d'un usage très répandu, de teindre les cheveux de façon permanente avec des produits de couplage de la paraphénylènediamine (PPD) en présence de peroxyde d'hydrogène. Cependant, de nouvelles bases d'oxydation mieux tolérées ont été recherchées et proposées comme alternatives à la PPD. Parmi elles, la base tertiaire N,N-bis(β-hydroxyéthyl)-paraphénylènediamine a été largement utilisée dans les produits de teinture capillaire du commerce.

Cependant, les colorations obtenues en mettant en oeuvre ces compositions ne sont pas toujours assez puissantes, chromatiques, ou résistantes aux différentes agressions que peuvent subir les cheveux.

Or, la Demanderesse vient maintenant de découvrir qu'il est possible d'obtenir de nouvelles teintures d'oxydation, capables de conduire à des colorations aux nuances variées, chromatiques, puissantes, esthétiques, peu sélectives, et résistant bien aux diverses agressions que peuvent subir les fibres, en associant au moins une base d'oxydation choisie parmi les 1-(4-aminophényl)-pyrrolidines et leurs sels d'addition avec un acide et au moins un colorant direct particulier.

Cette découverte est à la base de la présente invention.

L'invention a donc pour premier objet une composition pour la teinture d'oxydation des fibres kératiniques et en particulier des fibres kératiniques humaines telles que les cheveux, comprenant dans un milieu approprié pour la teinture, (i)au moins une 1-(4-aminophényl)-pyrrolidine de formule (I) suivante à titre de précurseur de colorant d'oxydation :

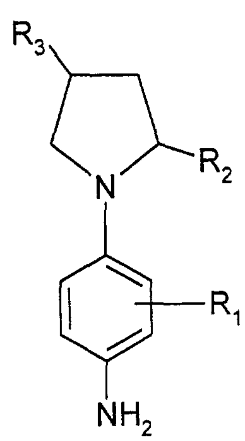

(I)

dans laquelle,

$R_1$ désigne un atome d'hydrogène, un radical alkyle en $C_1$-$C_6$, ou monohydroxyalkyle en $C_1$-$C_5$, ou polyhydroxyalkyle en $C_2$-$C_5$ ;
$R_2$ désigne un atome d'hydrogène, un radical -$CONH_2$, ou monohydroxyalkyle en $C_1$-$C_5$, ou polyhydroxyalkyle en $C_2$-$C_5$ ;
$R_3$ désigne un atome d'hydrogène, ou un radical OH ; et ses sels d'addition avec un acide,
**caractérisée** par le fait qu'elle comprend en outre au moins un colorant direct choisi dans le groupe formé par les colorants nitrés benzéniques et les colorants cationiques contenant un atome d'azote quaternisé et une liaison -Z=D- dans laquelle Z et D, identiques ou différents, désignent un atome d'azote ou un radical -CH-.

Les sels d'addition avec un acide des 1-(4-aminophényl)-pyrrolidines de formule (I) utilisables dans les compositions tinctoriales selon l'invention sont notamment choisis parmi les chlorhydrates, les bromhydrates, les sulfates, les tartrates, les lactates et les acétates.
La composition tinctoriale conforme à l'invention ainsi définie, conduit après mélange avec une composition oxydante, à des colorations dans des nuances variées, chromatiques, puissantes, esthétiques, présentant une faible sélectivité et d'excellentes propriétés de résistances à la fois vis-à-vis des agents atmosphériques tels que la lumière et les intempéries et vis-à-vis de la transpiration et des différents traitements que peuvent subir les cheveux.
Un autre objet de l'invention porte sur une composition prête à l'emploi pour la teinture d'oxydation des fibres kératiniques qui comprend, dans un milieu approprié pour la teinture, au moins une 1-(4-aminophényl)-pyrrolidine de formule (I), au moins un colorant direct choisi parmi les colorants nitrés benzéniques et les colorants cationiques contenant un atome d'azote quaternisé et une liaison -Z=D-dans laquelle Z et D, identiques ou différents, désignent un atome d'azote ou un radical -CH-, et au moins un agent oxydant.
Par composition prête à l'emploi, on entend au sens de la présente invention, toute composition destinée à être appliquée immédiatement sur les fibres kératiniques.
L'invention vise également un procédé de teinture d'oxydation des fibres kératiniques, et en particulier des fibres kératiniques humaines telles que les cheveux, consistant à appliquer sur les fibres une composition colorante contenant, dans un milieu approprié pour la teinture, au moins une 1-(4-aminophényl)-pyrrolidine de formule (I) en association avec au moins un colorant direct choisi parmi les colorants nitrés benzéniques et les colorants cationiques contenant un atome d'azote quaternisé et une liaison -Z=D- dans laquelle Z et D, identiques ou différents, désignent un atome d'azote ou un radical -CH-, la couleur étant révélée à pH alcalin, neutre ou acide, à l'aide d'une composition contenant au moins un agent oxydant, qui est mélangée juste au moment de l'emploi à la composition colorante ou qui est appliquée séquentiellement sans rinçage intermédiaire.
L'invention a également pour objet un dispositif de teinture à plusieurs compartiments ou " kit " pour la teinture d'oxydation des fibres kératiniques, en particulier des fibres kératiniques humaines telles que les cheveux, qui comporte au moins un compartiment contenant au moins une 1-(4-aminophényl)-pyrrolidine de formule (I), au moins un colorant direct choisi parmi les colorants nitrés benzéniques et les colorants cationiques contenant un atome d'azote quaternisé et une liaison -Z=D- dans laquelle Z et D, identiques ou différents, désignent un atome d'azote ou un radical -CH-, et au moins un autre compartiment contenant au moins un agent oxydant.
Un autre dispositif de teinture à plusieurs compartiments comporte au moins un compartiment contenant au moins une 1-(4-aminophényl)-pyrrolidine de formule (I), au moins un compartiment contenant au moins un colorant direct choisi parmi les colorants nitrés benzéniques et les colorants cationiques contenant un atome d'azote quaternisé et une liaison -Z=D- dans laquelle Z et D, identiques ou différents, désignent un atome d'azote ou un radical -CH-, et au moins un autre compartiment contenant au moins un agent oxydant.
Mais d'autres caractéristiques, aspects, objets et avantages de l'invention apparaîtront encore plus clairement à la lecture de la description et des exemples qui suivent.
Le ou les 1-(4-aminophényl)-pyrrolidines de formule (I) selon l'invention sont des composés bien connus de l'homme de l'art et notamment décrits et préparés dans les brevets américains N°- 5851237, 5876464, et 5993491.
Selon la présente invention, on préfère tout particulièrement utiliser des 1-(4-aminophényl)-pyrrolidines de formule (I) pour laquelle :

- $R_1$, $R_2$ et $R_3$ désignent un atome d'hydrogène; le composé de formule (I) est alors la 1-(4-aminophényl)-pyrrolidine, ou,
- $R_1$ et $R_3$ désignent un atome d'hydrogène et $R_2$ désigne le radical -$CH_2OH$; le composé de formule (I) est alors le 1-(4-aminophényl)-2-pyrrolidineméthanol,
  ou,
- $R_1$ désigne un atome d'hydrogène, $R_2$ désigne le radical -$CH_2OH$ et $R_3$ désigne le radical OH; le composé de formule (I) est alors le 1-(4-aminophényl)-4-hydroxy-2-pyrrolidineméthanol,

ou,

- $R_1$ et $R_3$ désignent un atome d'hydrogène et $R_2$ désigne le radical $-CONH_2$; le composé de formule (I) est alors la N-(4-aminophényl)-prolineamide.

Le ou les 1-(4-aminophényl)-pyrrolidines de formule (I) utilisés conformément à l'invention représentent de préférence de 0,001 à 10 % en poids environ du poids total de la composition conforme à l'invention et encore plus préférentiellement de 0,01 à 8% environ de ce poids.

Parmi les colorants directs nitrés benzéniques utilisables selon l'invention, on peut citer les composés de formule (II) suivante :

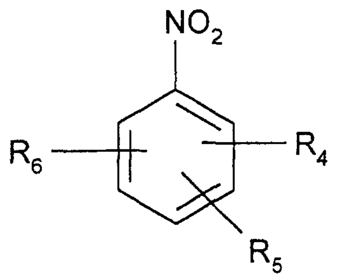

(II)

dans laquelle :

- $R_4$ représente un radical amino ; un radical amino monosubstitué ou disubstitué par un radical alkyle en $C_1$-$C_4$, monohydroxyalkyle en $C_1$-$C_4$, polyhydroxyalkyle en $C_2$-$C_4$, aminoalkyle en $C_1$-$C_4$, monoalkyl($C_1$-$C_4$)amino alkyle en $C_1$-$C_4$, dialkyl($C_1$-$C_4$)amino alkyle en $C_1$-$C_4$, uréidoalkyle en $C_1$-$C_4$, aryle, aryle dont le cycle aryle est substitué par un ou plusieurs radicaux hydroxyle, carboxyle, amino ou dialkyl($C_1$-$C_4$)amino,

- $R_5$ représente un atome d'hydrogène ; un radical amino ; hydroxyle ; alkyle en $C_1$-$C_4$ ; alcoxy en $C_1$-$C_4$ ; monohydroxyalkyle en $C_1$-$C_4$ ; polyhydroxyalkyle en $C_2$-$C_4$ ; monohydroxyalcoxy en $C_1$-$C_4$ ; polyhydroxyalcoxy en $C_2$-$C_4$ ; aminoalcoxy en $C_1$-$C_4$ ; un radical amino monosubstitué ou disubstitué par un radical alkyle en $C_1$-$C_4$, monohydroxyalkyle en $C_1$-$C_4$, polyhydroxyalkyle en $C_2$-$C_4$, aminoalkyle en $C_1$-$C_4$, monoalkyl($C_1$-$C_4$)amino alkyle en $C_1$-$C_4$, dialkyl($C_1$-$C_4$)amino alkyle en $C_1$-$C_4$, uréidoalkyle en $C_1$-$C_4$, aryle, aryle dont le cycle aryle est substitué par un ou plusieurs radicaux hydroxyle, carboxyle, amino ou dialkyl($C_1$-$C_4$)amino ;

- $R_6$ représente un atome d'hydrogène ou d'halogène, un radical alkyle en $C_1$-$C_4$, ou un groupement nitro.

Parmi les colorants nitrés benzéniques de formule (II) ci-dessus, on peut tout particulièrement citer :

- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,

- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- le 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyéthyl)amino 5-méthoxy nitrobenzène,
- le 2-amino 5-β-hydroxyéthyloxy nitrobenzène,
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β,γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-β,γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthyl)amino nitrobenzène,
- le 2-amino 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino 5-amino nitrobenzène,
- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-(β-aminoéthyl)amino 4-hydroxy nitrobenzène,
- l' [hydroxy-2 N-(β-hydroxyéthyl)amino-3 nitro-6] benzyloxy]-2 éthylamine, et
- l' [hydroxy-2 N-(β-hydroxypropyl)amino-3 nitro-6] benzyloxy]-2 éthylamine.

Parmi les colorants nitrés benzéniques de formule (II) ci-dessus, on préfère tout particulièrement :

- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

Parmi les colorants directs cationiques contenant un atome d'azote quaternisé et une liaison -Z=D- utilisables selon l'invention, on peut citer ceux de formules (III), (IV) et (V) suivantes :

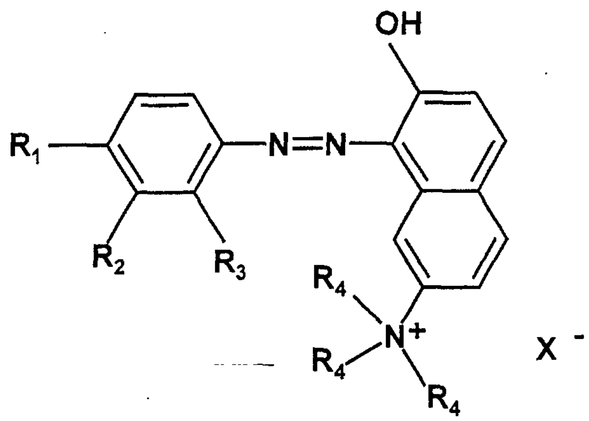

(III)

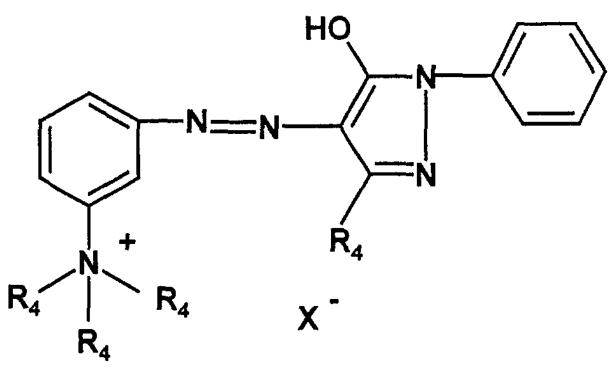

(IV)

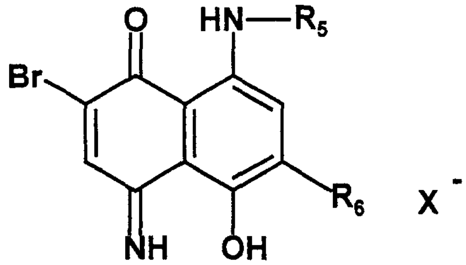

(V)

formules (III) à (V) dans lesquelles :

$R_1$ représente un atome d'hydrogène ou un radical amino ;
$R_2$ représente un atome d'hydrogène ou un groupement nitro ;
$R_3$ représente un atome d'hydrogène , un groupement nitro ou un radical alcoxy en $C_1$-$C_4$ ;
$R_4$ représente un radical alkyle en $C_1$-$C_4$ ;
$R_5$ représente un atome d'hydrogène ou un groupement paratrialkyl$C_1$-$C_4$ ammoniophényle ;
$R_6$ représente un atome de brome ou un groupement NHparatrialkyle$C_1$-$C_4$ ammoniophényle ;
$X^-$ représente un anion de préférence choisi parmi le chlorure, le méthyl sulfate et l'acétate.

Selon la présente invention, on peut également utiliser les composés correspondant aux formes mésomères des structures (III) à (V).
A titre d'exemples de composés de formule (III) on peut citer les colorants Basic Brown 16, Basic Red 76, Basic Brown 17 et Basic Red 118.
A titre d'exemple de composés de formule (IV) on peut citer le colorant Basic Yellow 57.
A titre d'exemple de composés de formule (V) on peut citer le colorant Basic Blue 99.
Ces appellations du Color Index recouvrent les structures chimiques suivantes (sous forme de chlorures) :

. 8-[(4-aminophényl)azo]-7-hydroxy-2- triméthylammonio-naphtalène,
. 8-[(2-méthoxyphényl)azo]-7-hydroxy-2-triméthylammonio-naphtalène,
. 8-[(4-amino-3-nitrophényl)azo]-7-hydroxy-2-triméthylammonio-naphtalène,
. 8-[(4-amino-2-nitrophényl)azo]-7-hydroxy-2-triméthylammonio-naphtalène,
. 3-[(3-méthyl-5-hydroxy-1-phényl-1H-pyrazol-4-yl)azo]-triméthylammonio-benzène,
. 3-[(4-amino-6-bromo-5,8-dihydro-1-hydroxy-8-imino-5-oxo-2-naphtalényl)-amino]-triméthylammonio-benzène,
. 3-[(3,7-dibromo-5,8-dihydro-4-hydroxy-5-imino-8-oxo-1-naphtalényl)- amino]-triméthylammonio-benzène.

Lesdits composés de formules (III) à (V) se retrouvent seuls ou en association dans les produits correspondant aux appellations commerciales, proposées par la société WARNER JENKINSON, suivantes :

- ARIANOR MAHOGANY ; ARIANOR STEEL BLUE ; ARIANOR MADDER RED ; ARIANOR SIENNA BROWN ; ARIANOR STRAW YELLOW ; ARIANOR BORDEAUX.

Parmi les colorants directs cationiques contenant un atome d'azote quaternisé et une liaison -Z=D- utilisables selon l'invention, on peut également citer ceux de formule (VI), (VII), (VIII), (VIII') et (IX) suivantes :

**a) les composés de formule (VI) suivante :**

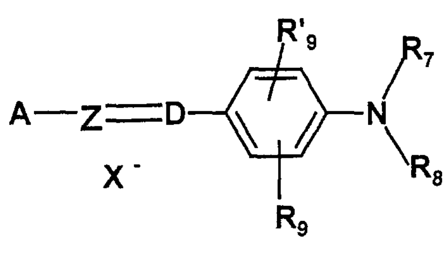

(VI)

dans laquelle :

Z et D représentent, identiques ou différents, un atome d'azote ou le radical -CH-, $R_7$ et $R_8$, identiques ou différents, représentent un atome d'hydrogène ; un radical 4'-aminophényle, un radical alkyle en $C_1$-$C_4$ pouvant être substitué par un radical -CN, -OH ou -$NH_2$ ou forment avec un atome de carbone du cycle benzénique un hétérocycle azoté pouvant contenir un ou plusieurs atomes d'oxygène et/ou un ou plusieurs autres atomes d'azote, et pouvant être substitué par un ou plusieurs radicaux alkyle en $C_1$-$C_4$ ;

$R_9$ et $R'_9$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,

$X^-$ représente un anion de préférence choisi parmi le chlorure, le méthyl sulfate et l'acétate,

A représente un groupement choisi par les structures A1 à A19 suivantes :

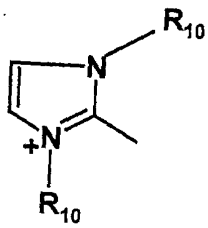

$A_1$ ;

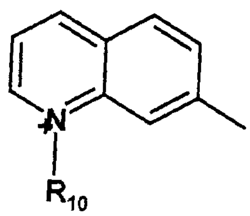

$A_2$ ;

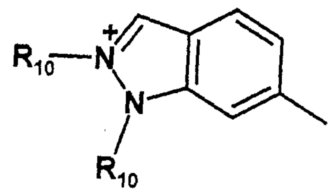

$A_3$ ;

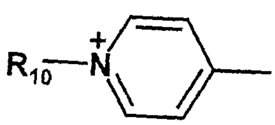

$A_4$ ;

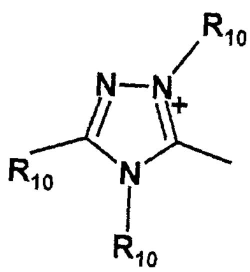

$A_5$ ;

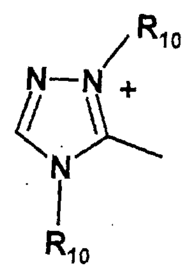

$A_6$ ;

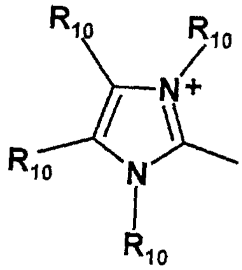

$A_7$ ;

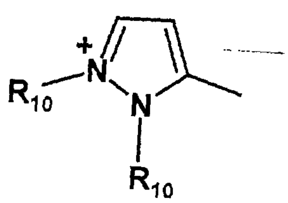

$A_8$ ;

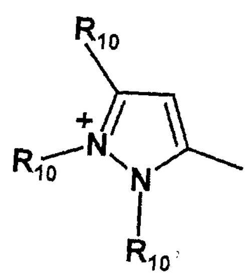

$A_9$ ;

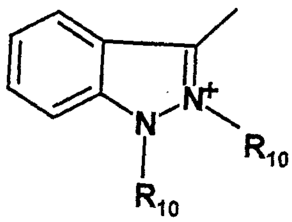

A10

;

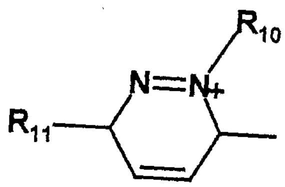

A11

;

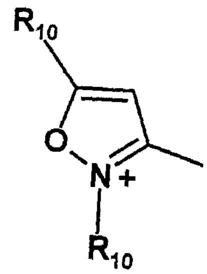

A12

;

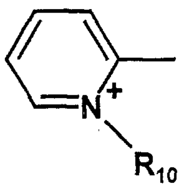

A13

;

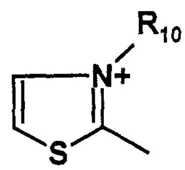

A14

;

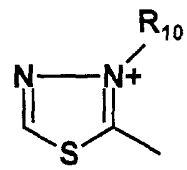

A15

;

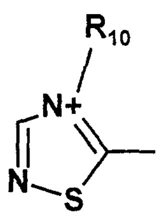

A16

;

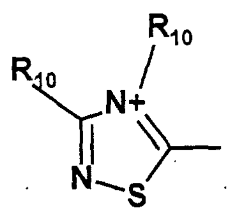

A17

;

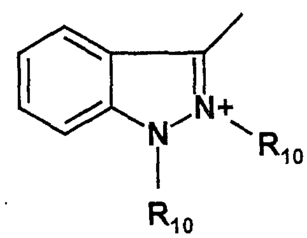

A18

et

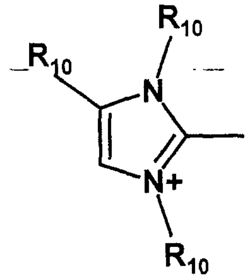

A19

;

dans lesquelles $R_{10}$ représente un radical alkyle en $C_1$-$C_4$ pouvant être substitué par un radical hydroxyle et $R_{11}$ représente un radical alcoxy en $C_1$-$C_4$;

**b) les composés de formule (VII) suivante :**

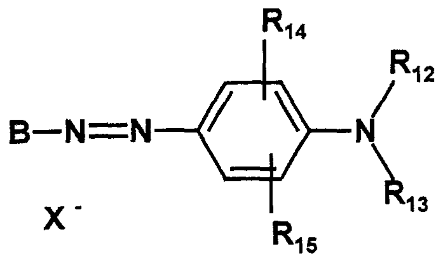

(VII)

dans laquelle :

$R_{12}$ représente un atome d'hydrogène ou un radical alkyle en $C_1$-$C_4$,
$R_{13}$ représente un atome d'hydrogène, un radical alkyle pouvant être substitué par un radical -CN ou par un groupement amino, un radical 4'-aminophényle, ou forme avec $R_{12}$ un hétérocycle éventuellement oxygéné et/ou azoté pouvant être substitué par un radical alkyle en $C_1$-$C_4$,
$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion de préférence choisi parmi le chlorure, le méthyl sulfate et l'acétate,
B représente un groupement choisi par les structures B1 à B6 suivantes :

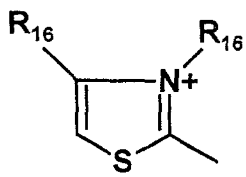
;
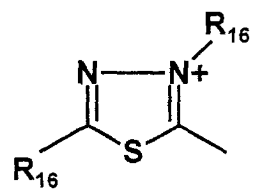
;
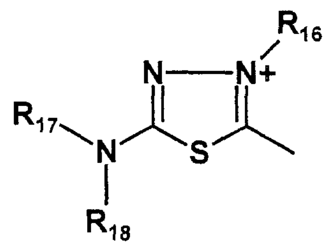
;

B1 B2 B3

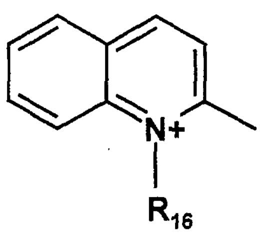
;
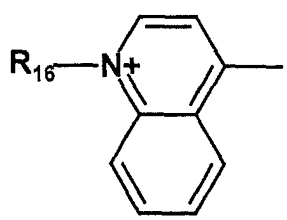
et
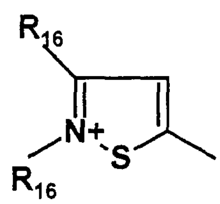
;

B4 B5 B6

dans lesquelles $R_{16}$ représente un radical alkyle en $C_1$-$C_4$, $R_{17}$ et $R_{18}$, identiques ou différents, représentent un atome d'hydrogène ou un radical alkyle en $C_1$-$C_4$ ;

**c) les composés de formules (VIII) et (VIII') suivantes :**

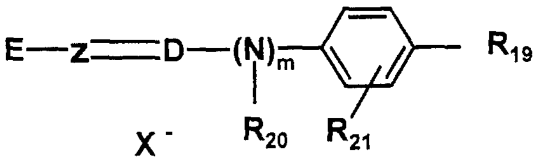

(VIII)

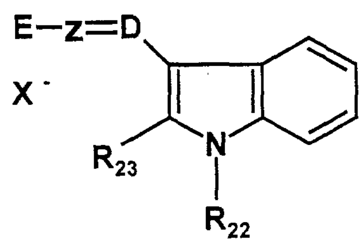

(VIII')

dans lesquelles :

Z et D, identiques ou différents, représentent un atome d'azote ou le radical -CH-,
$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
m = 0 ou 1,
$X^-$ représente un anion de préférence choisi parmi le chlorure, le méthyl sulfate et l'acétate,
E représente un groupement choisi par les structures E1 à E8 suivantes :

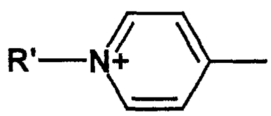

E1

;

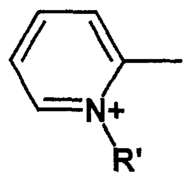

E2

;

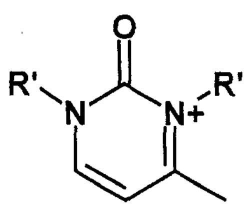

E3

;

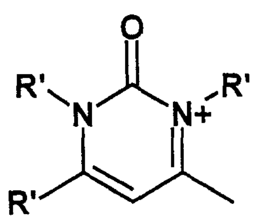

E4

;

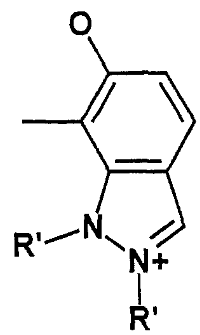

E5

;

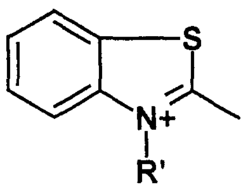 ; 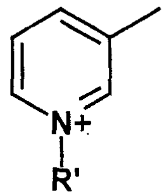 et 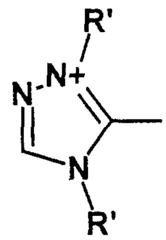

E6 E7 E8 ;

dans lesquelles,
R' représente un radical alkyle en $C_1$-$C_4$ ;
lorsque m = 0 et que $D_1$ représente un atome d'azote, alors E peut également désigner un groupement de structure E9 suivante :

E9 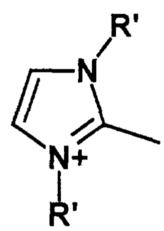 ;

dans laquelle R' représente un radical alkyle en $C_1$-$C_4$.

**d) les composés de formule (IX) suivante :**

**G-N=N-J (IX)**

dans laquelle :
**le symbole G** représente un groupement choisi parmi les structures $G_1$ à $G_3$ suivantes :

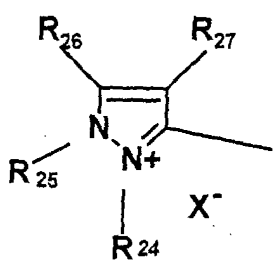

$G_1$

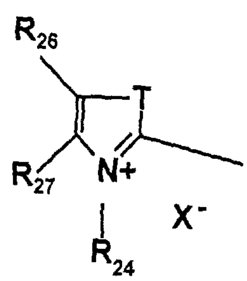

$G_2$

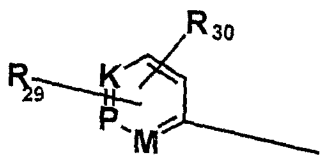

$G_3$

structures $G_1$ à $G_3$ dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{25}$ désigne un radical alkyle en $C_1$-$C_4$ ou un radical phényle;
$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$ , alcoxy en $C_1$-$C_4$, ou $NO_2$, ou forment ensemble dans $G_2$ un cycle benzénique éventuellement substitué par un ou plusieurs radicaux alkyle en $C_1$-$C_4$ , alcoxy en $C_1$-$C_4$, ou $NO_2$;
$R_{26}$ peut désigner en outre un atome d'hydrogène;
T désigne un atome d'oxygène, de soufre ou un groupement $-NR_{25}$;
M représente un groupement -CH, -CR (R désignant alkyle en $C_1$-$C_4$), ou $-NR_{28}(X^-)_r$;
K représente un groupement -CH, -CR (R désignant alkyle en $C_1$-$C_4$), ou $-NR_{28}(X^-)_r$;
P représente un groupement -CH, -CR (R désignant alkyle en $C_1$-$C_4$), ou $-NR_{28}(X^-)_r$; r désigne zéro ou 1;
$R_{28}$ représente un atome $O^-$, un radical alcoxy en $C_1$-$C_4$, ou un radical alkyle en $C_1$-$C_4$;
$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical $-NO_2$;
$X^-$ représente un anion de préférence choisi parmi le chlorure, l'iodure, le méthyl sulfate, l'éthyl sulfate, l'acétate et le perchlorate;

**le symbole J** représente :

- **(a)** un groupement de structure $J_1$ suivante :

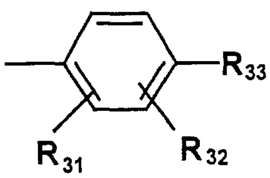

$J_1$

structure $J_1$ dans laquelle,

$R_{31}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{34}$, $-NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;
$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$ ou forme avec $R_{33}$ ou $R_{34}$ un cycle à 5 ou 6 chaînons contenant ou non un ou plusieurs hétéroatomes choisis parmi l'azote, l'oxygène ou le soufre;
$R_{33}$ représente un atome d'hydrogène, un radical -OH, un radical $-NHR_{34}$, un radical $-NR_{35}R_{36}$;
$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;
$R_{35}$ et $R_{36}$, identiques ou différents, représentent un radical alkyle en $C_1$-$C_4$ , un radical monohydroxyalkyle en $C_1$-$C_4$, polyhydroxyalkyle en $C_2$-$C_4$;

- **(b)** un groupement hétérocyclique azoté à 5 ou 6 chaînons susceptible de renfermer d'autres hétéroatomes

et/ou des groupements carbonylés et pouvant être substitué par un ou plusieurs radicaux alkyle en $C_1$-$C_4$, amino ou phényle, et notamment un groupement de structure $J_2$ suivante :

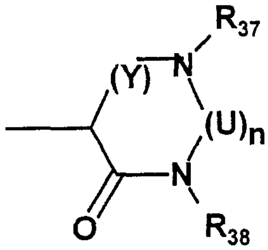

$J_2$

structure $J_2$ dans laquelle,

$R_{37}$ et $R_{38}$, identiques ou différents, représentent un atome d'hydrogène , un radical alkyle en $C_1$-$C_4$, un radical phényle;

Y désigne le radical -CO- ou le radical

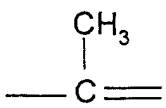

;

n = 0 ou 1, avec, lorsque n désigne 1, U désigne le radical -CO- .

On peut également utiliser des composés correspondant aux formes mésomères de ces structures (VI) à (IX).
Dans les structures (VI) à (IX) comme dans les structures (III) à (V) définies ci-dessus le groupement alkyle ou alcoxy en $C_1$-$C_4$ désigne de préférence méthyle, éthyle, butyle, méthoxy, éthoxy.
Les colorants cationiques de formules (VI), (VII), (VIII), et (VIII') sont des composés connus et décrits par exemple dans les demandes de brevets WO 95/15144, WO-95/01772 et EP-A 714954; ceux de formule (IX) sont des composés connus et décrits par exemple dans les demandes de brevets FR-2189006, FR-2285851 et FR-2140205 et ses certificats d'addition.
Parmi les colorants directs cationiques de formule (VI) utilisables dans les compositions tinctoriales conformes à l'invention, on peut plus particulièrement citer les composés répondant aux structures ($VI_1$) à ($VI_{54}$) suivantes :

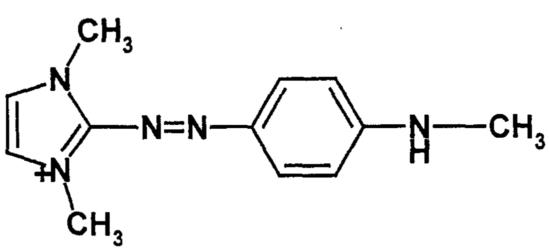

$Cl^-$ ($VI_1$)

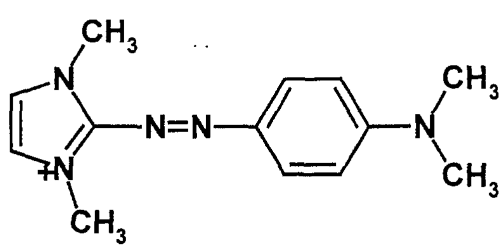

$Cl^-$ ($VI_2$)

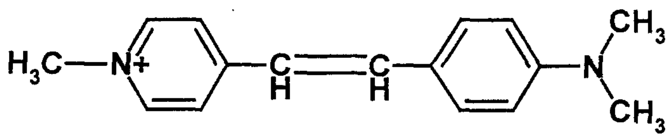

Cl ⁻ (VI$_3$)

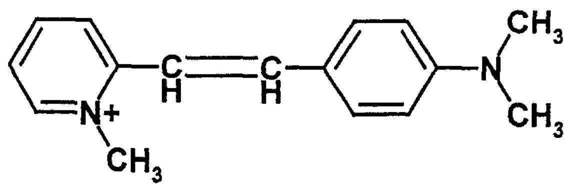

Cl ⁻ (VI$_4$)

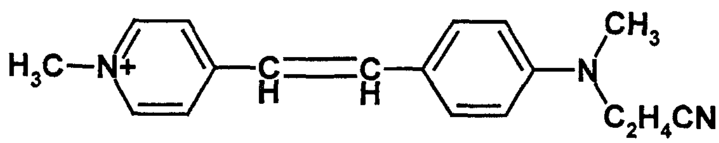

Cl ⁻ (VI$_5$)

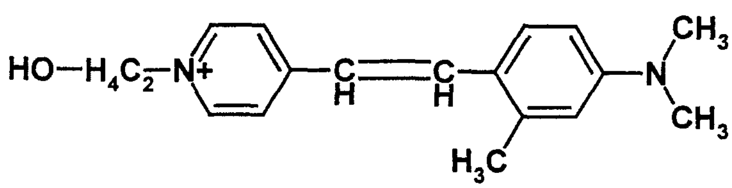

Cl ⁻ (VI$_6$)

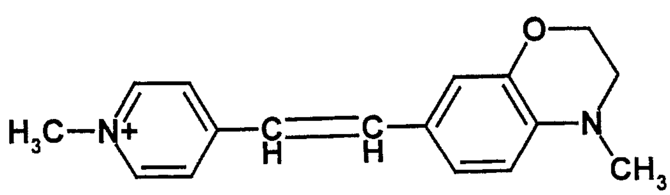

Cl ⁻ (VI$_7$)

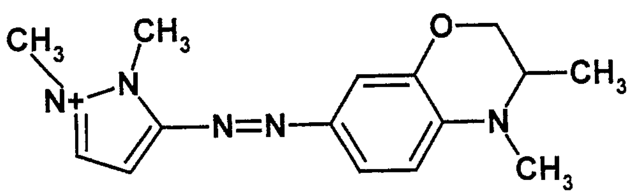

Cl ⁻ (VI$_8$)

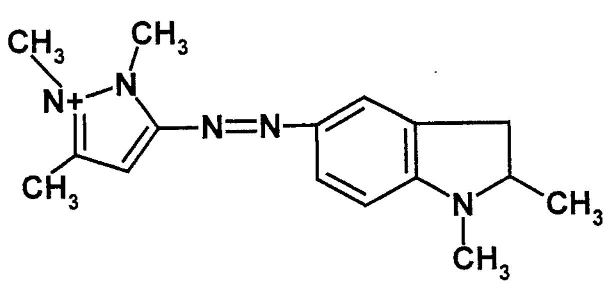

(VI$_9$)

Cl ⁻

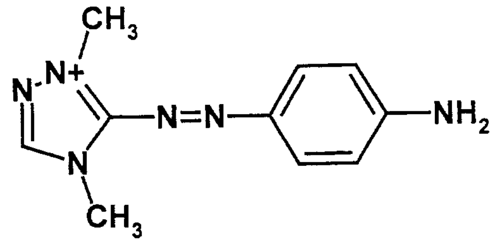

Cl $^-$ (VI$_{10}$)

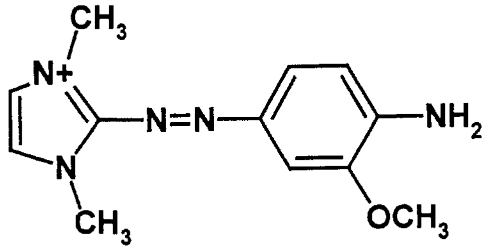

Cl $^-$ (VI$_{11}$)

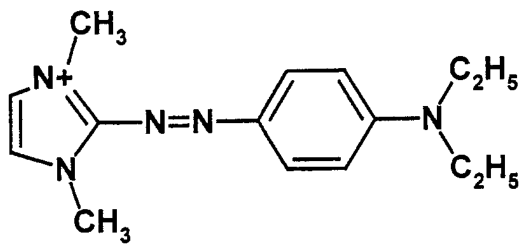

Cl $^-$ (VI$_{12}$)

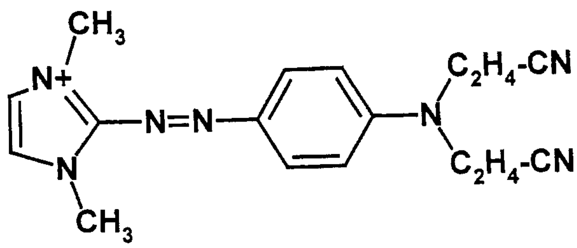

Cl $^-$ (VI$_{13}$)

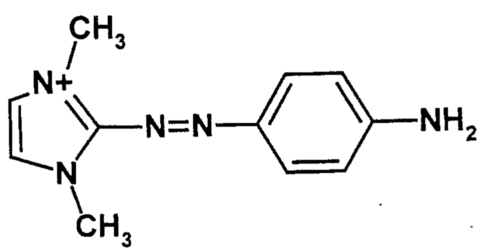

Cl $^-$ (VI$_{14}$)

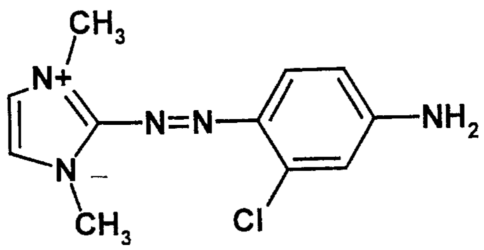

$Cl^-$ (VI$_{15}$)

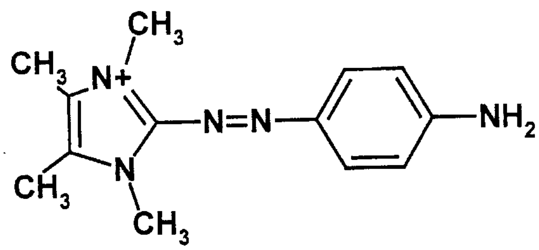

$Cl^-$ (VI$_{16}$)

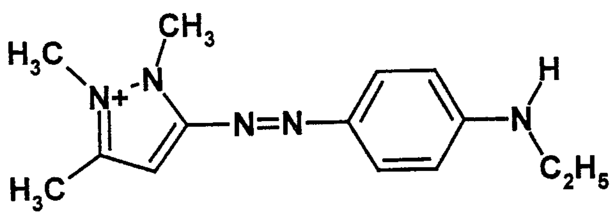

$Cl^-$ (VI$_{17}$)

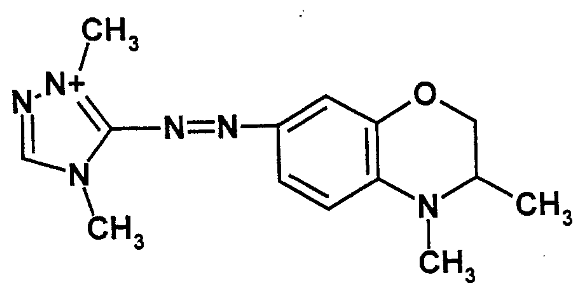

$Cl^-$ (VI$_{18}$)

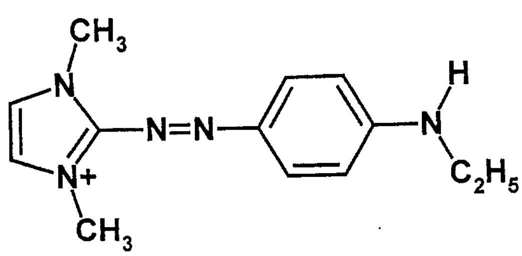

$Cl^-$ (VI$_{19}$)

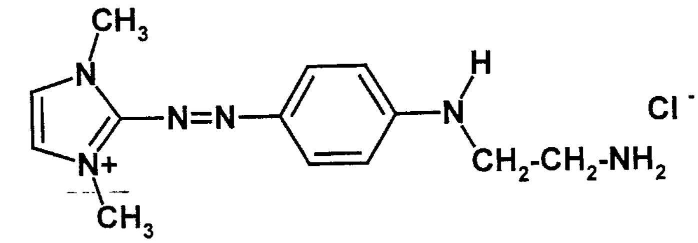

$(VI_{20})$

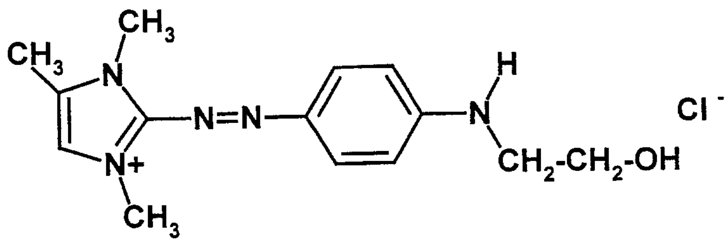

$(VI_{21})$

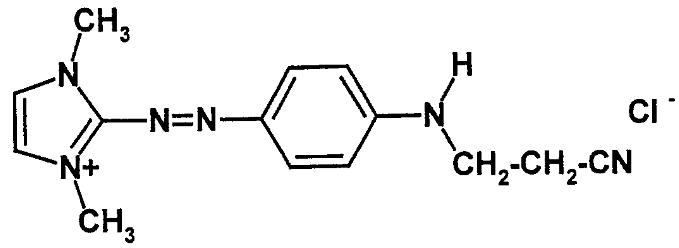

$(VI_{22})$

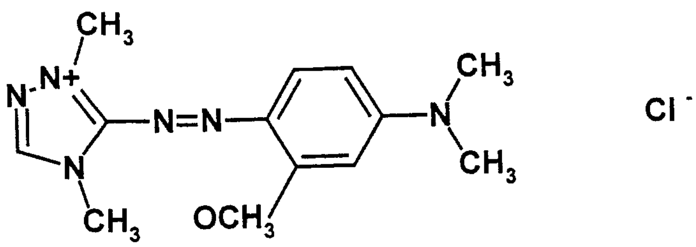

$(VI_{23})$

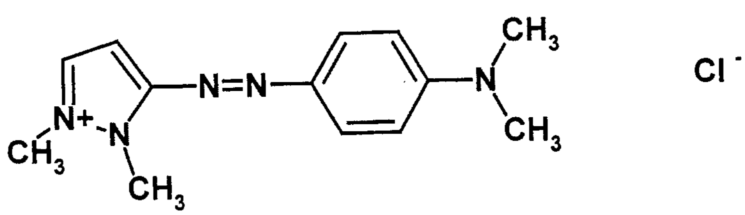

$(VI_{24})$

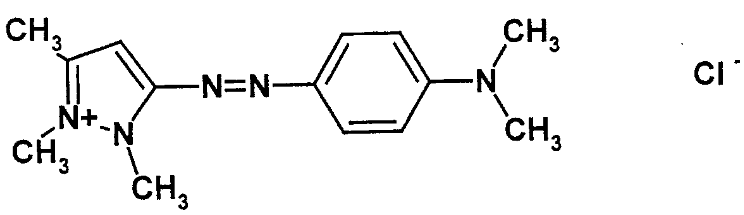

$(VI_{25})$

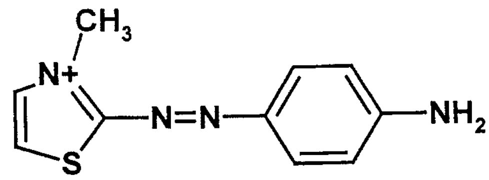

$Cl^-$ (VI$_{26}$)

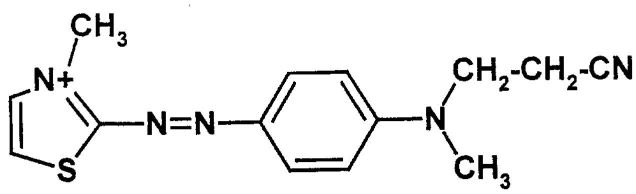

$Cl^-$ (VI$_{27}$)

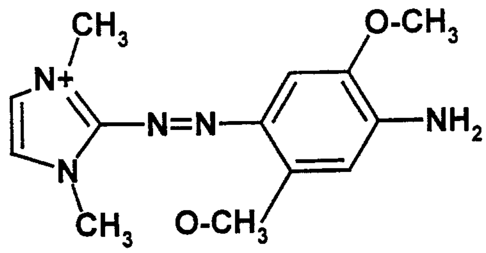

$Cl^-$ (VI$_{28}$)

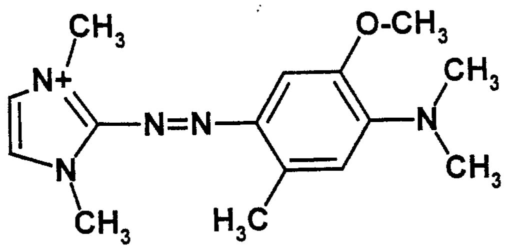

$Cl^-$ (VI$_{29}$)

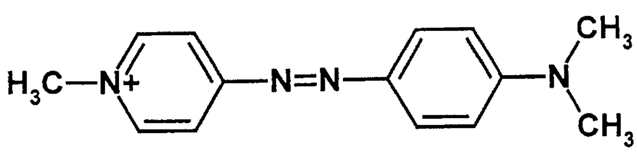

$Cl^-$ (VI$_{30}$)

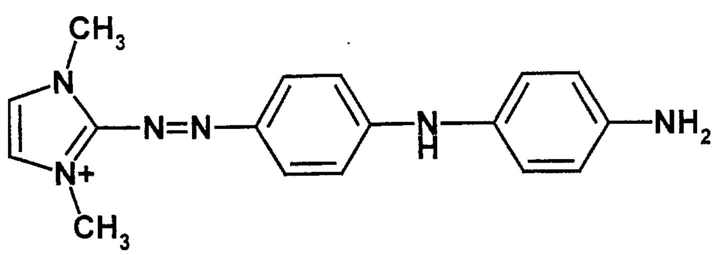

$Cl^-$ (VI$_{31}$)

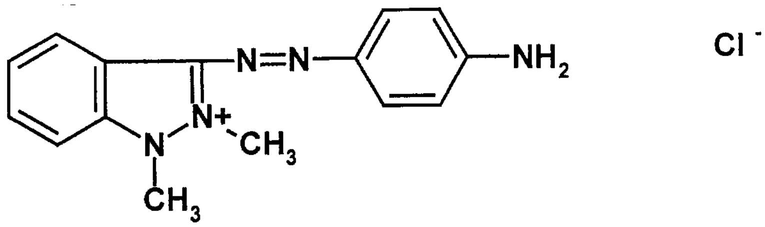

$(VI_{32})$

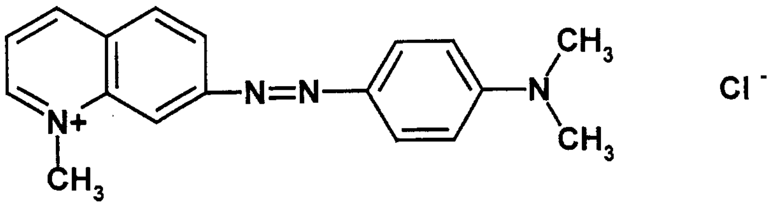

$(VI_{33})$

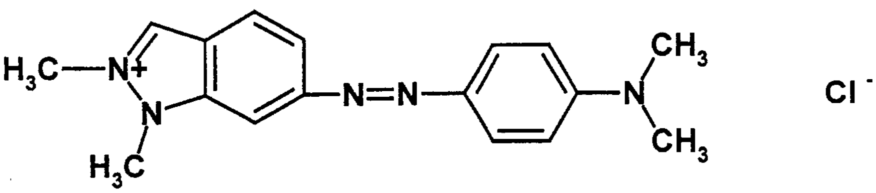

$(VI_{34})$

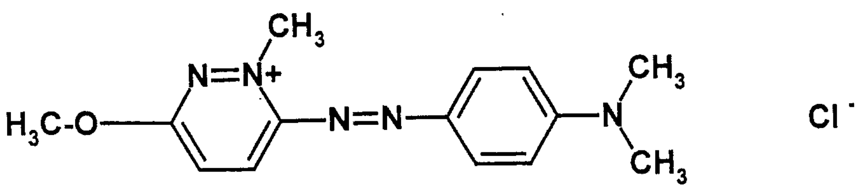

$(VI_{35})$

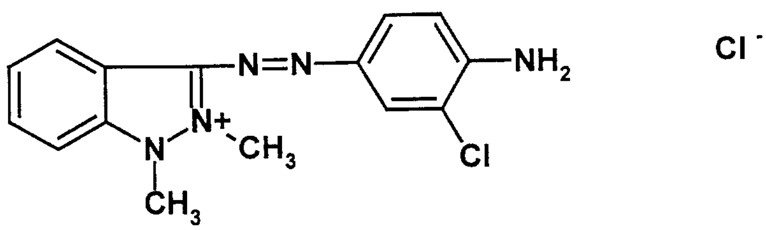

$(VI_{36})$

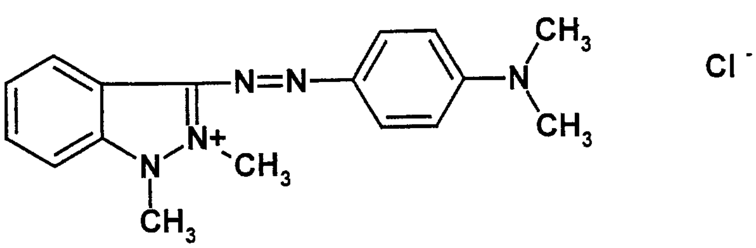

$(VI_{37})$

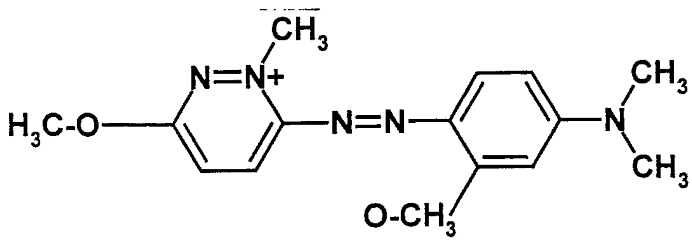

$Cl^-$ (VI$_{38}$)

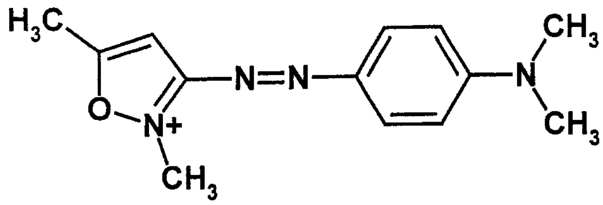

$Cl^-$ (VI$_{39}$)

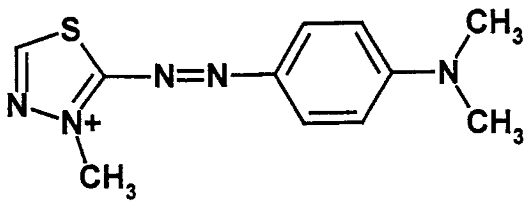

$Cl^-$ (VI$_{40}$)

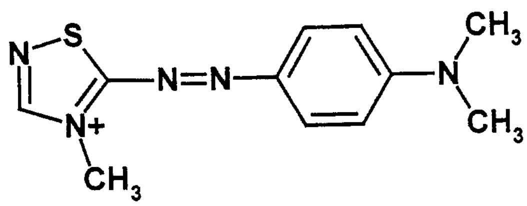

$Cl^-$ (VI$_{41}$)

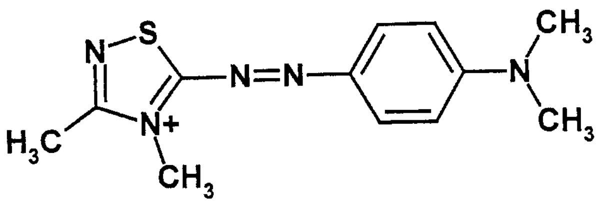

$Cl^-$ (VI$_{42}$)

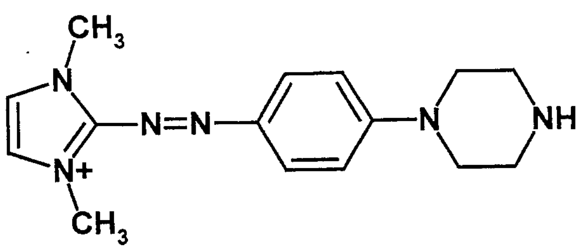

$Cl^-$ (VI$_{43}$)

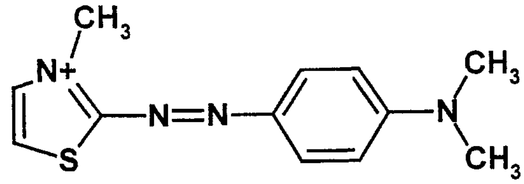

$Cl^-$ (VI$_{44}$)

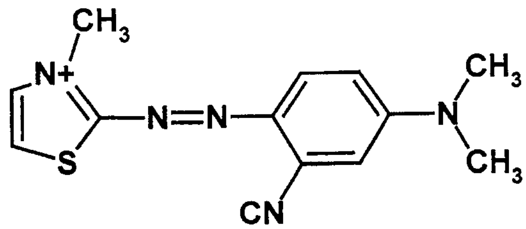

$Cl^-$ (VI$_{45}$)

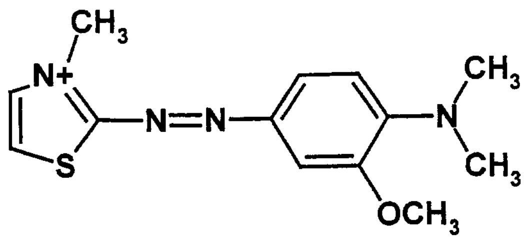

$Cl^-$ (VI$_{46}$)

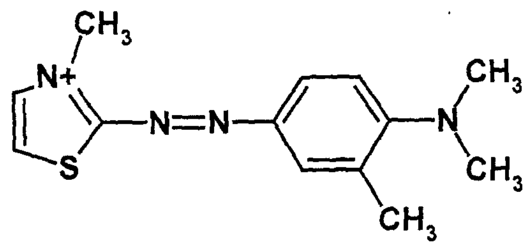

$Cl^-$ (VI$_{47}$)

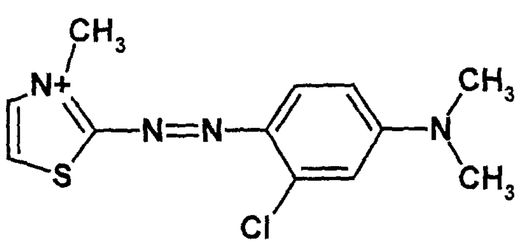

$CH_3SO_4^-$ (VI$_{48}$)

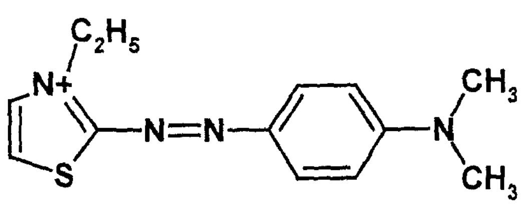

$CH_3SO_4^-$ (VI$_{49}$)

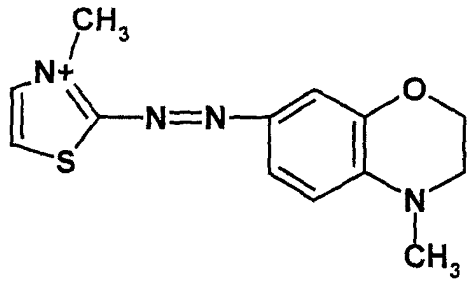

Cl⁻ (VI$_{50}$)

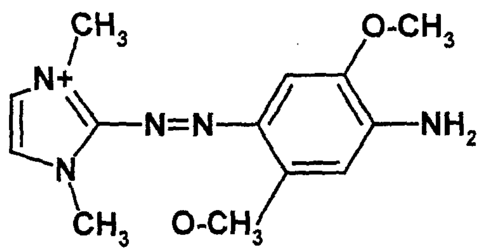

Cl⁻ (VI$_{51}$)

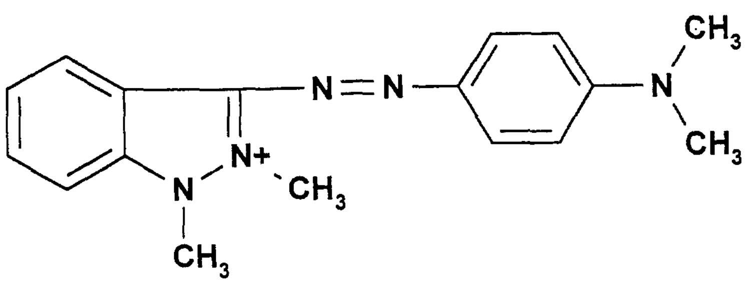

Cl⁻ (VI$_{52}$)

;

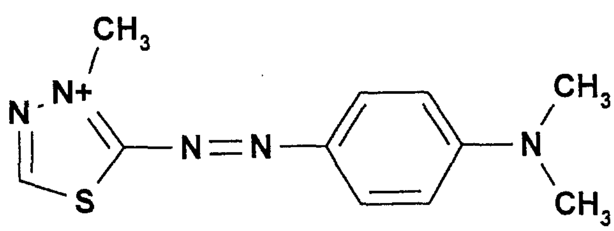

$CH_3SO_4^-$ (VI$_{53}$)

;

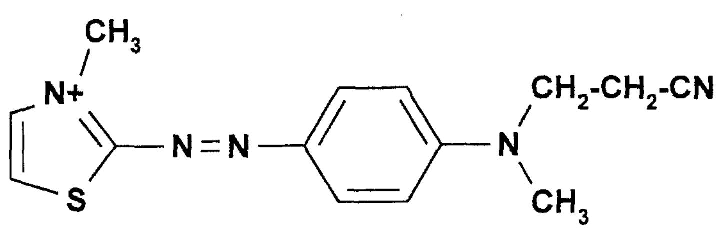

Cl⁻ (VI$_{54}$)

Parmi les composés de structures (VI$_1$) à (VI$_{54}$) décrits ci-dessus, on préfère tout particulièrement les composés répondant aux structures (VI$_1$), (VI$_2$), (VI$_{14}$) et (VI$_{31}$).

Parmi les colorants directs cationiques de formule (VII) utilisables dans les compositions tinctoriales conformes à l'invention, on peut plus particulièrement citer les composés répondant aux structures (VII$_1$) à (VII$_9$) suivantes :

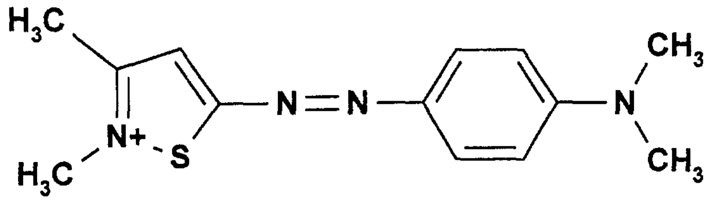

$Cl^-$ (VII$_1$)

;

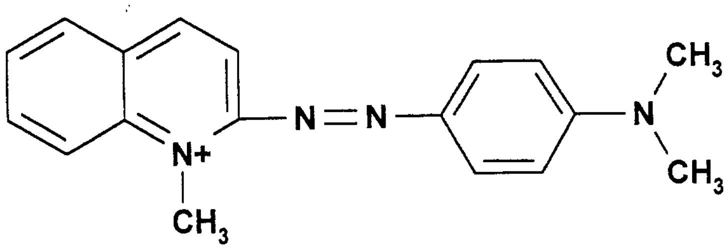

$Cl^-$ (VII$_2$)

;

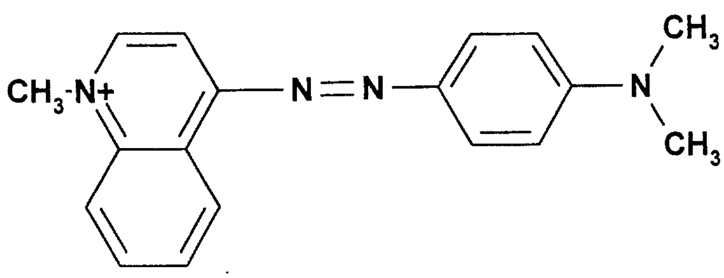

$Cl^-$ (VII$_3$)

;

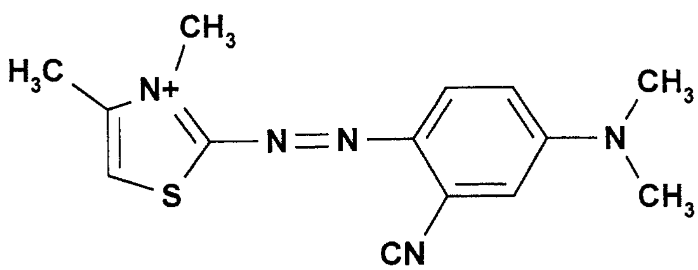

$Cl^-$ (VII$_4$)

;

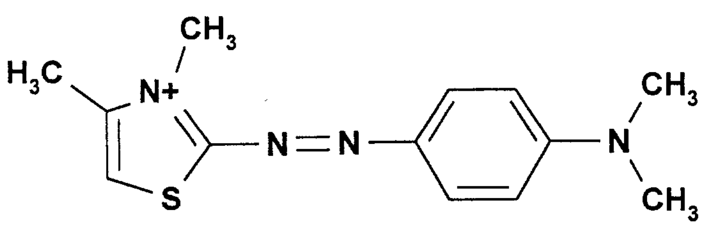

$CH_3SO_4^-$ (VII$_5$)

;

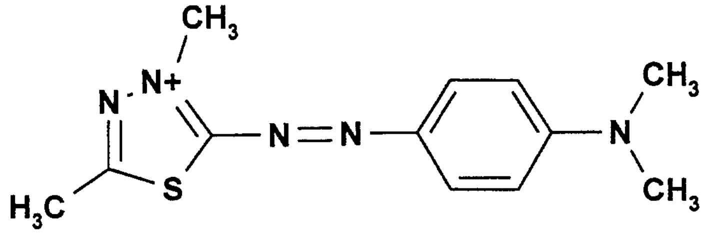

$CH_3SO_4^-$ (VII$_6$)

;

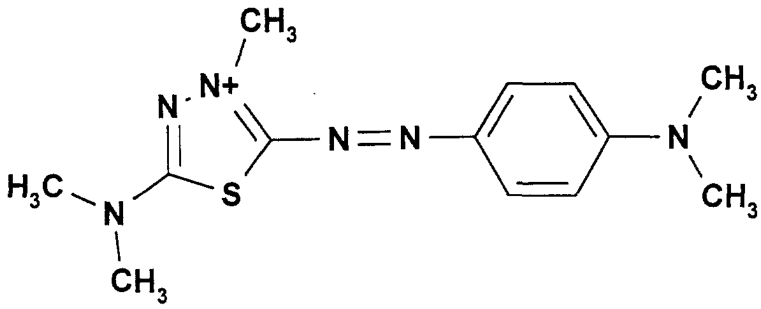

$CH_3SO_4^-$ (VII$_7$)

;

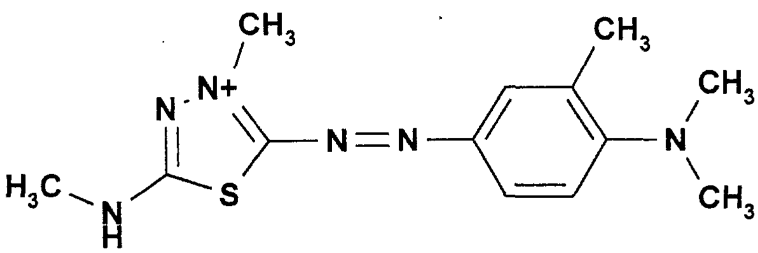

$CH_3SO_4^-$ (VII$_8$)

; et

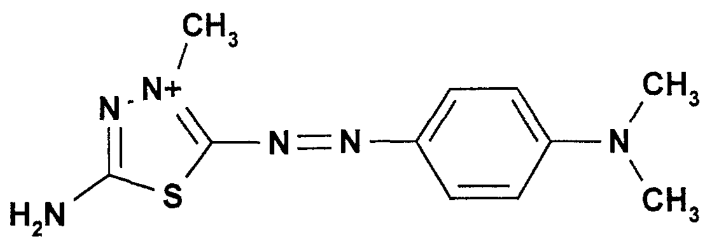

$CH_3SO_4^-$ (VII$_9$)

.

Parmi les colorants directs cationiques de formule (VIII), utilisables dans les compositions tinctoriales conformes à l'invention, on peut plus particulièrement citer les composés répondant aux structures (VIII$_1$) à (VIII$_{18}$) suivantes :

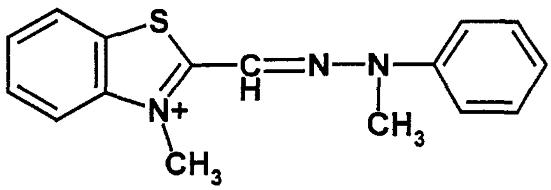

$(VIII_1)$

$Cl^-$

;

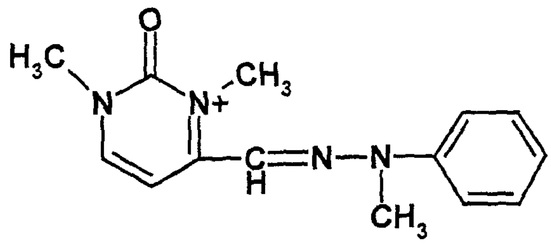

$(VIII_2)$

$Cl^-$

;

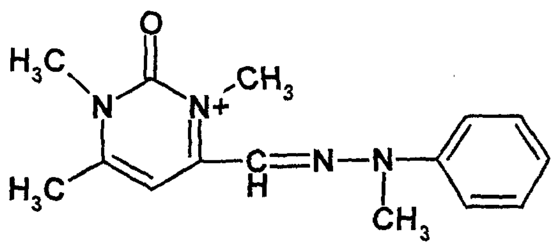

$(VIII_3)$

$Cl^-$

;

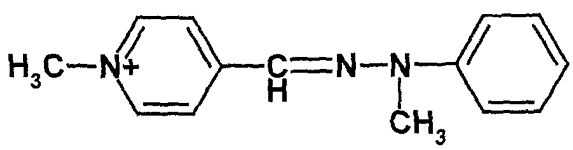

$(VIII_4)$

$CH_3SO_4^-$

;

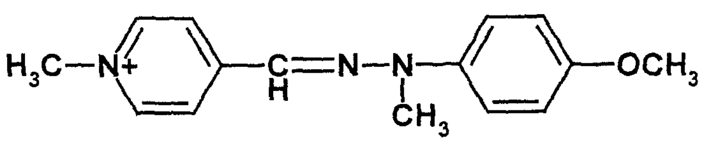

$(VIII_5)$

$Cl^-$

;

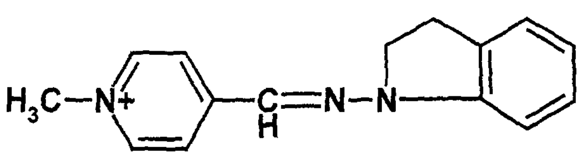

$(VIII_6)$

$CH_3SO_4^-$

;

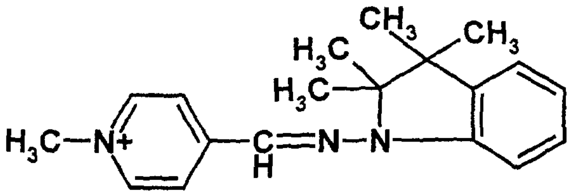

(VIII$_7$)

$CH_3SO_4^-$

;

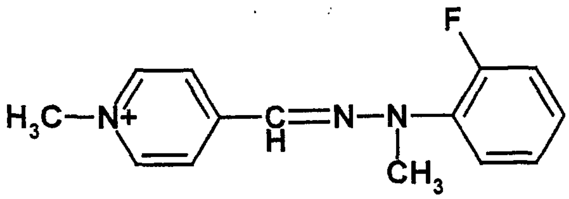

(VIII$_8$)

$Cl^-$

;

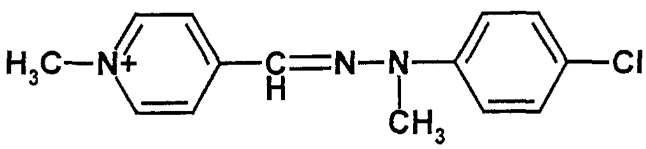

(VIII$_9$)

$Cl^-$

;

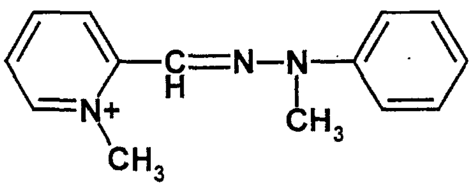

(VIII$_{10}$)

$CH_3SO_4^-$

;

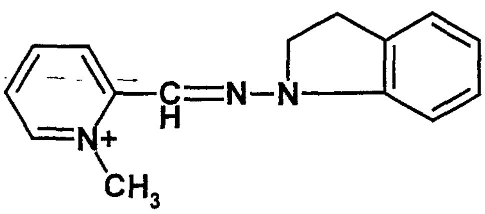

(VIII$_{11}$)

$CH_3SO_4^-$

;

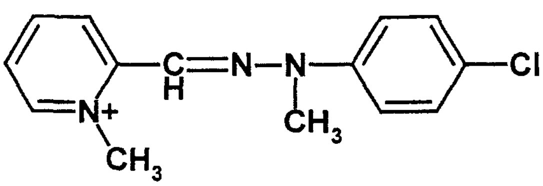

(VIII$_{12}$)

$CH_3SO_4^-$

;

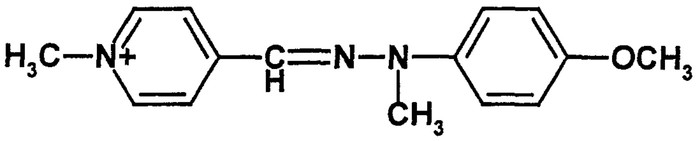

$CH_3SO_4^-$ (VIII$_{13}$)

;

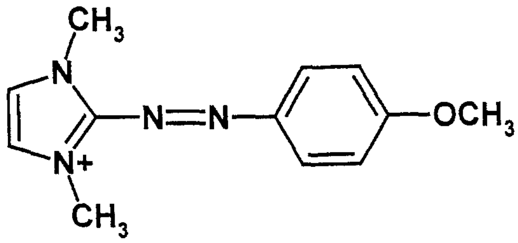

$Cl^-$ (VIII$_{14}$)

;

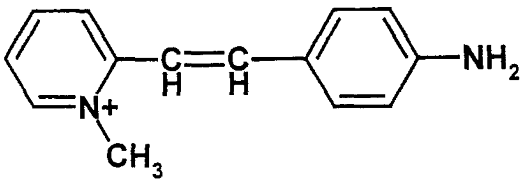

$CH_3COO^-$ (VIII$_{15}$)

;

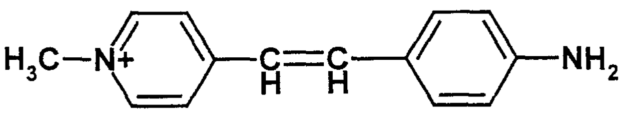

$CH_3COO^-$ (VIII$_{16}$)

;

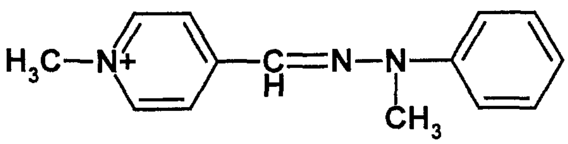

$Cl^-$ (VIII$_{17}$)

; et

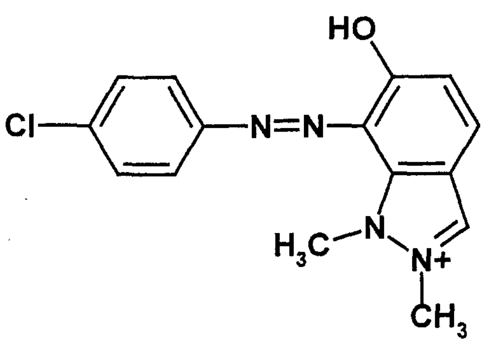

$Cl^-$ (VIII$_{18}$)

.

Parmi les composés particuliers de structures ($VIII_1$) à ($VIII_{18}$) décrits ci-dessus, on préfère tout particulièrement les composés répondant aux structures ($VIII_4$), ($VIII_5$) et ($VIII_{13}$).

Parmi les colorants directs cationiques de formule (VIII'), utilisables dans les compositions tinctoriales conformes à l'invention, on peut plus particulièrement citer les composés répondant aux structures ($VIII'_1$) à ($VIII'_3$) suivantes :

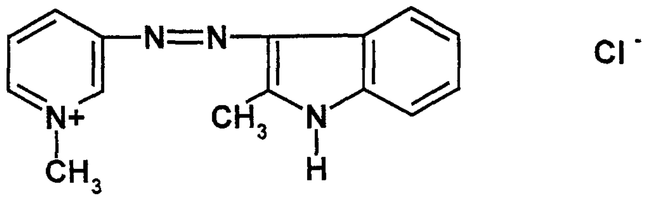

($VIII'_1$)

;

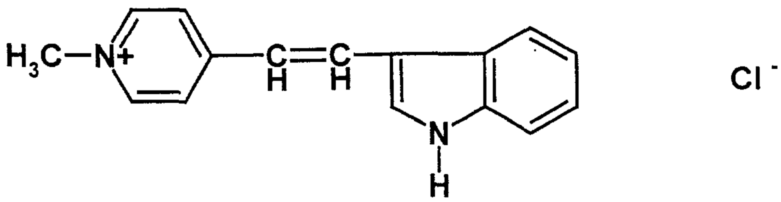

($VIII'_2$)

; et

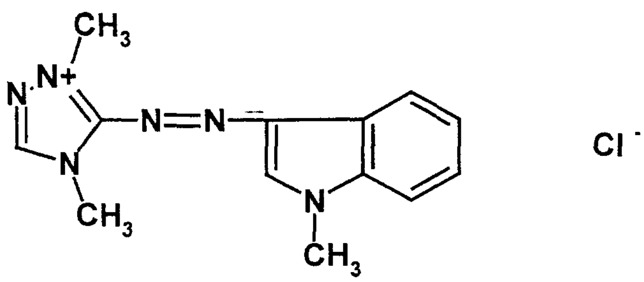

($VIII'_3$)

Parmi les colorants directs cationiques de formule (IX) utilisables dans les compositions tinctoriales conformes à l'invention, on peut citer plus particulièrement les composés de structures ($IX_1$) à ($IX_{77}$) suivantes :

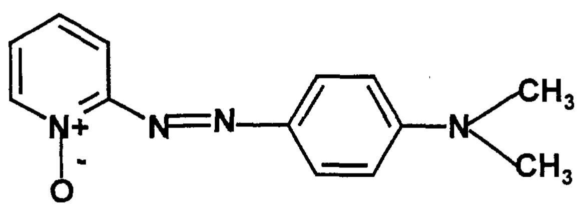

($IX_1$)

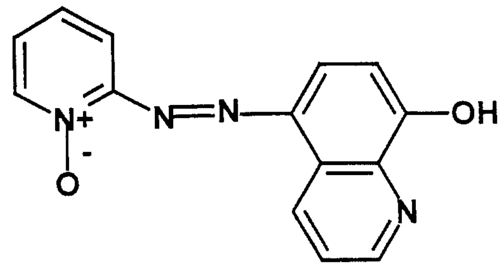

(IX$_2$)

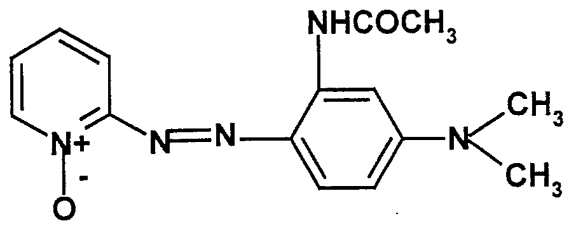

(IX$_3$)

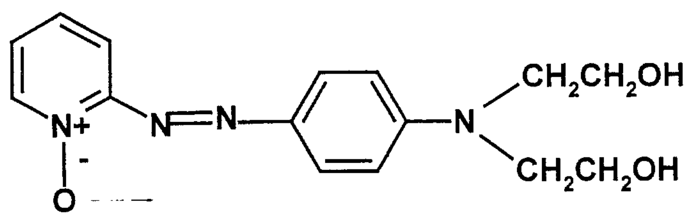

(IX$_4$)

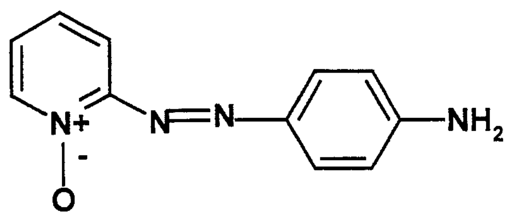

(IX$_5$)

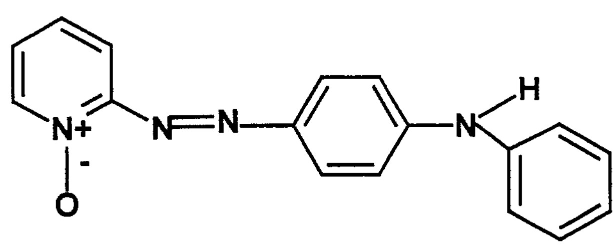

(IX$_6$)

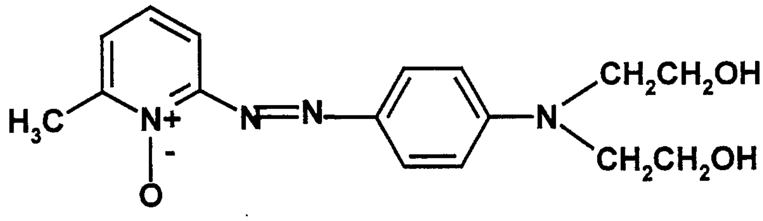

(IX$_7$)

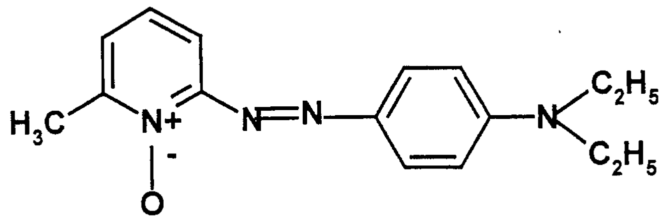

(IX8)

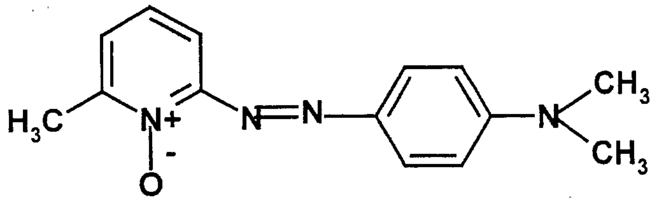

(IX9)

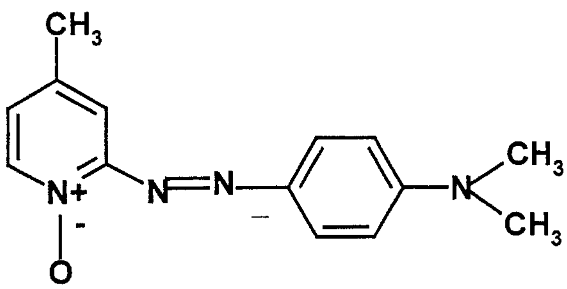

(IX10)

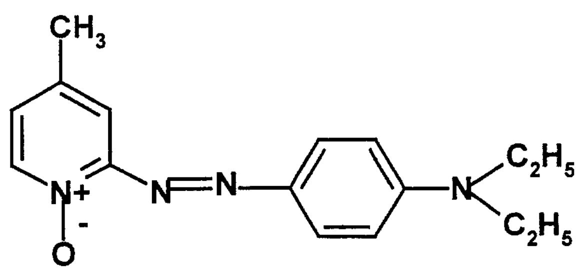

(IX11)

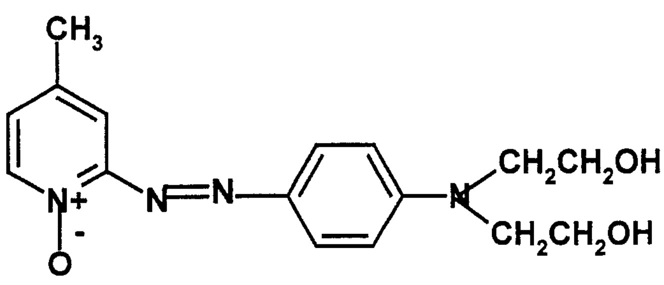

(IX12)

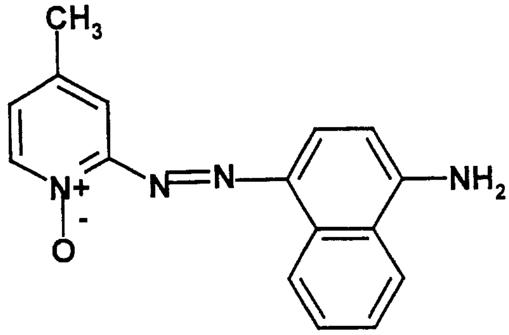

$(IX_{13})$

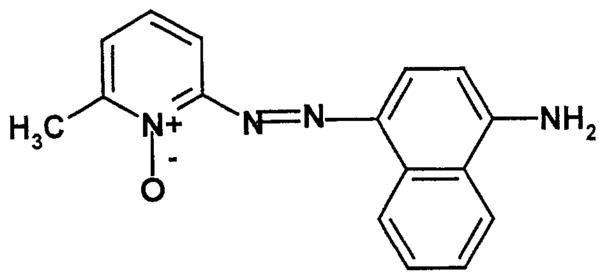

$(IX_{14})$

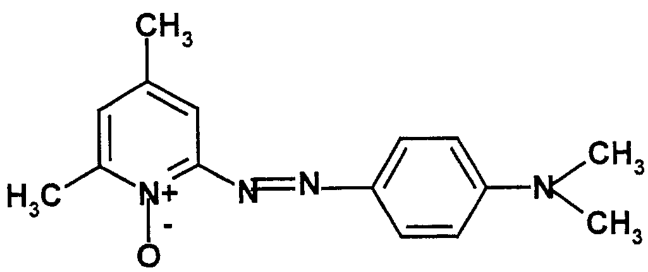

$(IX_{15})$

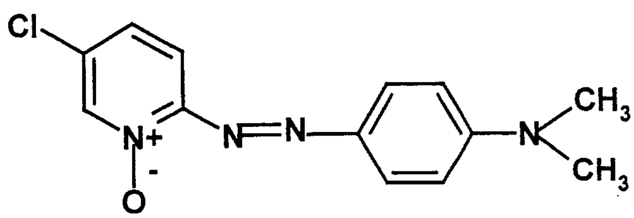

$(IX_{16})$

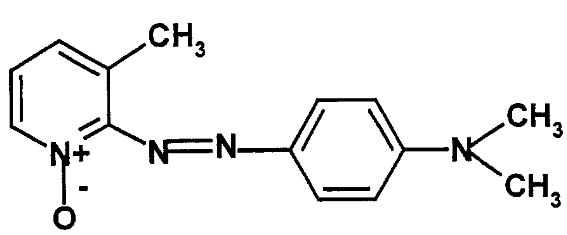

$(IX_{17})$

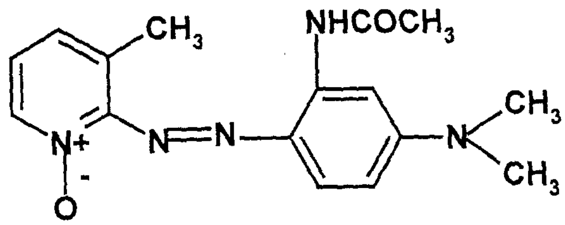

(IX18)

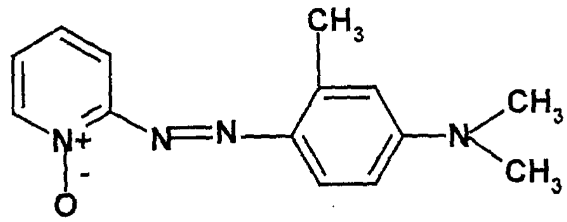

(IX19)

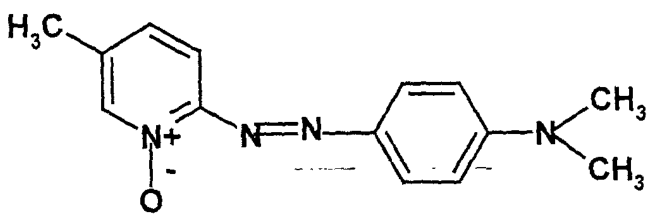

(IX20)

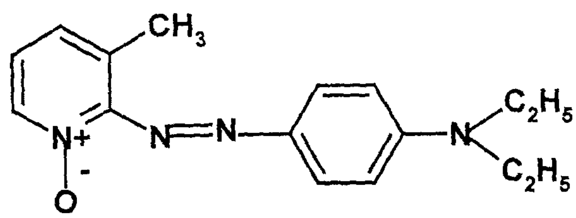

(IX21)

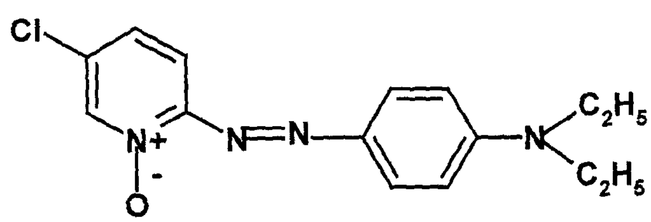

(IX22)

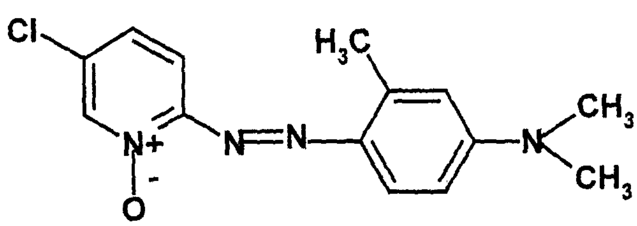

(IX23)

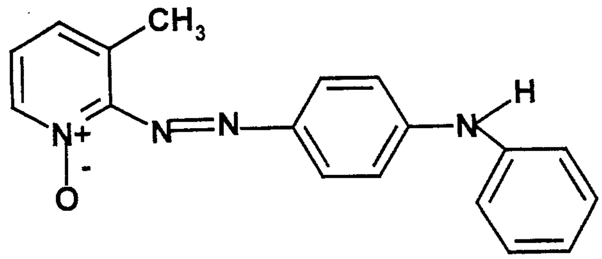

(IX24)

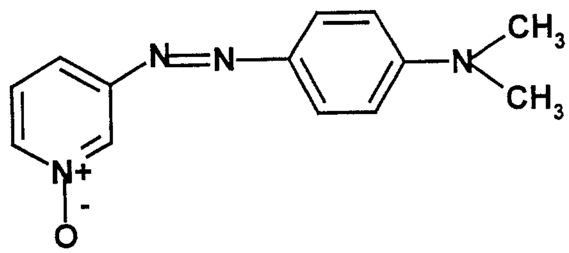

(IX25)

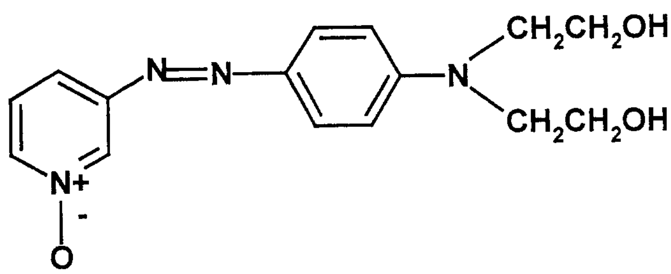

(IX26)

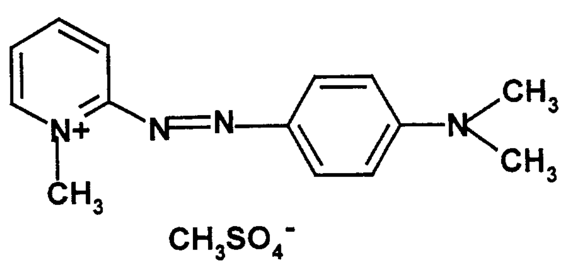

(IX27)

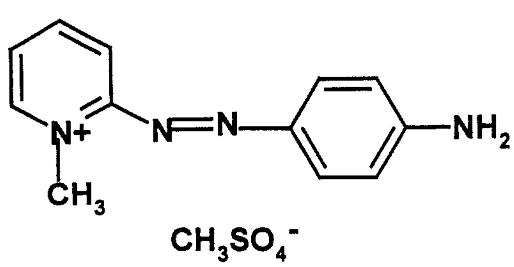

(IX28)

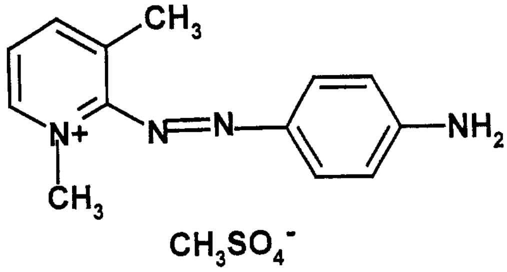

(IX$_{29}$)

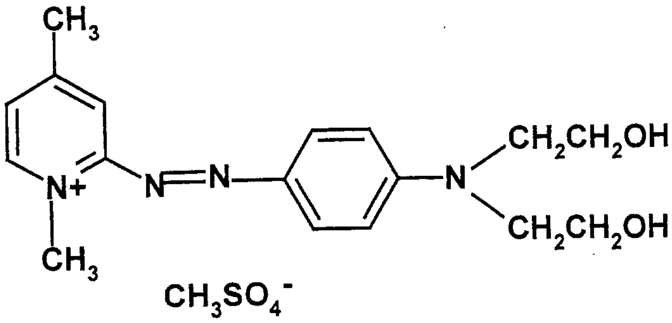

(IX$_{30}$)

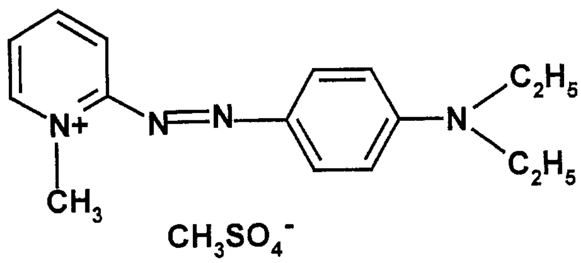

(IX$_{31}$)

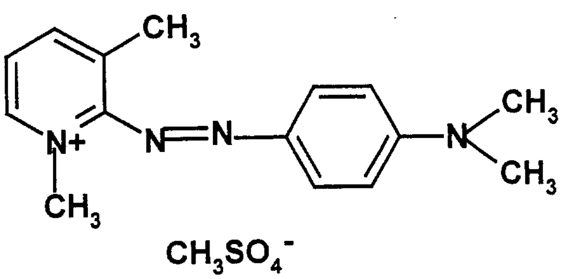

(IX$_{32}$)

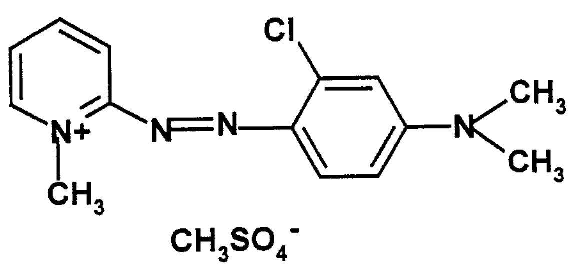

(IX$_{33}$)

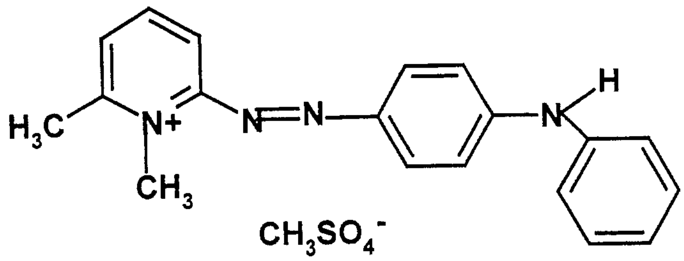

$(IX_{34})$

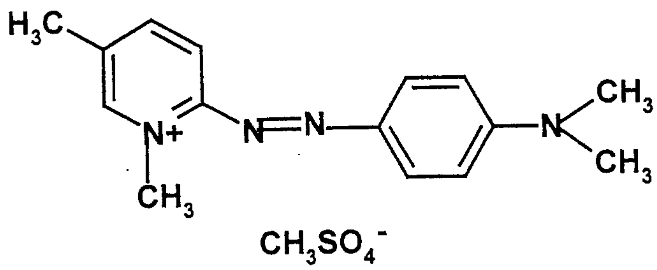

$(IX_{35})$

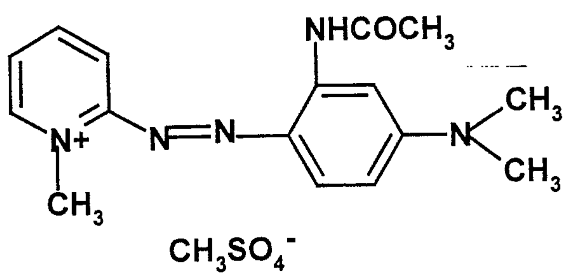

$(IX_{36})$

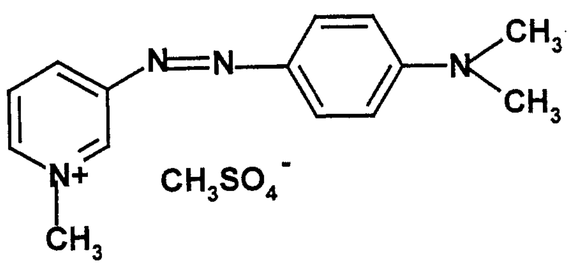

$(IX_{37})$

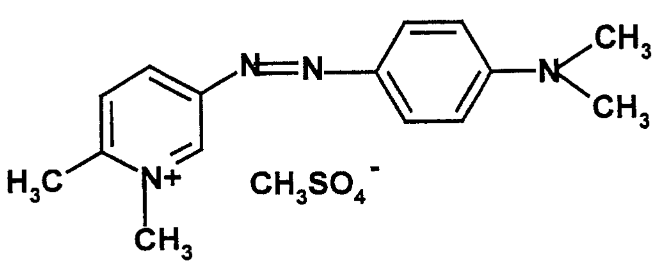

$(IX_{38})$

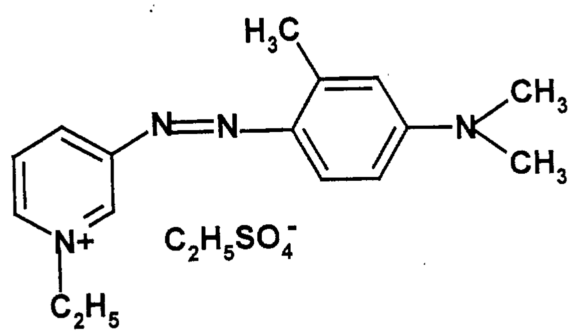

(IX39)

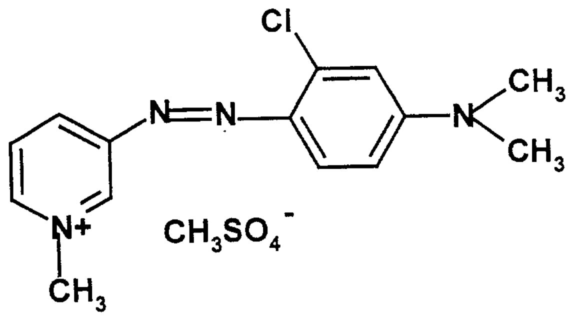

(IX40)

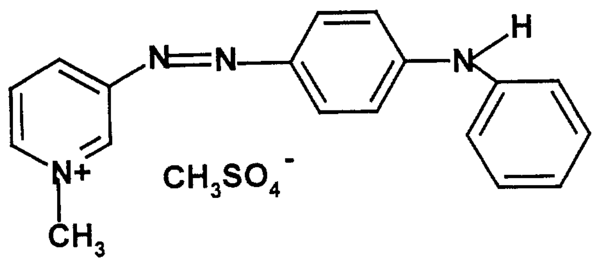

(IX41)

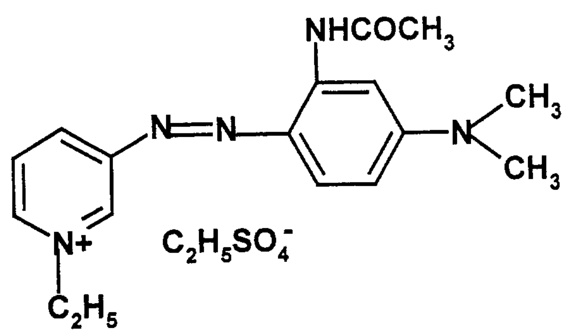

(IX42)

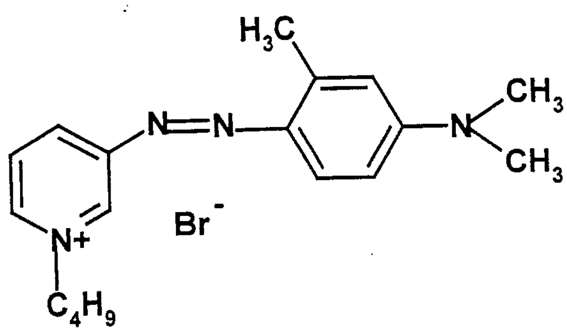

(IX43)

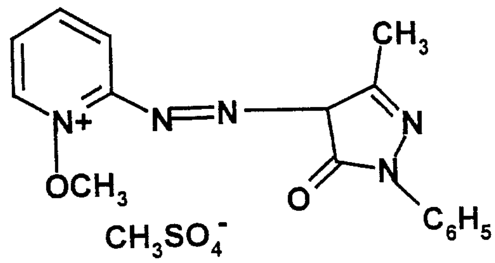

(IX44)

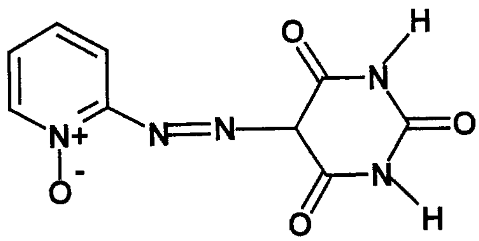

(IX45)

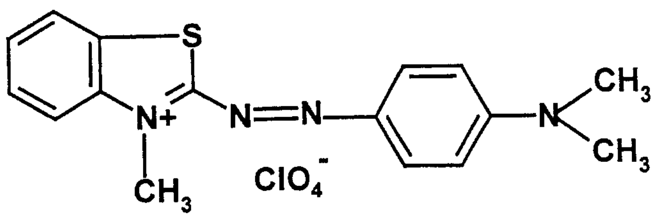

(IX46)

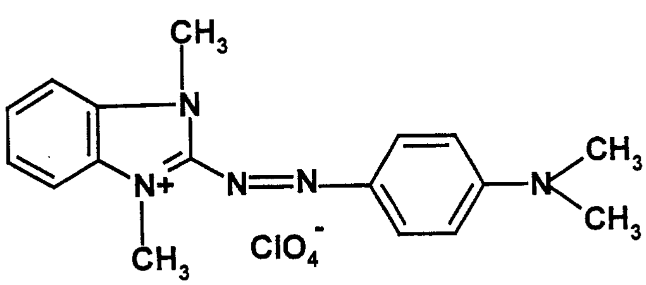

(IX47)

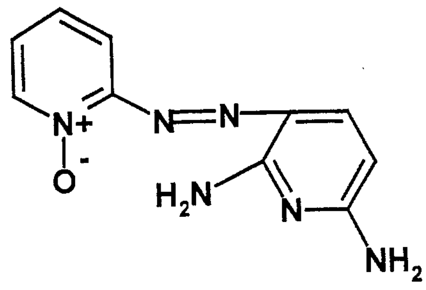

(IX48)

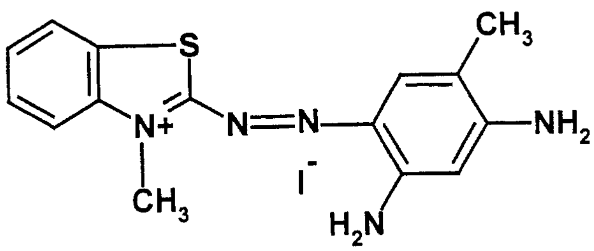

(IX49)

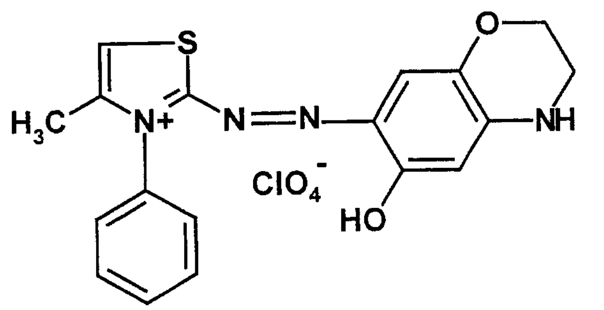

(IX50)

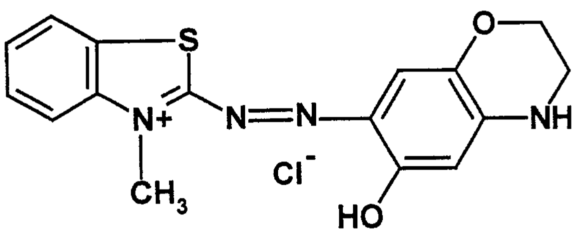

(IX51)

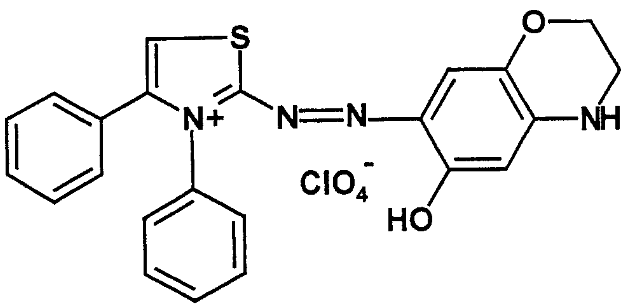

(IX52)

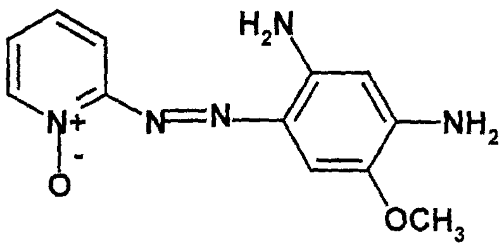

(IX$_{53}$)

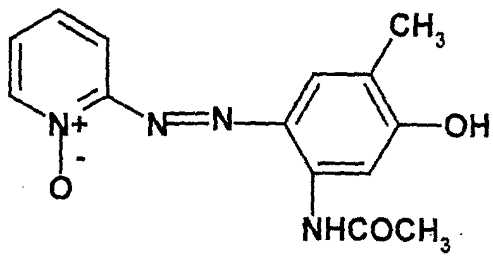

(IX$_{54}$)

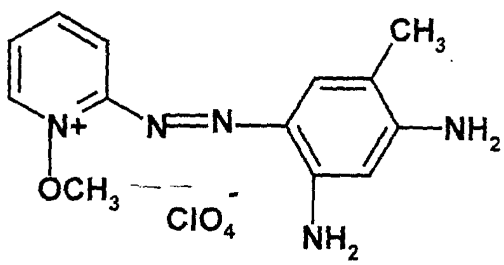

(IX$_{55}$)

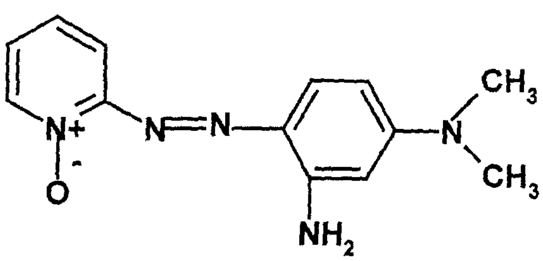

(IX$_{56}$)

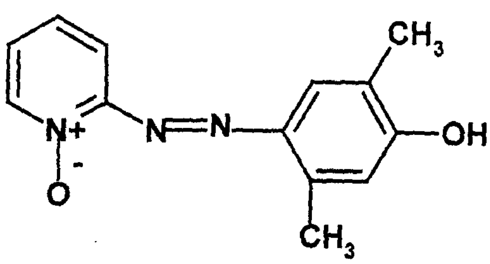

(IX$_{57}$)

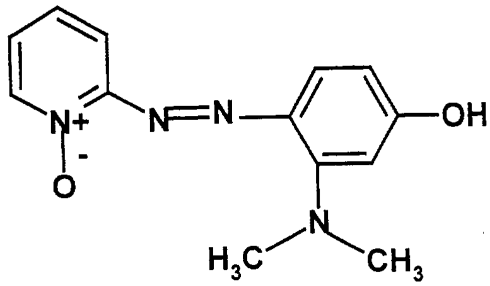

(IX58)

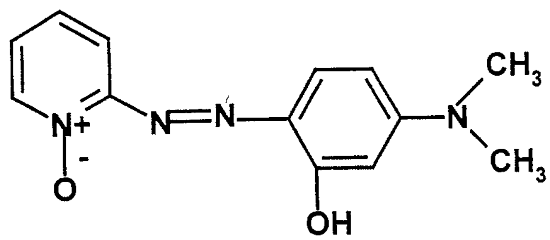

(IX59)

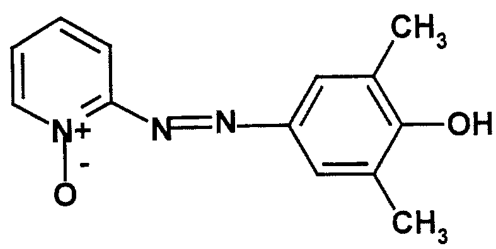

(IX60)

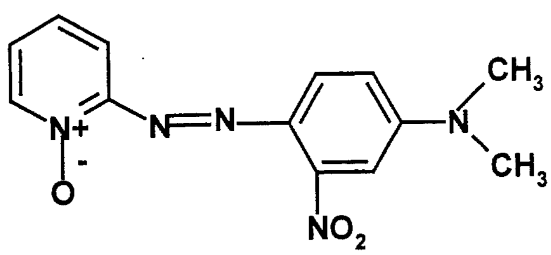

(IX61)

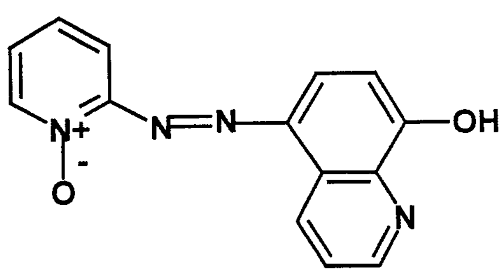

(IX62)

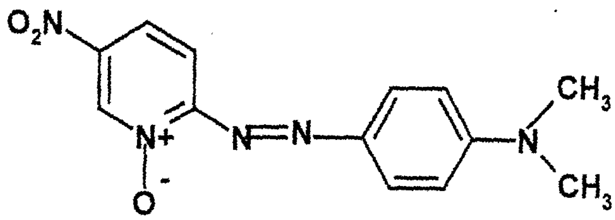

(IX63)

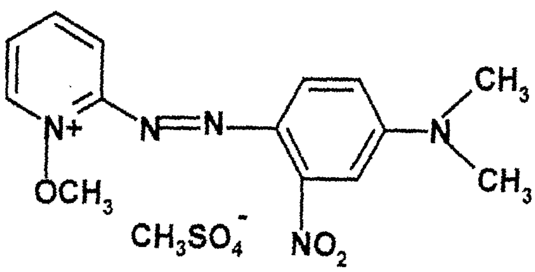

(IX64)

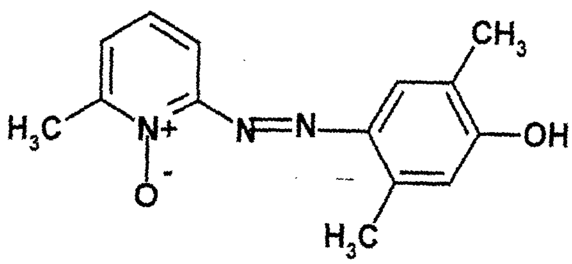

(IX65)

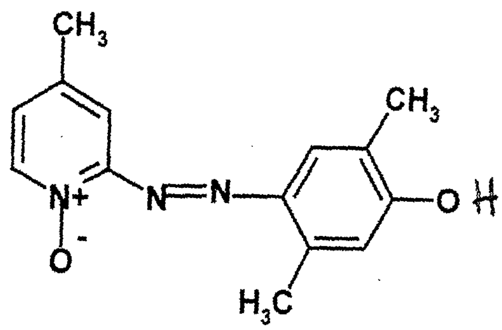

(IX66)

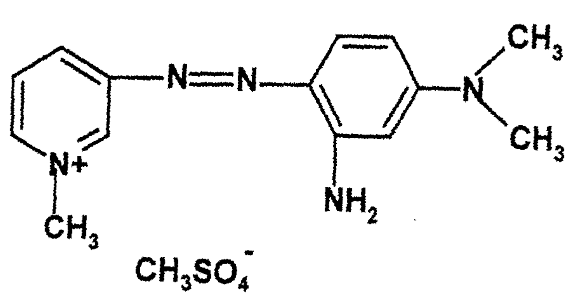

(IX67)

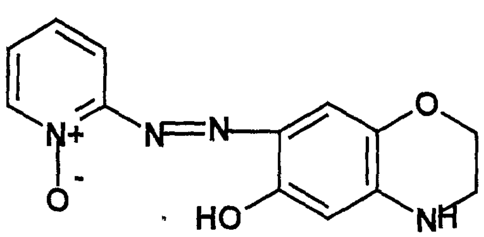

$(IX_{68})$

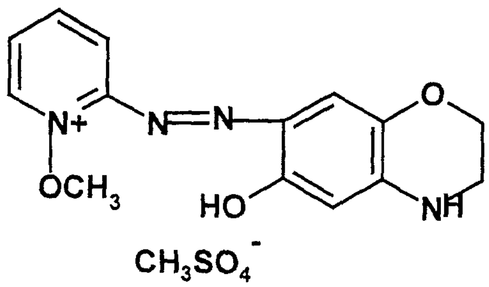

$(IX_{69})$

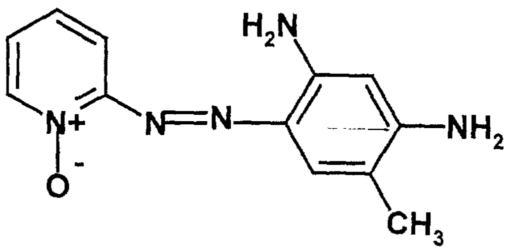

$(IX_{70})$

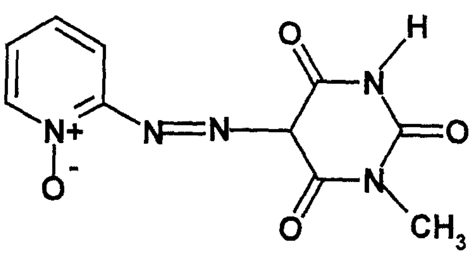

$(IX_{71})$

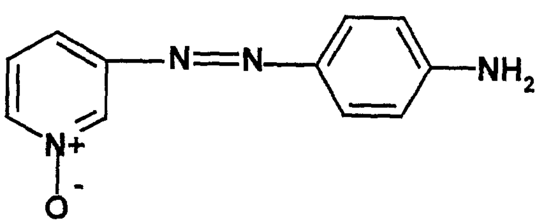

$(IX_{72})$

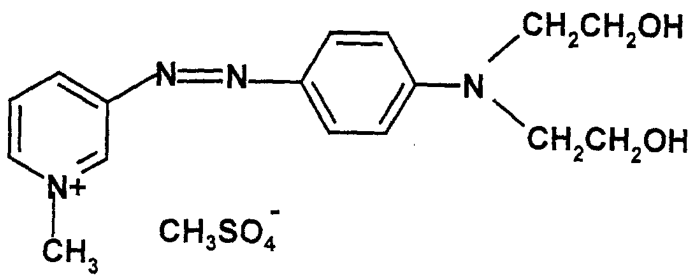

(IX73)

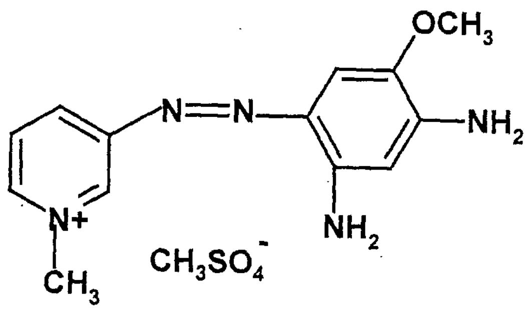

(IX74)

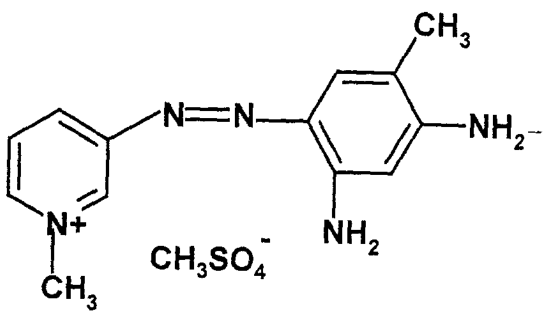

(IX75)

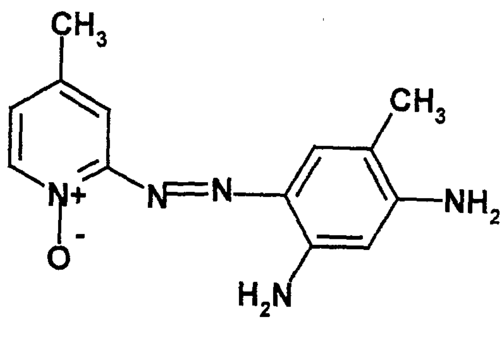

(IX76)

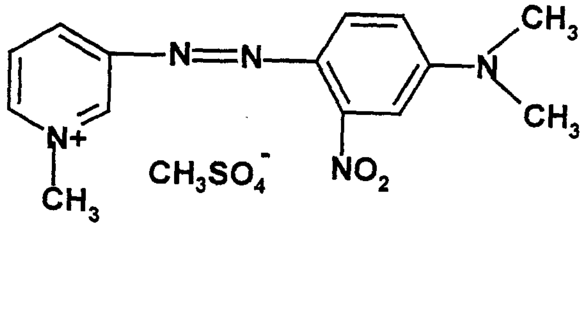

(IX77)

Le ou les colorants directs nitrés benzéniques et/ou les colorants directs cationiques selon l'invention représentent de 0,001 à 20% en poids environ du poids total de la composition et encore plus préférentiellement de 0,005 à 10% en poids environ.

De préférence, les compositions de l'invention contiennent au moins un coupleur. Parmi ces coupleurs, on peut notamment citer les métaphénylènediamines, les méta-aminophénols, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Le ou les coupleurs peuvent être présents dans la dite composition selon l'invention à une concentration comprise entre 0,0001 et 15% en poids par rapport au poids total de la composition.

La composition tinctoriale conforme à l'invention peut, en outre, renfermer au moins une base d'oxydation additionnelle différente des 1-(4-aminophényl)-pyrrolidines de formule (I) et/ou au moins un colorant direct différent de ceux de la présente invention.

Parmi les bases d'oxydation additionnelles utilisables selon l'invention, on peut citer la paraphénylènediamine, la paratoluylènediamine, la 2-hydroxyéthylparaphénylènediamine, la 1-N,N-bis(2-hydroxyéthyl)-paraphénylènediamine, les para-aminophénols tels que le 3-méthyl-4-aminophénol et le 4-aminophénol, les orthophénylènes diamines, les orthoaminophénols, les bases doubles, les bases hétérocycliques comme les pyrimidines telles que la 2,4,5,6-tétraaminopyrimidine ou comme les pyrazoles tel que le 1-(2-hydroxyéthyl)-4,5-diamino-pyrazole. La ou les bases d'oxydation additionnelles peuvent être présentes à une concentration comprise entre 0,0001 et 15% en poids par rapport au poids total de ladite composition.

Le milieu de la composition approprié pour la teinture est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 1 et 40% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des agents épaississants tels que les acides polyacryliques réticulés ou les hydroxyalkylcelluloses etc...., et des polymères cationiques.

Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant au moins un des motifs de formules (I), (II), (III) ou (IV) suivantes:

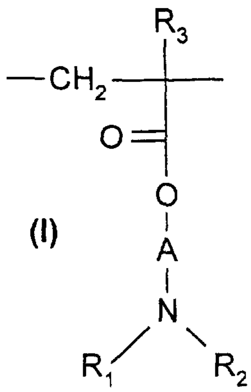

(I)

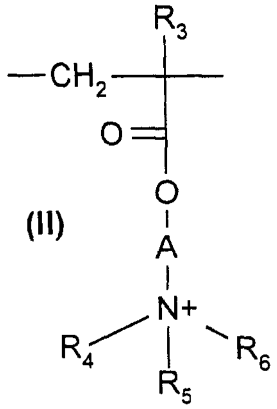

(II)

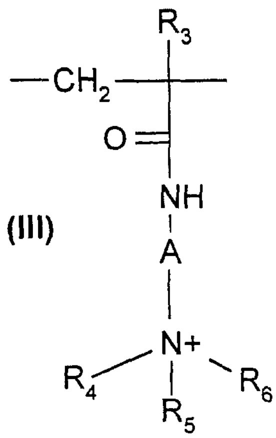

(III)

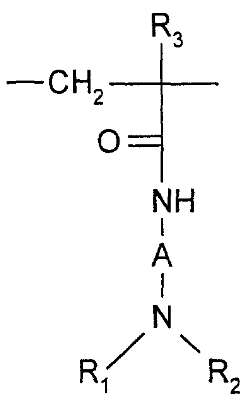

(IV)

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,

- les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination-"GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP.

**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
Les produits commercialisés répondant à cette définition sont plus particulièrement les produits vendus sous la dénomination "Celquat L 200" et "Celquat H 100" par la Société National Starch.

**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.

**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

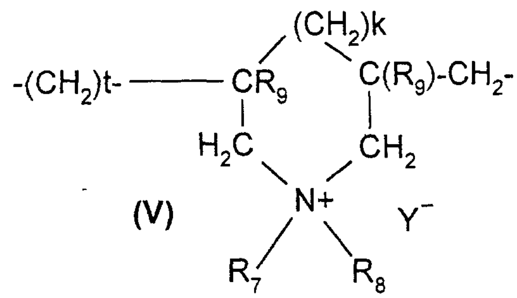

(V)

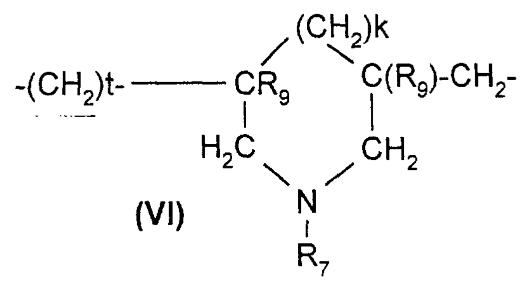

(VI)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

**(10)** Le polymère de diammonium quaternaire contenant des motifs récurrents répondant à la formule :

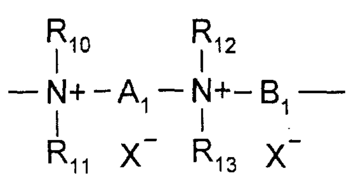

(VII)

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$,

$R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH- $R_{14}$-D où $R_{14}$ est un alkylène et D un groupement ammonium quaternaire ;

A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

$X^-$ désigne un anion dérivé d'un acide minéral ou organique;

A1, $R_{10}$ et $R_{12}$ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement $-(CH_2)n-CO-D-OC-(CH_2)n-$ dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH-.

De préférence, $X^-$ est un anion tel que le chlorure ou le bromure.

Ces polymères ont une masse moléculaire moyenne en nombre généralement comprise entre 1000 et 100000. Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante:

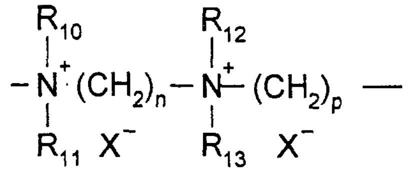

**(VIII)**

dans laquelle $R_{10}$, $R_{11}$, $R_{12}$ et $R_{13}$, identiques ou différents, désignent un radical alkyle ou hydroxyalkyle ayant de

1 à 4 atomes de carbone environ, n et p sont des nombres entiers variant de 2 à 20 environ et, $X^-$ est un anion dérivé d'un acide minéral ou organique.

**(11)** Les polymères de polyammonium quaternaire constitués de motifs récurrents de formule (IX) :

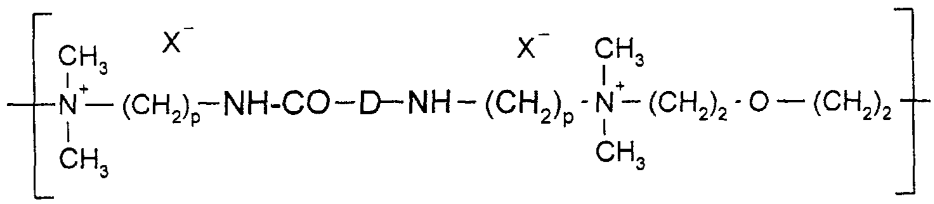

**(IX)**

dans laquelle p désigne un nombre entier variant de 1 à 6 environ, D peut être nul ou peut représenter un groupement $-(CH_2)_r-CO-$ dans lequel r désigne un nombre égal à 4 ou à 7, $X^-$ est un anion ;

De tels polymères peuvent être préparés selon les procédés décrits dans les brevets U.S.A. n° 4 157 388, 4 390 689, 4 702 906, 4 719 282. Ils sont notamment décrits dans la demande de brevet EP-A-122 324.

Parmi eux, on peut par exemple citer, les produits "Mirapol A 15", "Mirapol AD1", "Mirapol AZ1" et "Mirapol 175" vendus par la société Miranol.

**(12)** Les polymères quaternaires de vinylpyrrolidone et de vinylimidazole tels que par exemple les produits commercialisés sous les dénominations Luviquat FC 905, FC 550 et FC 370 par la société B.A.S.F.

**(13)** Les polyamines comme le Polyquart H vendu par HENKEL, référencé sous le nom de " POLYETHYLENEGLYCOL (15) TALLOW POLYAMINE " dans le dictionnaire CTFA.

**(14)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères de formules (W) et (U) suivantes :

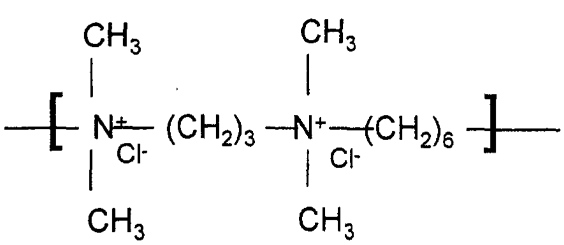

**(W)**

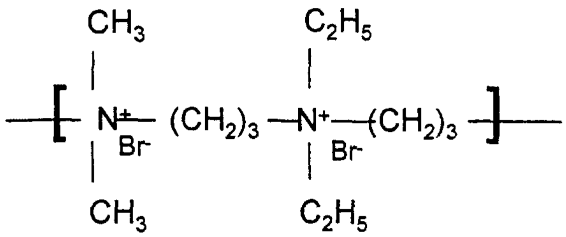 **(U)**

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.
Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.
La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras peuvent représenter de 0,001 à 20% en poids environ du poids total de la composition.
De préférence, la composition de l'invention contient au moins un tensioactif nonionique, anionique, cationique ou amphotère dans la proportion d'environ 0,1 à 20% en poids.
Encore plus préférentiellement ladite composition contient au moins un tensioactif nonionique.
Bien entendu, l'homme de l'art veillera à choisir le ou les éventuels composés complémentaires mentionnés ci-avant, de manière telle que les propriétés avantageuses attachées intrinsèquement à la composition de teinture d'oxydation selon l'invention ne soient pas, ou substantiellement pas, altérées par la ou les adjonctions envisagées.
Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.
Le pH de la composition colorante ou de la composition prête à l'emploi et appliquée sur les fibres kératiniques [composition résultant du mélange de la composition colorante selon l'invention et de la composition oxydante], est généralement compris entre les valeurs 3 et 12. Il est de préférence compris entre 6 et 11, et peut être ajusté à la valeur désirée au moyen d'agents acidifiants ou alcalinisants bien connus de l'état de la technique en teinture des fibres kératiniques.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (X) suivante :

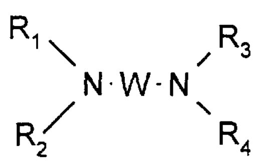 **(X)**

dans laquelle, W est un reste propylène éventuellement substitué par un groupement hydroxyle ou un radical alkyle en $C_1$-$C_4$ ; $R_1$, $R_2$, $R_3$ et $R_4$, identiques ou différents, représentent un atome d'hydrogène, un radical alkyle en $C_1$-$C_4$ ou hydroxyalkyle en $C_1$-$C_4$.
Les agents acidifiants sont classiquement, à titre d'exemple, des acides minéraux ou organiques comme

l'acide chlorhydrique, l'acide orthophosphorique, des acides carboxyliques comme l'acide tartrique, l'acide citrique, l'acide lactique, ou des acides sulfoniques.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

Le procédé de teinture selon l'invention consiste, de préférence, à appliquer la composition prête à l'emploi, réalisée extemporanément au moment de l'emploi à partir de la composition colorante selon l'invention et de la composition oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Les exemples qui suivent sont destinés à illustrer l'invention.

## EXEMPLES

On a préparé les compositions tinctoriales suivantes :
(exprimées en grammes)

| **EXEMPLES** | **1** invention | **2** art antérieur |
|---|---|---|
| Dichlorhydrate de 1-(4-aminophényl)-pyrrolidine (base d'oxydation selon l'invention) | 0,235 | |
| Dichlorhydrate de 2,4-diamino-1-(β-hydroxyéthyloxy)-benzène (coupleur) | 0,241 | 0,241 |
| Sulfate de N,N-bis(β-hydroxyéthyl)-paraphénylènediamine (base d'oxydation selon l'art antérieur) | | 0,196 |
| Colorant direct cationique de formule ($VI_2$)..[Basic Red 51] | 0,168 MA* | 0,168 MA* |
| Support de teinture (*) | qs | qs |
| Eau déminéralisée q.s.p | 100 | 100 |

(*) Support de teinture
- Alkyl $C_8$-$C_{10}$ polyglucoside en solution aqueuse à 60%, vendu sous la dénomination ORAMIX CG 110 ® par la société SEPPIC 3,24 g MA*
- Ethanol 18,0 g
- Alcool benzylique 1,8 g
- Polyéthylène glycol 400 2,7 g
- Sel pentasodique de l'acide diéthylène triamine pentacétique en solution aqueuse à 40%, vendu sous la dénomination DISSOLUINE D-40 ® par la société AKZO 0,43 g MA*
- Métabisulfite de sodium 0,205 g
- Ammoniaque à 20,5% de $NH_3$ 10,0 g

MA* désigne Matière Active

Au moment de l'emploi, on a mélangé poids pour poids chacune des compositions tinctoriales décrites ci-dessus avec une solution de peroxyde d'hydrogène à 20 volumes (6% en poids).

Les mélanges ainsi réalisés ont été appliqués pendant 30 minutes d'une part, sur des mèches de cheveux gris naturels permanentés à 90 % de blancs, d'autre part sur des mèches de cheveux gris naturels à 90 % de blancs. Les mèches ont ensuite été rincées, lavées avec un shampooing standard, rincées à nouveau puis séchées.

La couleur a ensuite été mesurée au colorimètre MINOLTA CM2002 dans le système L*a*b*.

Dans le système L*a*b* les 3 paramètres désignent respectivement l'intensité (L* ), la nuance (a* ), et la saturation (b*).

Selon ce système, plus la valeur de L est élevée, plus la couleur est claire ou peu intense. Inversement, plus la valeur de L est faible, plus la couleur est foncée ou très intense.

a* et b* indiquent deux axes de couleurs, a* indique l'axe de couleur vert/rouge et b* l'axe de couleur bleu/jaune. Des valeurs proches de zéro pour a* et b* correspondent à des nuances grises.

La sélectivité de la coloration ΔE peut être calculée en appliquant l'équation suivante :

$$\Delta E = \sqrt{(L^*-L_o^*)^2 + (a^*-a_o^*)^2 + (b^*-b_o^*)^2}$$

Dans cette équation, ΔE représente la différence de couleur entre deux mèches, (dans le cas présent la sélectivité de la coloration) , L* , a* , et b* représentent respectivement l'intensité, la nuance et la saturation de la

mèche de cheveux naturels teinte, $L_o$*, $a_o$* et $b_o$* représentant respectivement l'intensité, la nuance et la saturation de la mèche de cheveux permanentés teinte.

Plus la valeur de ΔE est importante, plus la différence de couleur entre les deux mèches est importante, et dans le cas présent, plus la sélectivité de la teinture est importante.

Les résultats ont été réunis dans le tableau (I) ci-dessous.

Tableau (I)

| EXEMPLES | Types de Cheveux | L* | a* | b* | **Sélectivité** (ΔE entre cheveux naturels et permanentés) |
|---|---|---|---|---|---|
| 2 | naturels | 38.25 | 10.81 | -2.72 | |
| 2 | permanentés | 29.55 | 14.25 | -11.15 | **12.59** |
| 1 | naturels | 20.36 | 6.96 | -9.34 | |
| 1 | permanentés | 17.50 | 3.09 | -4.76 | **6.64** |

Conclusion:

La teinture avec l'association selon l'invention (1) est presque deux fois moins sélective que celle de l'art antérieur (2) [6.64 contre 12.59].

La teinture avec l'association selon l'invention (1) est plus puissante que celle de l'art antérieur (2) [valeur de L plus basse].

## Revendications

1. Composition pour la teinture d'oxydation des fibres kératiniques et en particulier des fibres kératiniques humaines telles que les cheveux, comprenant dans un milieu approprié pour la teinture, (i)au moins une 1-(4-aminophényl)-pyrrolidine de formule (I) suivante à titre de précurseur de colorant d'oxydation :

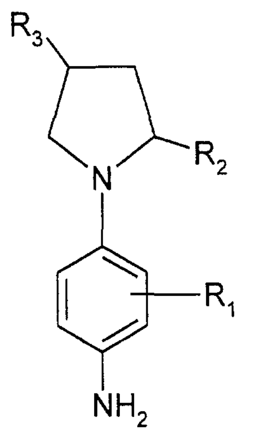

(I)

dans laquelle,

$R_1$ désigne un atome d'hydrogène, un radical alkyle en $C_1$-$C_6$, ou monohydroxyalkyle en $C_1$-$C_5$, ou polyhydroxyalkyle en $C_2$-$C_5$ ;
$R_2$ désigne un atome d'hydrogène, un radical -$CONH_2$, ou monohydroxyalkyle en $C_1$-$C_5$, ou polyhydroxyalkyle en $C_2$-$C_5$ ;
$R_3$ désigne un atome d'hydrogène, ou un radical OH ; et ses sels d'addition avec un acide,
**caractérisée par le fait qu'**elle comprend en outre au moins un colorant direct choisi dans le groupe formé par les colorants nitrés benzéniques et les colorants cationiques contenant un atome d'azote quaternisé et une liaison -Z=D- dans laquelle Z et D, identiques ou différents, désignent un atome d'azote ou un radical -CH-.

2. Composition selon la revendication 1, **caractérisée par le fait que** dans la formule (I), $R_1$, $R_2$, et $R_3$ désignent un

atome d'hydrogène.

**3.** Composition selon la revendication 1, **caractérisée par le fait que** dans la formule (I), $R_1$ et $R_3$ désignent un atome d'hydrogène et $R_2$ désigne le radical - $CH_2OH$.

**4.** Composition selon la revendication 1, **caractérisée par le fait que** dans la formule (I), $R_1$ désigne un atome d'hydrogène, $R_2$ désigne le radical $-CH_2OH$ et $R_3$ désigne le radical OH.

**5.** Composition selon la revendication 1, **caractérisée par le fait que** dans la formule (I), $R_1$ et $R_3$ désignent un atome d'hydrogène et $R_2$ désigne le radical $-CONH_2$.

**6.** Composition selon l'une quelconque des revendications précédentes, **caractérisée par le fait que** les sels d'addition avec un acide sont choisis parmi les chlorhydrates, les bromhydrates, les sulfates, les tartrates, les lactates et les acétates.

**7.** Composition selon l'une quelconque des revendications précédentes, **caractérisée par le fait que** les 1-(4-aminophényl)-pyrrolidines de formule (I) et leurs sels d'addition avec un acide représentent de 0,001 à 10 % % en poids du poids total de la composition.

**8.** Composition selon la revendication 7, **caractérisée par le fait que** les 1-(4-aminophényl)-pyrrolidines de formule (I) et leurs sels d'addition avec un acide représentent de 0,01 à 8% % en poids du poids total de la composition.

**9.** Composition selon l'une quelconque des revendications 1 à 8, **caractérisée par le fait que** le ou les colorants directs nitrés benzéniques sont de formule (II) suivante :

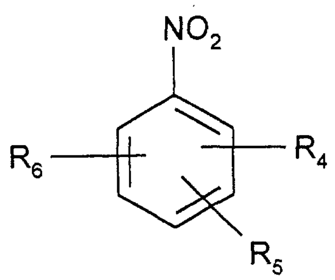

(II)

dans laquelle :

- $R_4$ représente un radical amino ; un radical amino monosubstitué ou disubstitué par un radical alkyle en $C_1$-$C_4$, monohydroxyalkyle en $C_1$-$C_4$, polyhydroxyalkyle en $C_2$-$C_4$, aminoalkyle en $C_1$-$C_4$, monoalkyl($C_1$-$C_4$)amino alkyle en $C_1$-$C_4$, dialkyl($C_1$-$C_4$)amino alkyle en $C_1$-$C_4$, uréidoalkyle en $C_1$-$C_4$, aryle, aryle dont le cycle aryle est substitué par un ou plusieurs radicaux hydroxyle, carboxyle, amino ou dialkyl($C_1$-$C_4$)amino,
- $R_5$ représente un atome d'hydrogène ; un radical amino ; hydroxyle ; alkyle en $C_1$-$C_4$ ; alcoxy en $C_1$-$C_4$ ; monohydroxyalkyle en $C_1$-$C_4$ ; polyhydroxyalkyle en $C_2$-$C_4$ ; monohydroxyalcoxy en $C_1$-$C_4$ ; polyhydroxyalcoxy en $C_2$-$C_4$ ; aminoalcoxy en $C_1$-$C_4$ ; un radical amino monosubstitué ou disubstitué par un radical alkyle en $C_1$-$C_4$, monohydroxyalkyle en $C_1$-$C_4$, polyhydroxyalkyle en $C_2$-$C_4$, aminoalkyle en $C_1$-$C_4$, monoalkyl($C_1$-$C_4$)amino alkyle en $C_1$-$C_4$, dialkyl($C_1$-$C_4$)amino alkyle en $C_1$-$C_4$, uréidoalkyle en $C_1$-$C_4$, aryle, aryle dont le cycle aryle est substitué par un ou plusieurs radicaux hydroxyle, carboxyle, amino ou dialkyl($C_1$-$C_4$)amino ;
- $R_6$ représente un atome d'hydrogène ou d'halogène, un radical alkyle en $C_1$-$C_4$, ou un groupement nitro.

**10.** Composition selon la revendication 9, **caractérisée par le fait que** le ou les colorants directs nitrés benzéniques sont choisis parmi :

- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,

- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- le 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyéthyl)amino 5-méthoxy nitrobenzène,
- le 2-amino 5-β-hydroxyéthyloxy nitrobenzène,
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β,γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-β,γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthyl)amino nitrobenzène,
- le 2-amino 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino 5-amino nitrobenzène,

- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-(β-aminoéthyl)amino 4-hydroxy nitrobenzène,
- l' [hydroxy-2 N-(β-hydroxyéthyl)amino-3 nitro-6] benzyloxy]-2 éthylamine, et
- l' [hydroxy-2 N-(β-hydroxypropyl)amino-3 nitro-6] benzyloxy]-2 éthylamine.

**11.** Composition selon la revendication 10, **caractérisée par le fait que** le ou les colorants directs nitrés benzéniques sont choisis parmi :

- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

**12.** Composition selon l'une quelconque des revendications 1 à 8, **caractérisée par le fait que** le ou les colorants directs cationiques sont choisis parmi les composés de formules (III), (IV) et (V) suivantes :

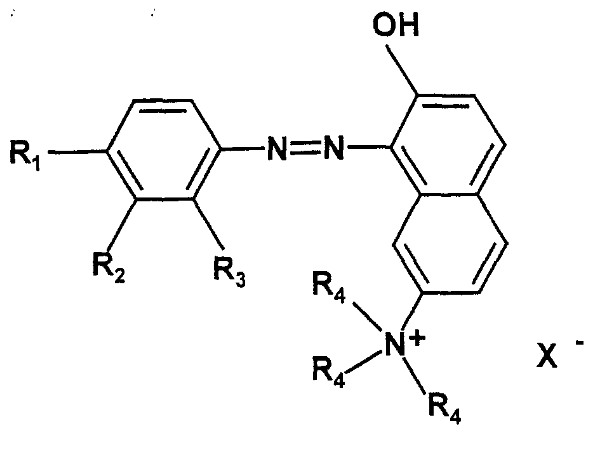

(III)

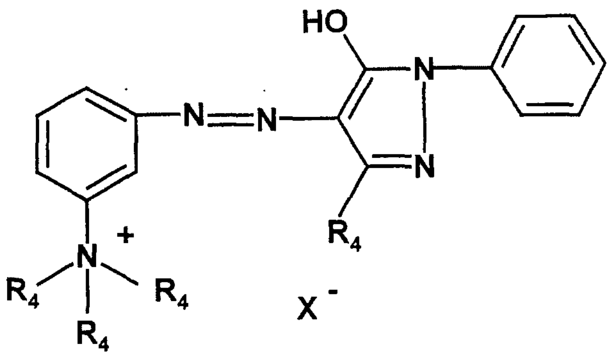

(IV)

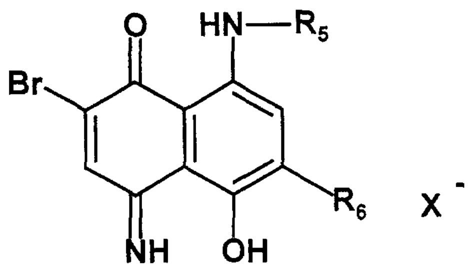

(V)

formules (III) à (V) dans lesquelles :

$R_1$ représente un atome d'hydrogène ou un radical amino ;
$R_2$ représente un atome d'hydrogène ou un groupement nitro ;
$R_3$ représente un atome d'hydrogène , un groupement nitro ou un radical alcoxy en $C_1$-$C_4$ ;
$R_4$ représente un radical alkyle en $C_1$-$C_4$ ;
$R_5$ représente un atome d'hydrogène ou un groupement paratrialkyl$C_1$-$C_4$ ammoniophényle ;
$R_6$ représente un atome de brome ou un groupement NHparatrialkyle$C_1$-$C_4$ ammoniophényle ;
X- représente un anion ; et les formes mésomères de ces structures (III) à (V).

**13.** Composition selon la revendication 12, **caractérisée par le fait que** les colorants sont choisis parmi les :

. 8-[(4-aminophényl)azo]-7-hydroxy-2- triméthylammonio-naphtalène,
. 8-[(2-méthoxyphényl)azo]-7-hydroxy-2-triméthylammonio-naphtalène,
. 8-[(4-amino-3-nitrophényl)azo]-7-hydroxy-2-triméthylammonio-naphtalène,
. 8-[(4-amino-2-nitrophényl)azo]-7-hydroxy-2-triméthylammonio-naphtalène,
. 3-[(3-méthyl-5-hydroxy-1-phényl-1H-pyrazol-4-yl)azo]-triméthylammonio-benzène,
. 3-[(4-amino-6-bromo-5,8-dihydro-1-hydroxy-8-imino-5-oxo-2-naphtalényl)-amino]-triméthylammonio-benzène,
. 3-[(3,7-dibromo-5,8-dihydro-4-hydroxy-5-imino-8-oxo-1-naphtalényl)- amino]-triméthylammonio-benzène.

**14.** Composition selon l'une quelconque des revendications 1 à 8, **caractérisée par le fait que** le ou les colorants directs cationiques sont choisis parmi les composés de formules (VI), (VII), (VIII), (VIII') et (IX) suivantes :

**a) les composés de formule (VI) suivante :**

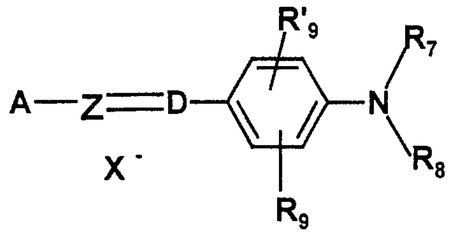

(VI)

dans laquelle :

Z et D représentent, identiques ou différents, un atome d'azote ou le radical -CH-,

$R_7$ et $R_8$, identiques ou différents, représentent un atome d'hydrogène ; un radical 4'-aminophényle, un radical alkyle en $C_1$-$C_4$ pouvant être substitué par un radical -CN, -OH ou -$NH_2$ ou forment avec un atome de carbone du cycle benzénique un hétérocycle azoté pouvant contenir un ou plusieurs atomes d'oxygène et/ou un ou plusieurs autres atomes d'azote, et pouvant être substitué par un ou plusieurs radicaux alkyle en $C_1$-$C_4$ ;

$R_9$ et $R'_9$, identiques ou différents, représentent un atome d'hydrogène ou d'halogène choisi parmi le chlore, le brome, l'iode et le fluor, un radical cyano, alkyl en $C_1$-$C_4$, alcoxy en $C_1$-$C_4$ ou acétyloxy,

$X^-$ représente un anion ;

A représente un groupement choisi par les structures A1 à A19 suivantes :

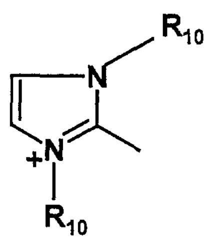

$A_1$ ;

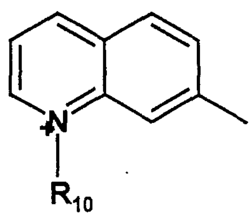

$A_2$ ;

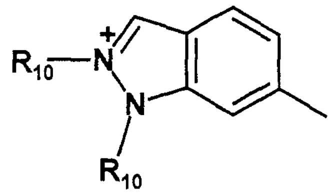

$A_3$ ;

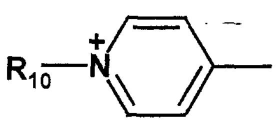

$A_4$ ;

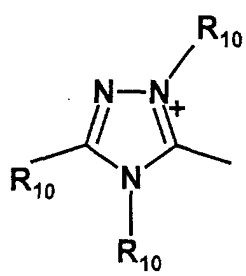

$A_5$ ;

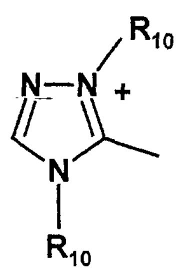

$A_6$ ;

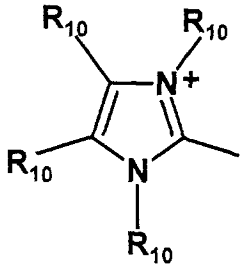

A7

;

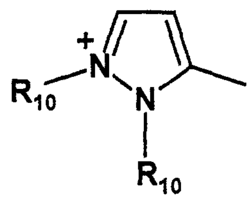

A8

;

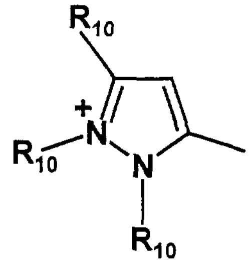

A9

;

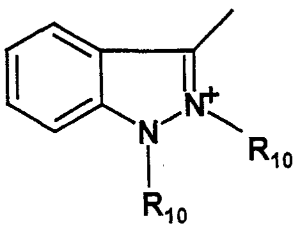

A10

;

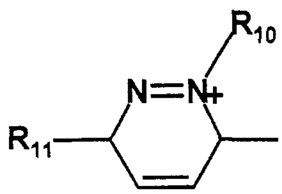

A11

;

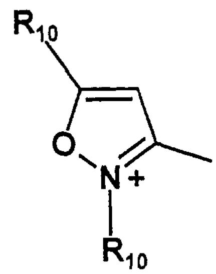

A12

;

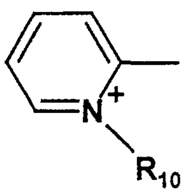

A13

;

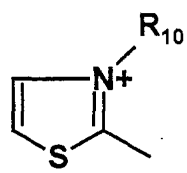

A14

;

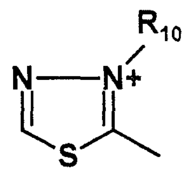

A15

;

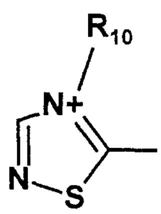

A16

;

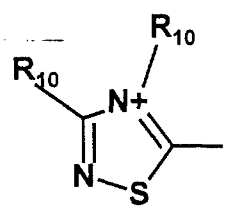

A17

;

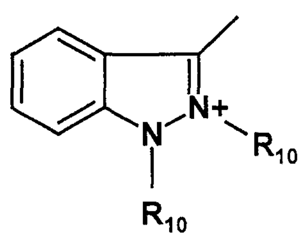

A18

et

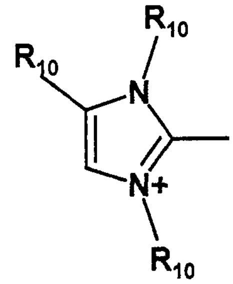

A19

;

dans lesquelles $R_{10}$ représente un radical alkyle en $C_1$-$C_4$ pouvant être substitué par un radical hydroxyle et $R_{11}$ représente un radical alcoxy en $C_1$-$C_4$;

**b) les composés de formule (VII) suivante :**

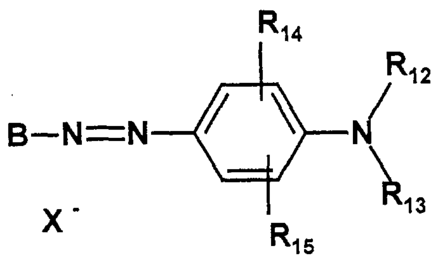

(VII)

dans laquelle :

$R_{12}$ représente un atome d'hydrogène ou un radical alkyle en $C_1$-$C_4$,
$R_{13}$ représente un atome d'hydrogène, un radical alkyle pouvant être substitué par un radical -CN ou par un groupement amino, un radical 4'-aminophényle, ou forme avec $R_{12}$ un hétérocycle éventuellement oxygéné et/ou azoté pouvant être substitué par un radical alkyle en $C_1$-$C_4$,
$R_{14}$ et $R_{15}$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical -CN,
$X^-$ représente un anion ;
B représente un groupement choisi par les structures B1 à B6 suivantes :

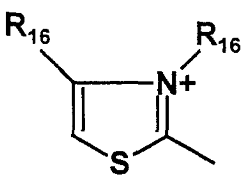

B1

;

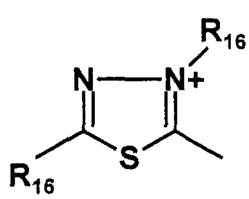

B2

;

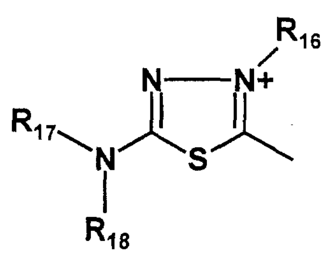

B3

;

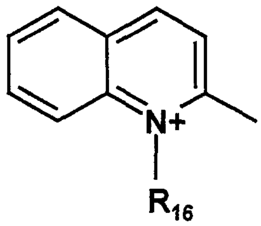 ; 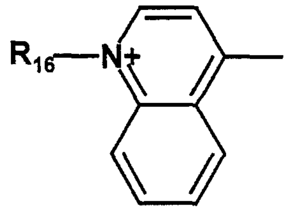 et 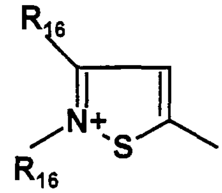 ;

B4 B5 B6

dans lesquelles $R_{16}$ représente un radical alkyle en $C_1$-$C_4$, $R_{17}$ et $R_{18}$, identiques ou différents, représentent un atome d'hydrogène ou un radical alkyle en $C_1$-$C_4$ ;

**c) les composés de formules (VIII) et (VIII') suivantes :**

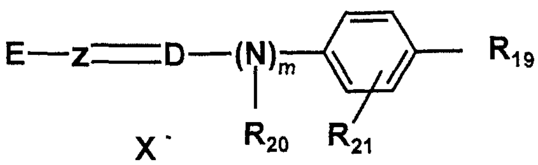

(VIII)

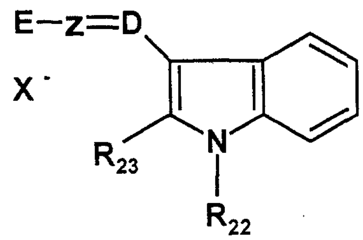

(VIII')

dans lesquelles :

Z et D, identiques ou différents, représentent un atome d'azote ou le radical -CH-,
$R_{19}$ représente un atome d'hydrogène, un radical alcoxy en $C_1$-$C_4$, un atome d'halogène tel que le brome, le chlore, l'iode ou le fluor ou un radical amino,
$R_{20}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,
$R_{21}$ représente un atome d'hydrogène ou d'halogène tel que le brome, le chlore, l'iode ou le fluor,
$R_{22}$ et $R_{23}$, identiques ou différents, représentent un atome d'hydrogène ou un radical alkyle en $C_1$-$C_4$,
m = 0 ou 1,
$X^-$ représente un anion ;
E représente un groupement choisi par les structures E1 à E8 suivantes :

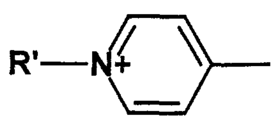 ; 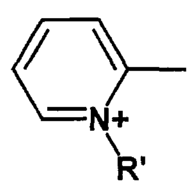 ;

E1 E2

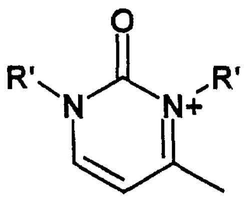

E3

;

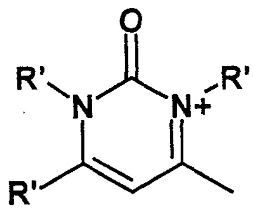

E4

;

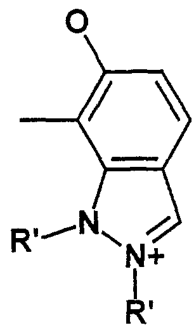

E5

;

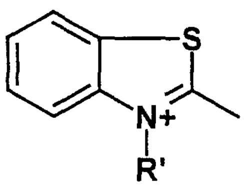

E6

;

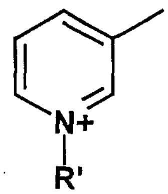

E7

et

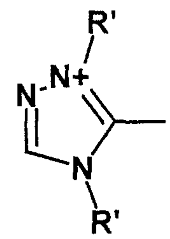

E8

;

dans lesquelles,
R' représente un radical alkyle en $C_1$-$C_4$ ;

lorsque m = 0 et que $D_1$ représente un atome d'azote, alors E peut également désigner un groupement de structure E9 suivante :

E9

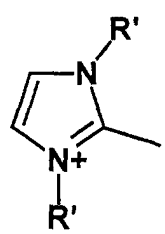

;

dans laquelle R' représente un radical alkyle en $C_1$-$C_4$.

**d) les composés de formule (IX) suivante :**

**G-N=N-J (IX)**

dans laquelle :

**le symbole G** représente un groupement choisi parmi les structures $G_1$ à $G_3$ suivantes :

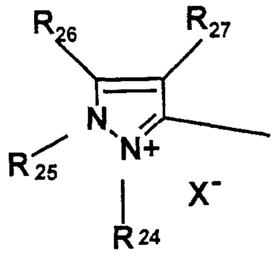

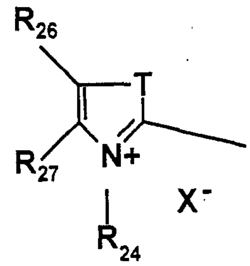

$G_1$ $G_2$

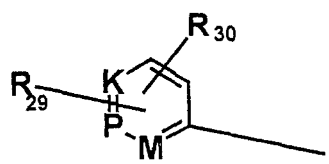

$G_3$

structures $G_1$ à $G_3$ dans lesquelles,

$R_{24}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{25}$ désigne un radical alkyle en $C_1$-$C_4$ ou un radical phényle;
$R_{26}$ et $R_{27}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$ , alcoxy en $C_1$-$C_4$, ou $NO_2$, ou forment ensemble dans $G_2$ un cycle benzénique éventuellement substitué par un ou plusieurs radicaux alkyle en $C_1$-$C_4$, alcoxy en $C_1$-$C_4$, ou $NO_2$;
$R_{26}$ peut désigner en outre un atome d'hydrogène;
T désigne un atome d'oxygène, de soufre ou un groupement $-NR_{25}$;
M représente un groupement -CH, -CR (R désignant alkyle en $C_1$-$C_4$), ou $-NR_{28}(X^-)_r$;
K représente un groupement -CH, -CR (R désignant alkyle en $C_1$-$C_4$), ou $-NR_{28}(X^-)_r$;
P représente un groupement -CH, -CR (R désignant alkyle en $C_1$-$C_4$), ou $-NR_{28}(X^-)_r$; r désigne zéro ou 1;
$R_{28}$ représente un atome $O^-$, un radical alcoxy en $C_1$-$C_4$, ou un radical alkyle en $C_1$-$C_4$;
$R_{29}$ et $R_{30}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$, un radical $-NO_2$;
$X^-$ représente un anion ;
**le symbole J** représente :

- **(a)** un groupement de structure $J_1$ suivante :

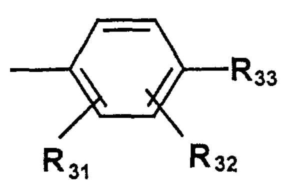

$J_1$

structure $J_1$ dans laquelle,

$R_{31}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{34}$, $-NR_{35}R_{36}$, -NHCOalkyle en $C_1$-$C_4$, ou forme avec $R_{32}$ un cycle à 5 ou 6 chaînons contenant ou non un ou plusieurs hétéroatomes choisis parmi l'azote, l'oxygène ou le soufre;
$R_{32}$ représente un atome d'hydrogène, un atome d'halogène choisi parmi le chlore, le brome, l'iode et le fluor, un radical alkyle en $C_1$-$C_4$, alcoxy en $C_1$-$C_4$ ou forme avec $R_{33}$ ou $R_{34}$ un cycle à 5 ou 6 chaînons contenant ou non un ou plusieurs hétéroatomes choisis parmi l'azote, l'oxygène ou le soufre;
$R_{33}$ représente un atome d'hydrogène, un radical -OH, un radical $-NHR_{34}$, un radical $-NR_{35}R_{36}$;
$R_{34}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;
$R_{35}$ et $R_{36}$, identiques ou différents, représentent un radical alkyle en $C_1$-$C_4$ , un radical monohydroxyalkyle en $C_1$-$C_4$, polyhydroxyalkyle en $C_2$-$C_4$;

- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle, et notamment un groupement de structure $J_2$ suivante :

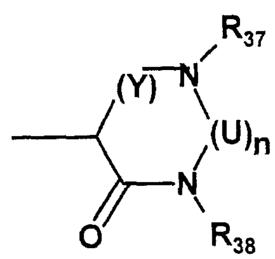

$J_2$

structure $J_2$ dans laquelle,

$R_{37}$ et $R_{38}$, identiques ou différents, représentent un atome d'hydrogène , un radical alkyle en $C_1$-$C_4$, un radical phényle;

Y désigne le radical -CO- ou le radical

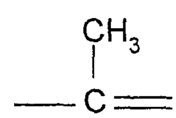 ;

n = 0 ou 1, avec, lorsque n désigne 1, U désigne le radical -CO- .

et les formes mésomères de ces structures (VI) à (IX).

**15.** Composition selon la revendication 14, **caractérisée par le fait que** les colorants cationiques de formule (VI) sont choisis parmi ceux de structures ($VI_1$) à ($VI_{54}$) suivantes :

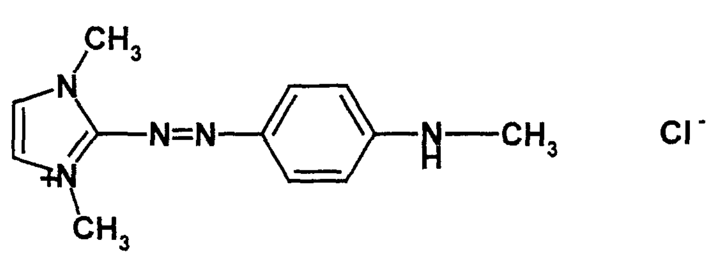

($VI_1$)

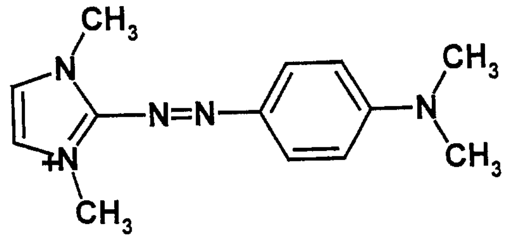

$Cl^-$ (VI$_2$)

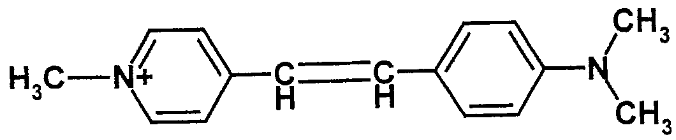

$Cl^-$ (VI$_3$)

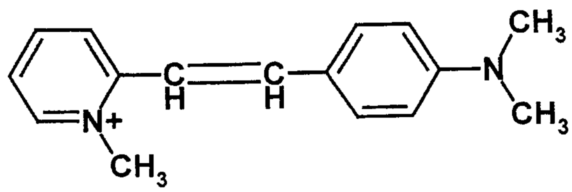

$Cl^-$ (VI$_4$)

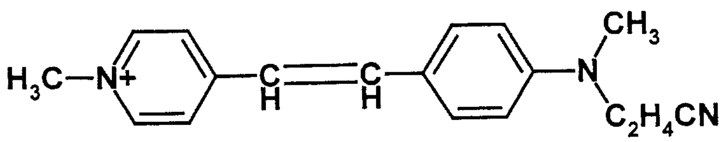

$Cl^-$ (VI$_5$)

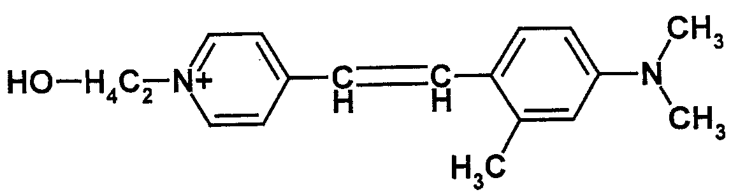

$Cl^-$ (VI$_6$)

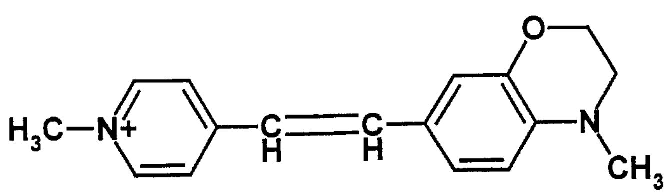

$Cl^-$ (VI$_7$)

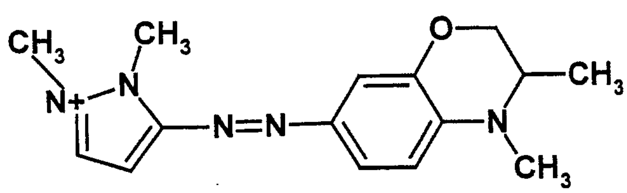

$Cl^-$ (VI$_8$)

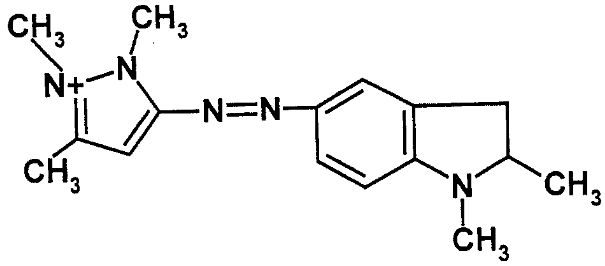

Cl -

(VI$_9$)

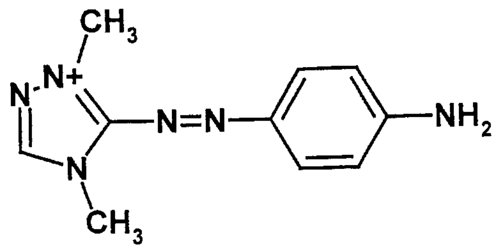

Cl -

(VI$_{10}$)

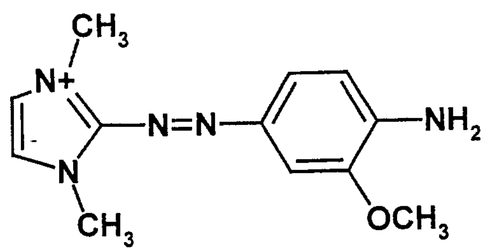

Cl -

(VI$_{11}$)

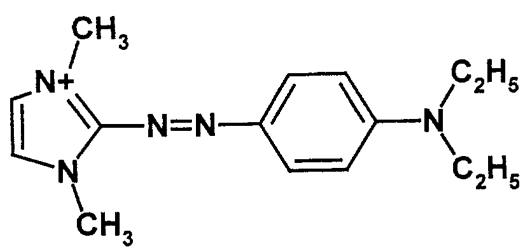

Cl -

(VI$_{12}$)

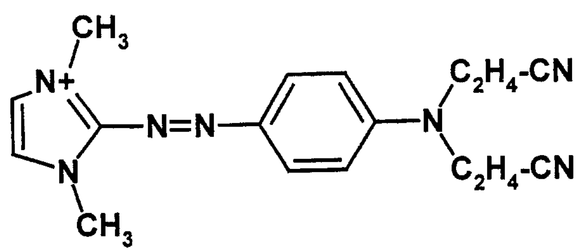

Cl -

(VI$_{13}$)

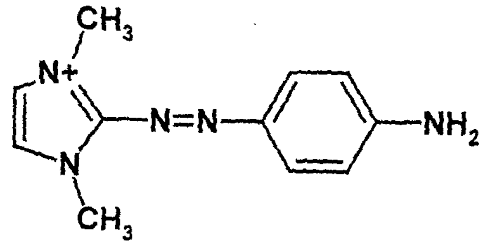

Cl ⁻ (VI$_{14}$)

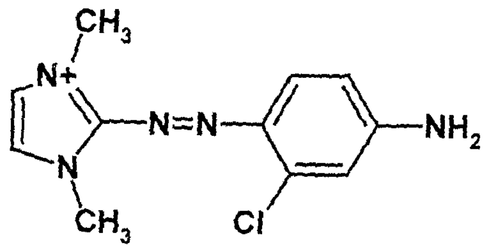

Cl ⁻ (VI$_{15}$)

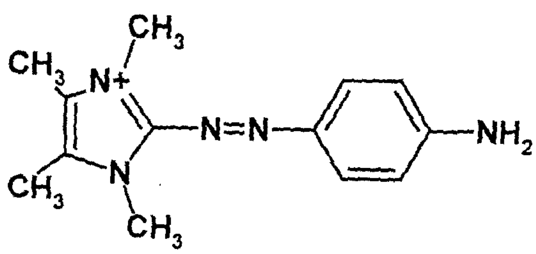

Cl ⁻ (VI$_{16}$)

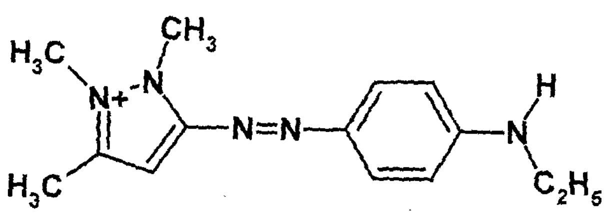

Cl ⁻ (VI$_{17}$)

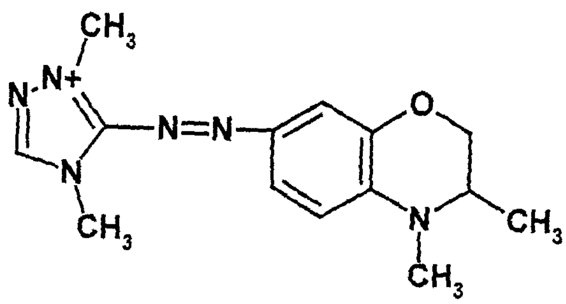

Cl ⁻ (VI$_{18}$)

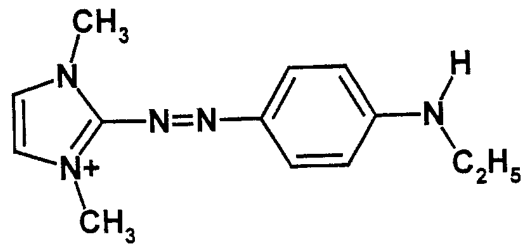

Cl -

(VI19)

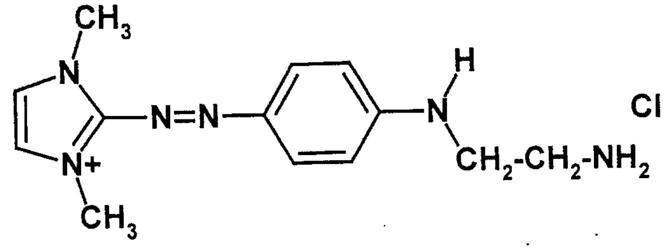

Cl -

(VI20)

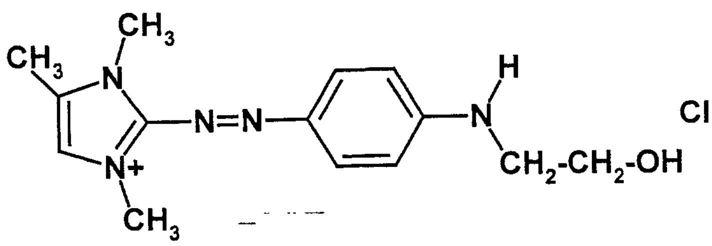

Cl -

(VI21)

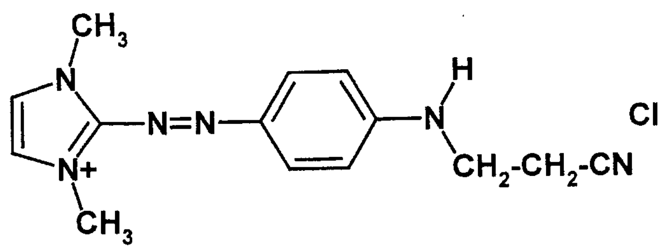

Cl -

(VI22)

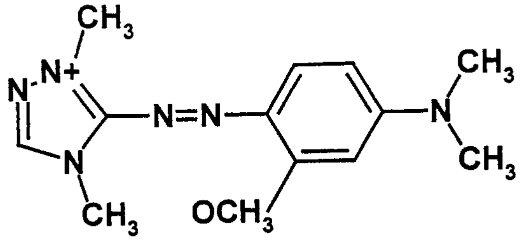

Cl -

(VI23)

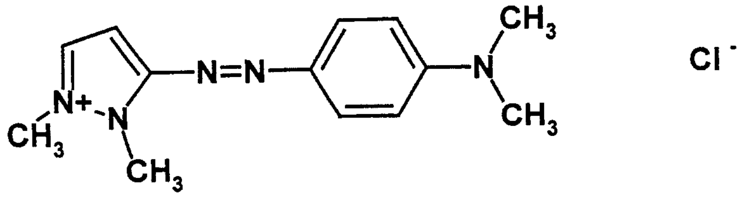

(VI24)

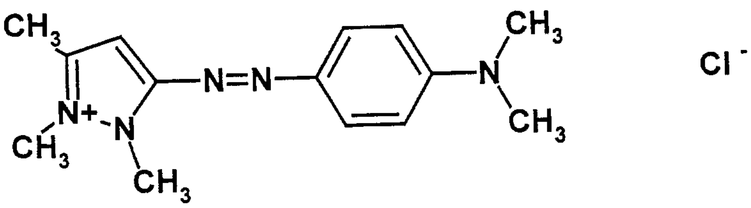

(VI25)

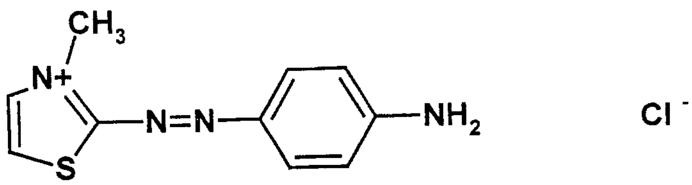

(VI26)

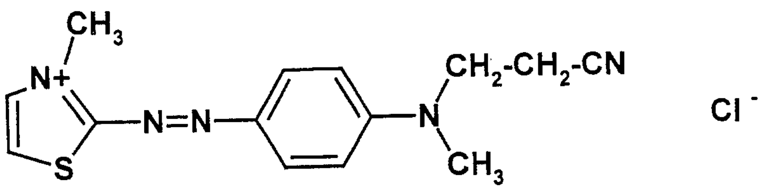

(VI27)

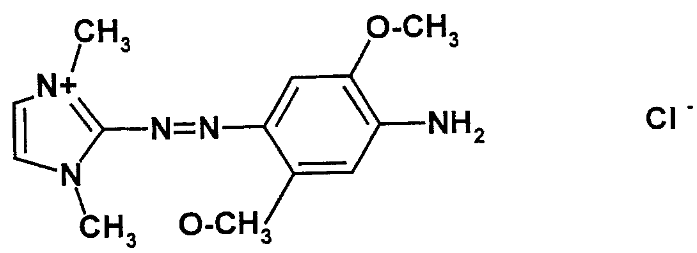

(VI28)

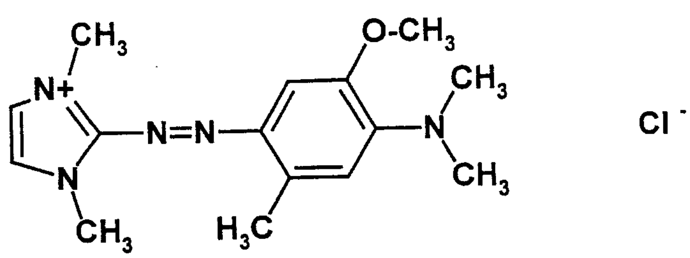

(VI29)

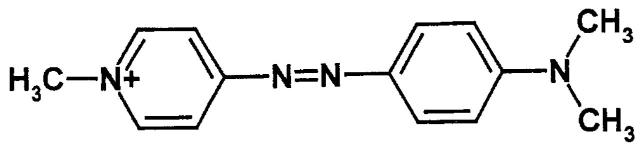

$Cl^-$ ($VI_{30}$)

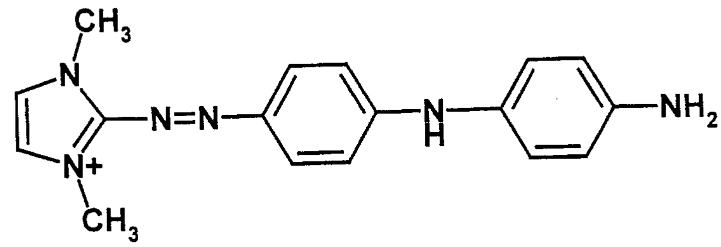

$Cl^-$ ($VI_{31}$)

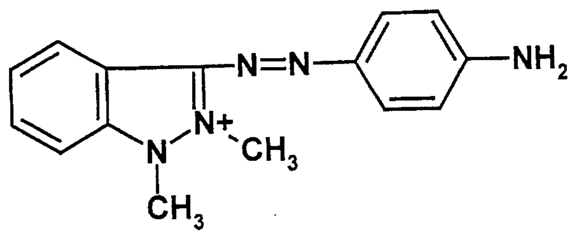

$Cl^-$ ($VI_{32}$)

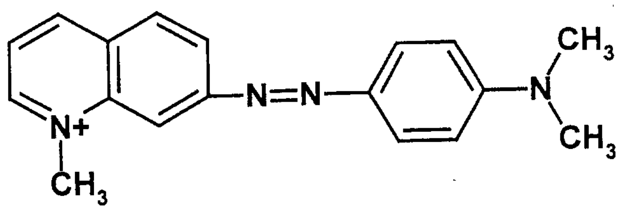

$Cl^-$ ($VI_{33}$)

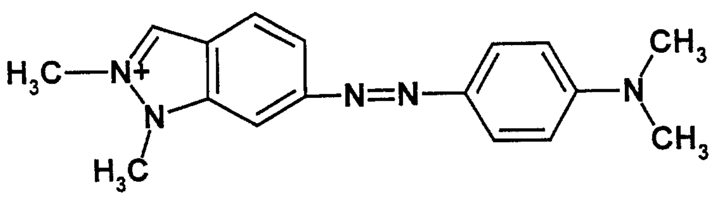

$Cl^-$ ($VI_{34}$)

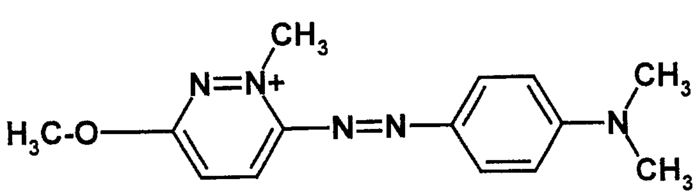

$Cl^-$ ($VI_{35}$)

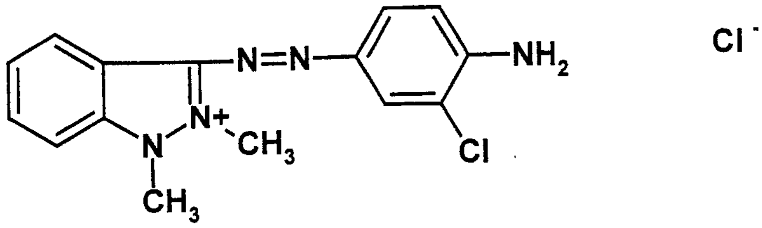

$(VI_{36})$

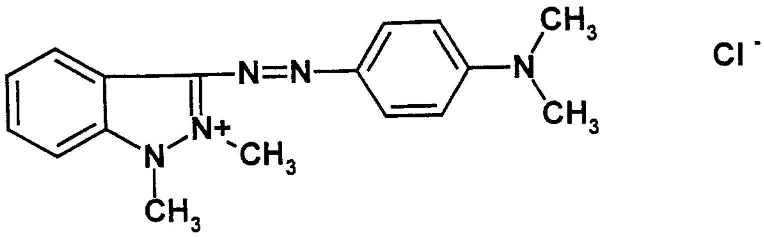

$(VI_{37})$

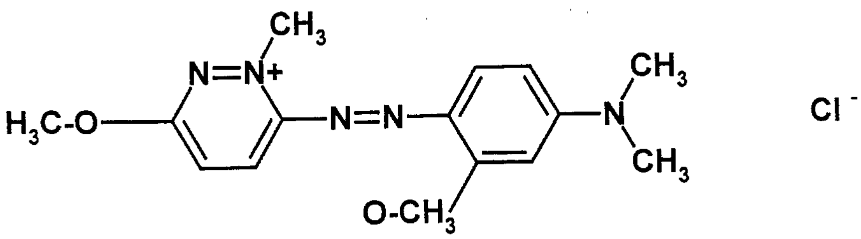

$(VI_{38})$

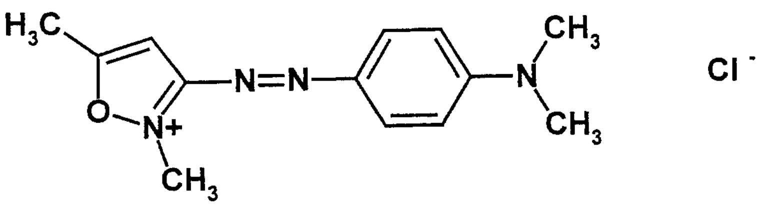

$(VI_{39})$

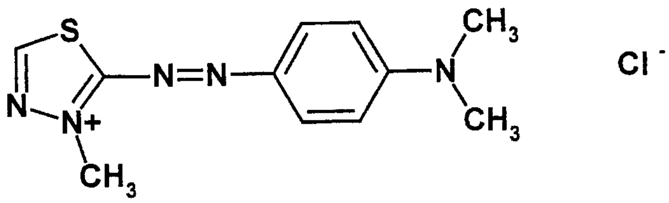

$(VI_{40})$

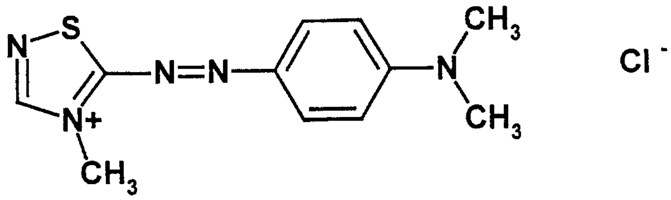

$(VI_{41})$

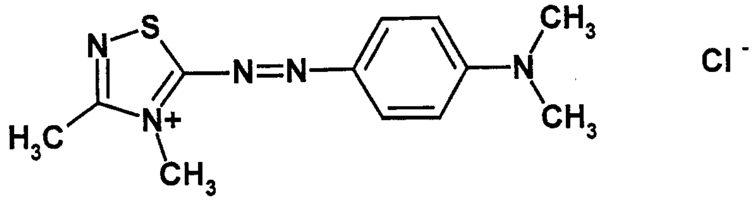

$(VI_{42})$

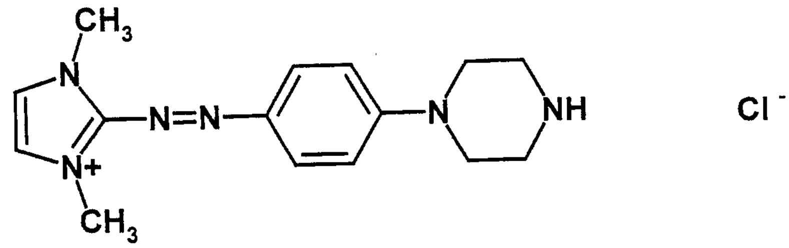

$(VI_{43})$

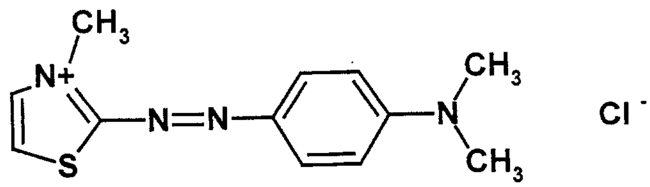

$(VI_{44})$

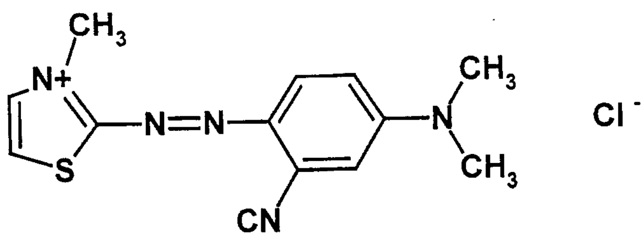

$(VI_{45})$

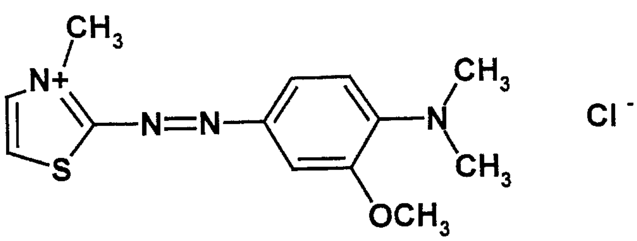

$(VI_{46})$

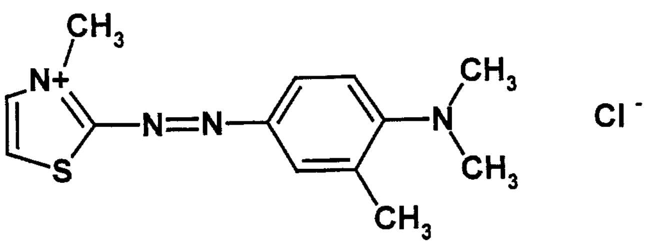

$(VI_{47})$

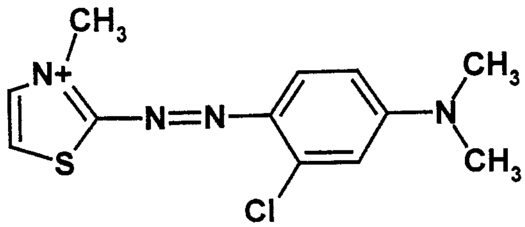

$CH_3SO_4^-$ (VI48)

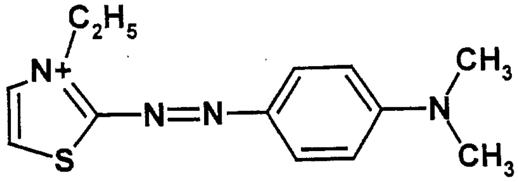

$CH_3SO_4^-$ (VI49)

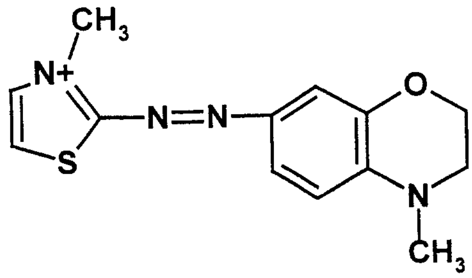

$Cl^-$ (VI50)

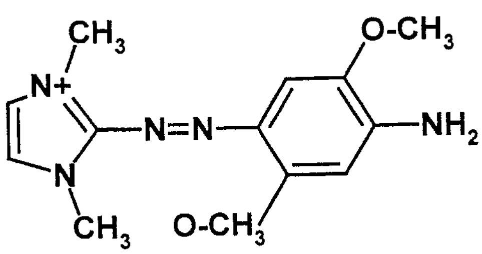

$Cl^-$ (VI51)

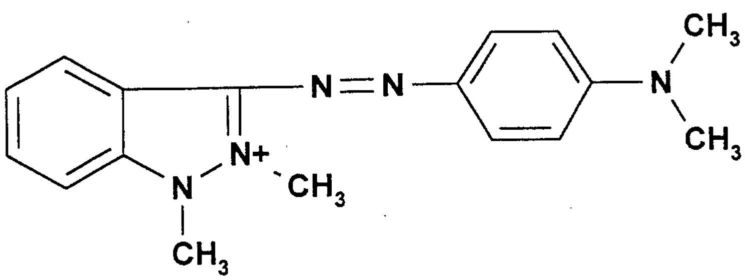

$Cl^-$ (VI52)

;

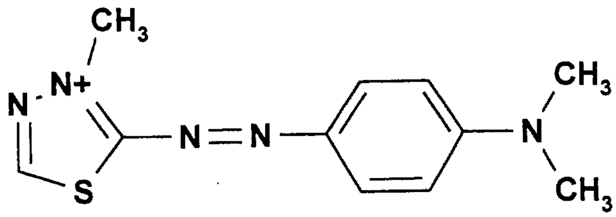

$CH_3SO_4^-$ (VI$_{53}$)

;

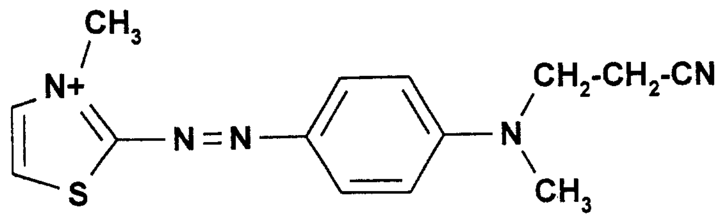

$Cl^-$ (VI$_{54}$)

**16.** Composition selon la revendication 14, **caractérisée par le fait que** les colorants cationiques de formule (VII) sont choisis parmi ceux de structures (VII$_1$) à (VII$_9$) suivantes :

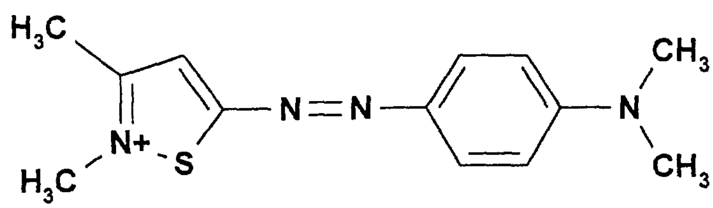

$Cl^-$ (VII$_1$)

;

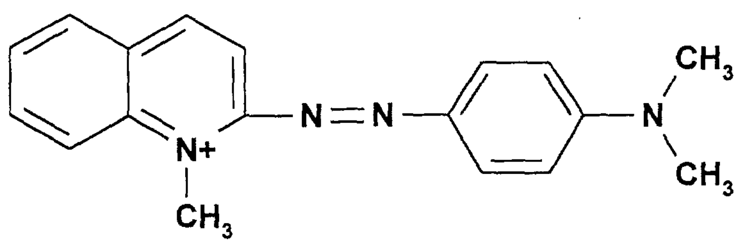

$Cl^-$ (VII$_2$)

;

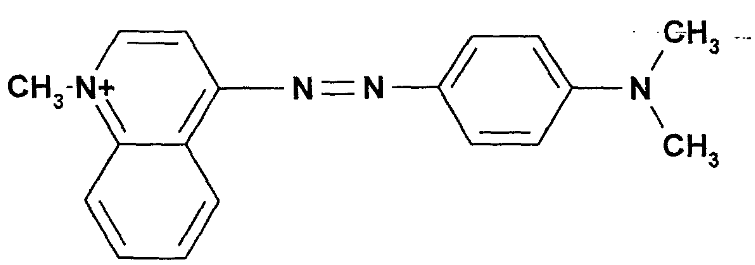

$Cl^-$ (VII$_3$)

;

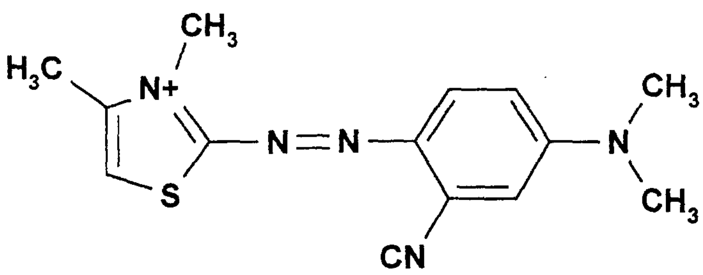 $Cl^-$ (VII$_4$)

;

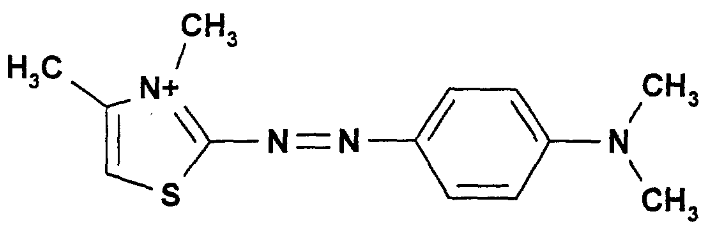 $CH_3SO_4^-$ (VII$_5$)

;

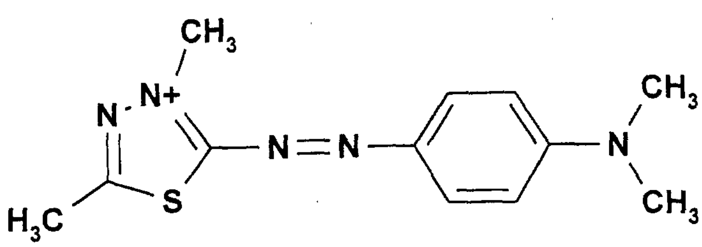 $CH_3SO_4^-$ (VII$_6$)

;

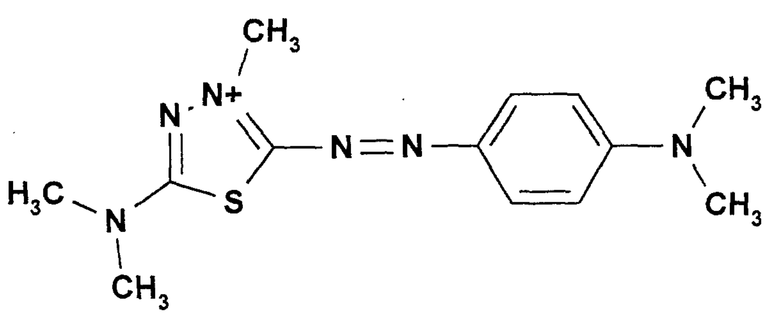 $CH_3SO_4^-$ (VII$_7$)

;

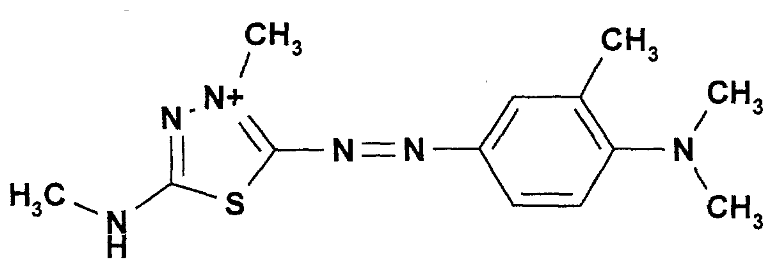 $CH_3SO_4^-$ (VII$_8$)

;

et

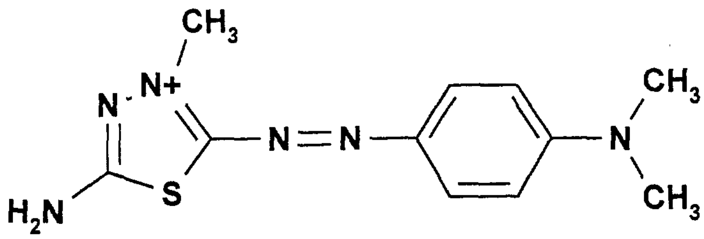

$CH_3SO_4^-$ (VII$_9$)

.

**17.** Composition selon la revendication 14, **caractérisée par le fait que** les colorants cationiques de formule (VIII) sont choisis parmi ceux de structures (VIII$_1$) à (VIII$_{18}$) suivantes :

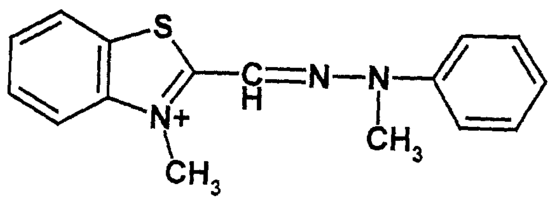

$Cl^-$ (VIII$_1$)

;

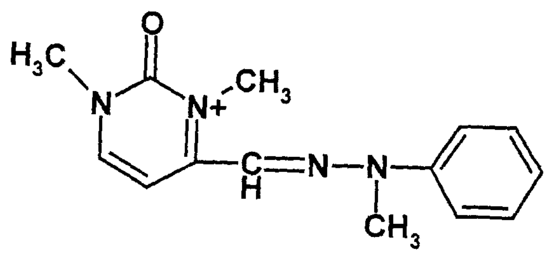

$Cl^-$ (VIII$_2$)

;

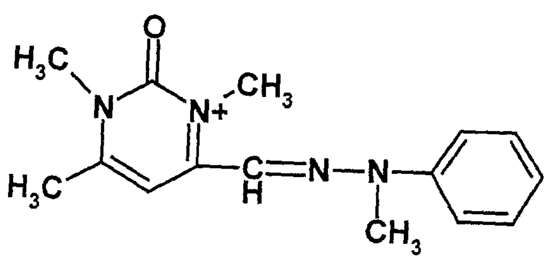

$Cl^-$ (VIII$_3$)

;

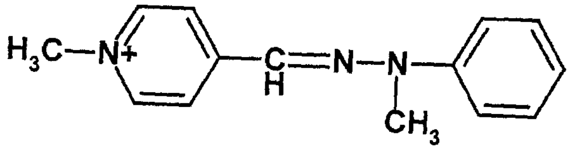

$CH_3SO_4^-$ (VIII$_4$)

;

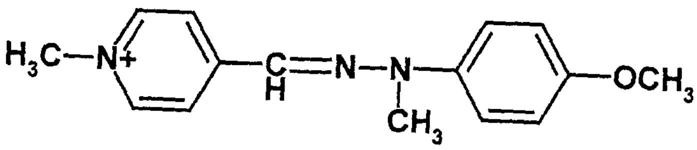

$Cl^-$ (VIII$_5$)

;

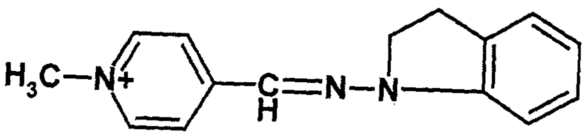

$CH_3SO_4^-$ (VIII$_6$)

;

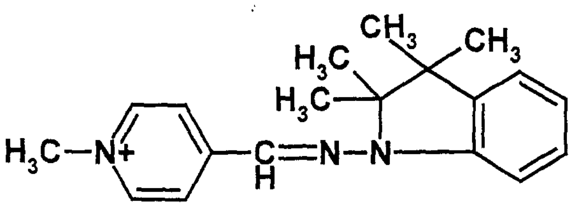

$CH_3SO_4^-$ (VIII$_7$)

;

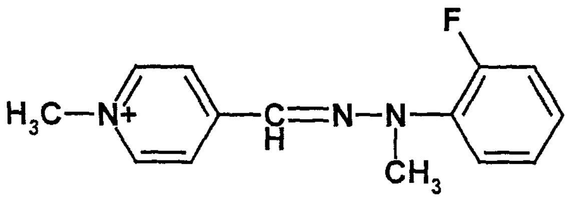

$Cl^-$ (VIII$_8$)

;

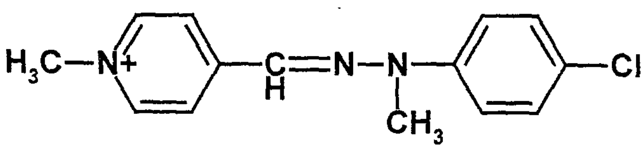

$Cl^-$ (VIII$_9$)

;

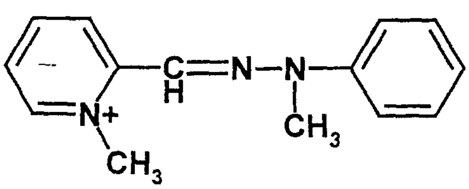

$CH_3SO_4^-$ (VIII$_{10}$)

;

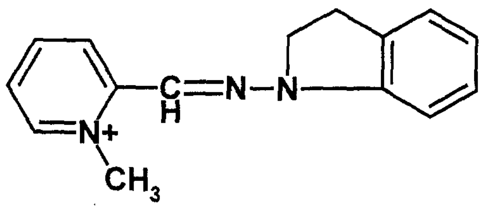

$CH_3SO_4^-$ (VIII$_{11}$)

;

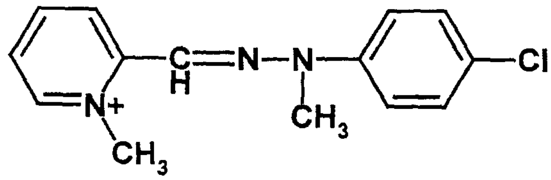

$CH_3SO_4^-$ (VIII$_{12}$)

;

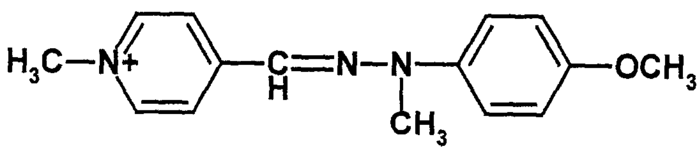

$CH_3SO_4^-$ (VIII$_{13}$)

;

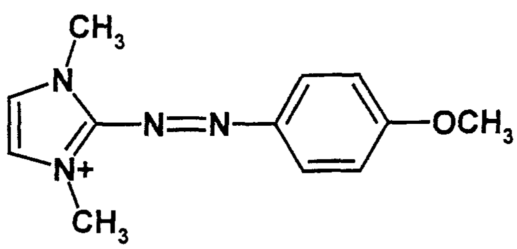

$Cl^-$ (VIII$_{14}$)

;

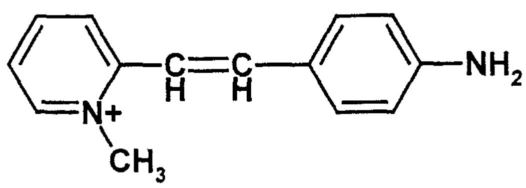

$CH_3COO^-$ (VIII$_{15}$)

;

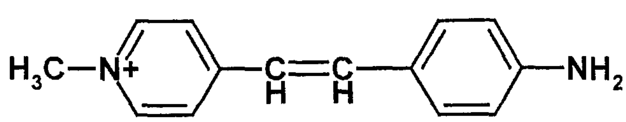

$CH_3COO^-$ (VIII$_{16}$)

;

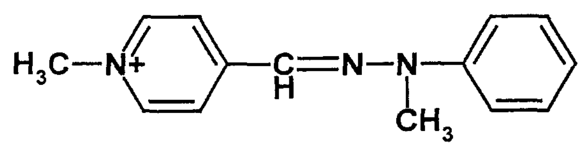 $Cl^-$ (VIII$_{17}$)

;

et

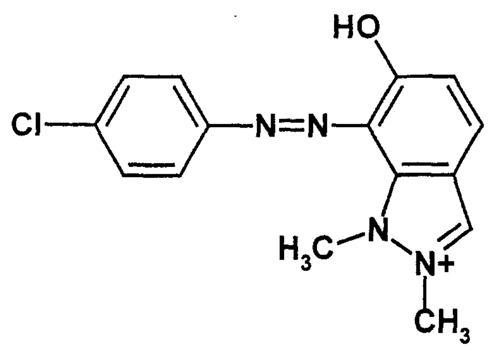 $Cl^-$ (VIII$_{18}$)

**18.** Composition selon la revendication 14, **caractérisée par le fait que** les colorants cationiques de formule (VIII') sont choisis parmi ceux de structures (VIII'$_1$) à (VIII'$_3$) suivantes :

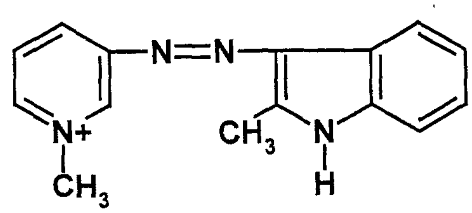 $Cl^-$ (VIII'$_1$)

;

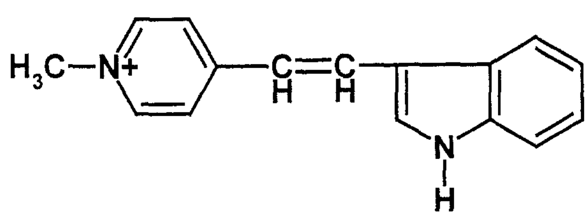 $Cl^-$ (VIII'$_2$)

;

et

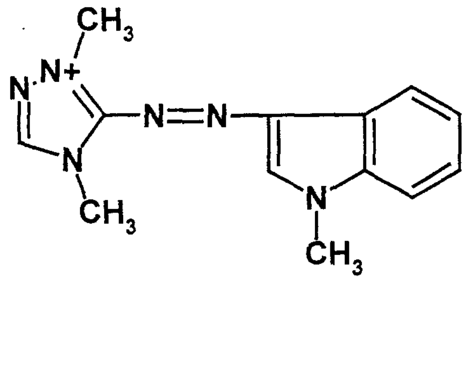 $Cl^-$ (VIII'$_3$)

.

**19.** Composition- selon la revendication 14, **caractérisée par le fait que** les colorants cationiques de formule (IX) sont choisis parmi ceux de structures ($IX_1$) à ($IX_{77}$) suivantes :

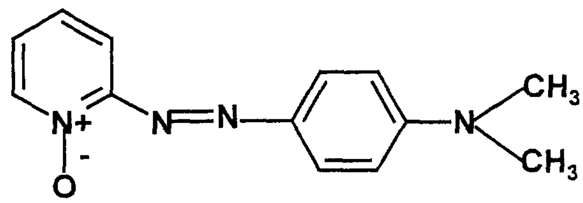

($IX_1$)

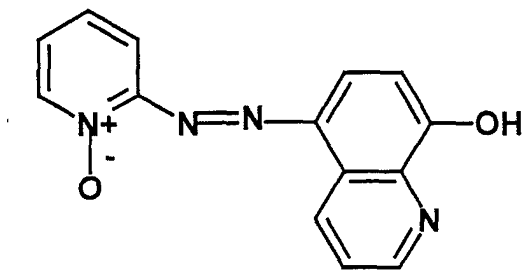

($IX_2$)

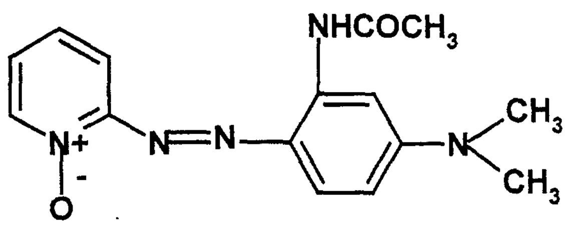

($IX_3$)

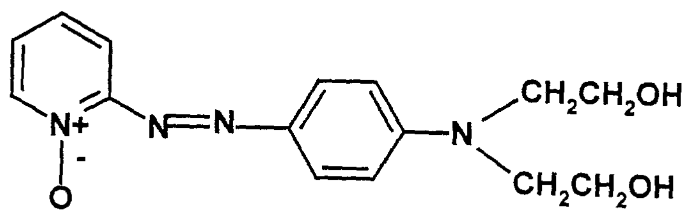

($IX_4$)

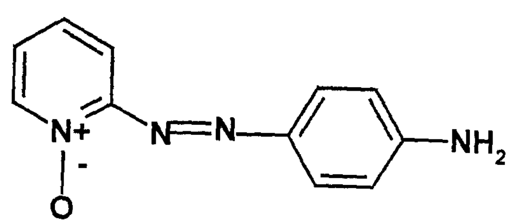

($IX_5$)

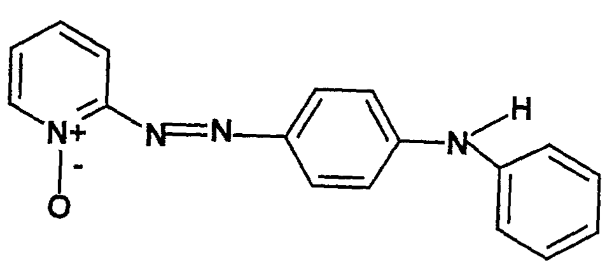

($IX_6$)

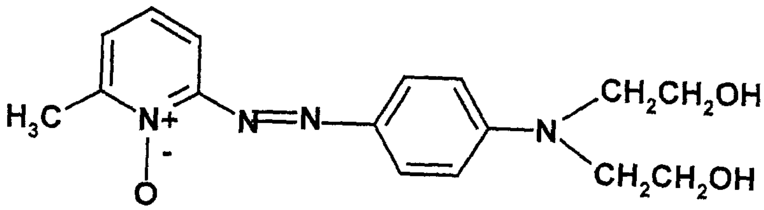

$(IX_7)$

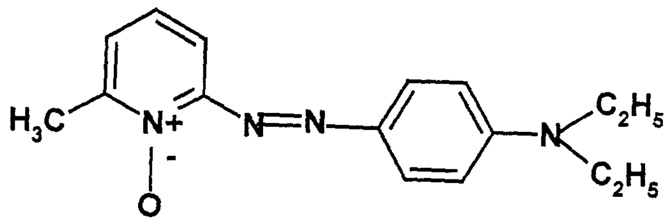

$(IX_8)$

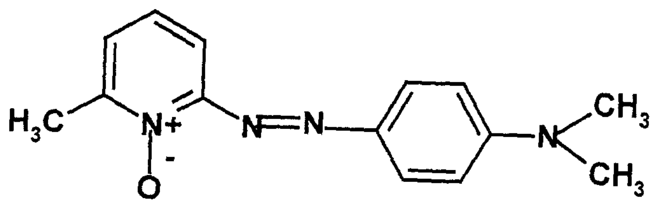

$(IX_9)$

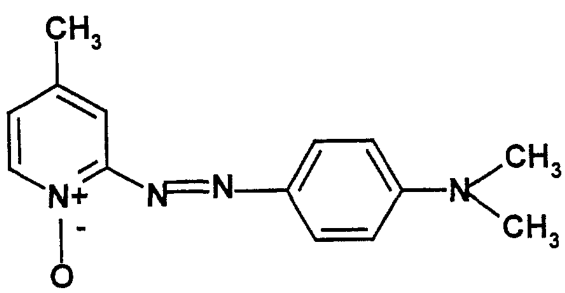

$(IX_{10})$

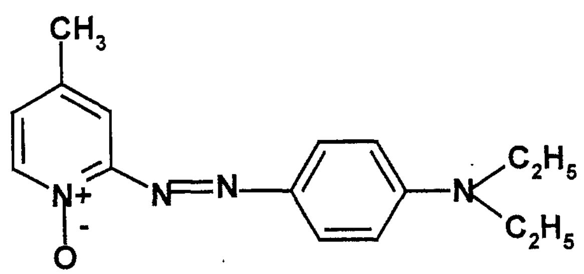

$(IX_{11})$

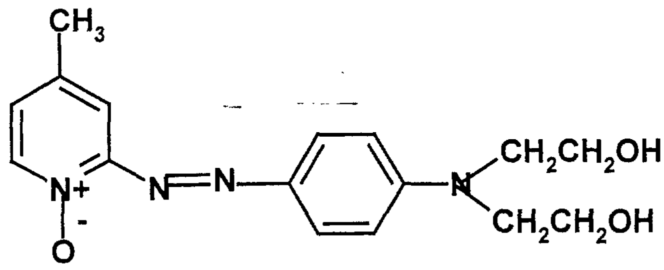

(IX$_{12}$)

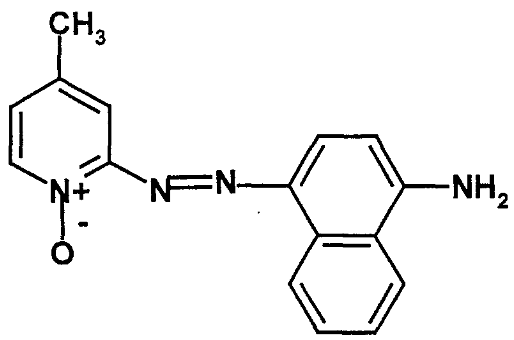

(IX$_{13}$)

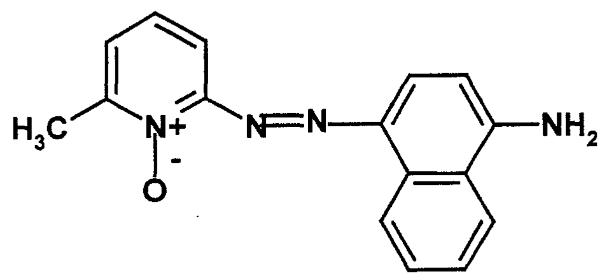

(IX$_{14}$)

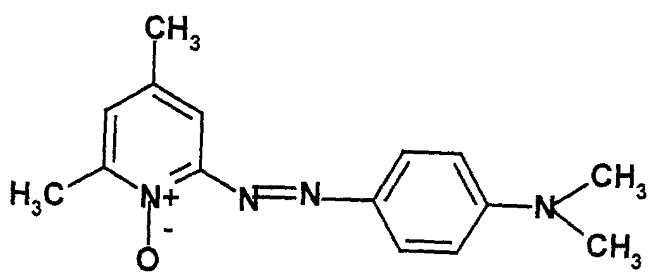

(IX$_{15}$)

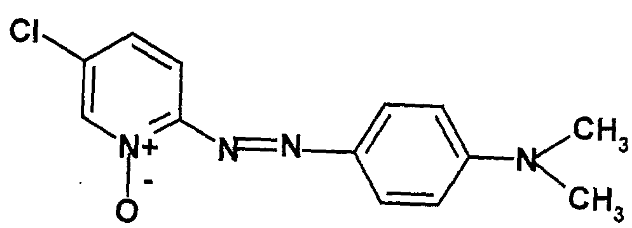

(IX$_{16}$)

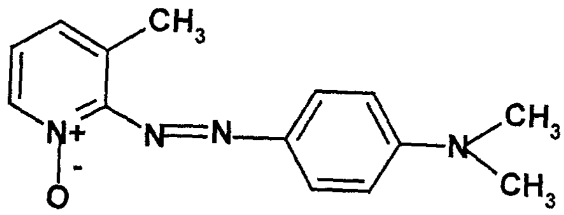

(IX17)

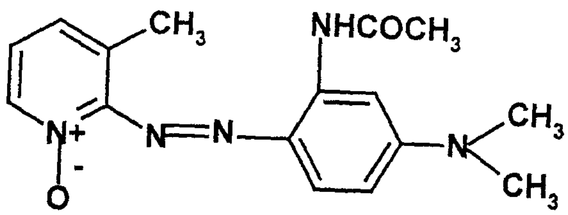

(IX18)

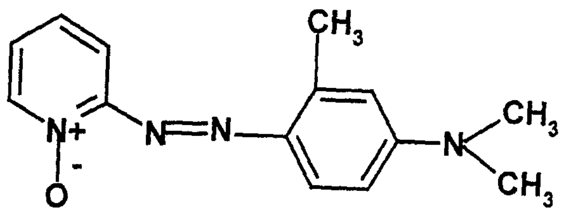

(IX19)

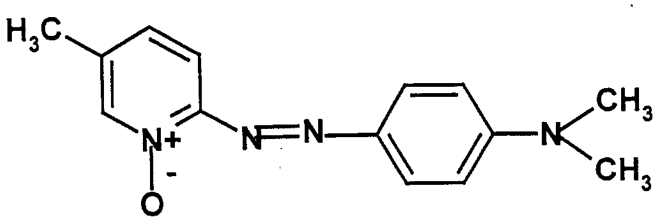

(IX20)

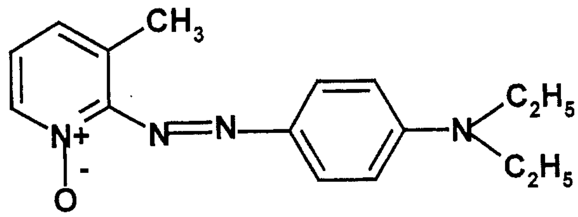

(IX21)

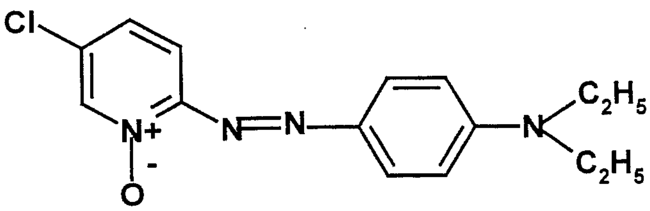

(IX22)

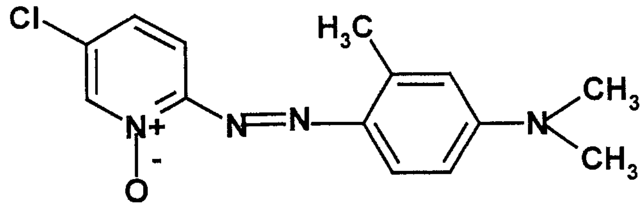

($IX_{23}$)

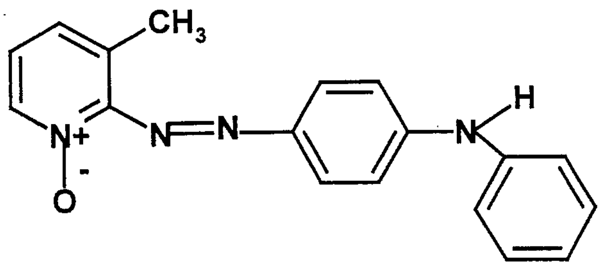

($IX_{24}$)

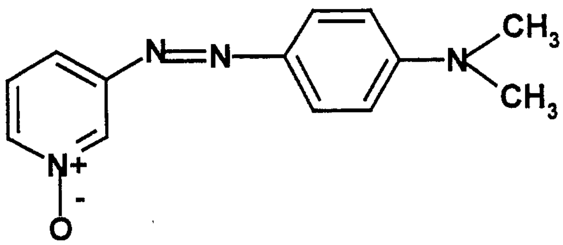

($IX_{25}$)

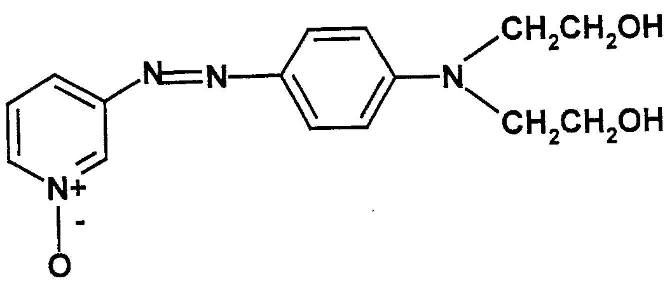

($IX_{26}$)

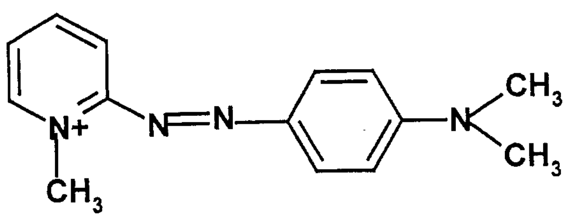

($IX_{27}$)

$CH_3SO_4^-$

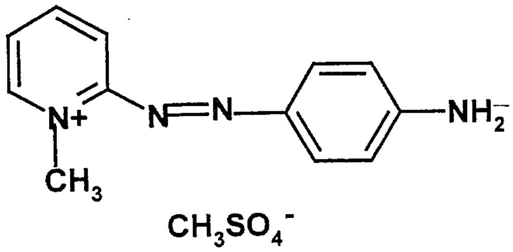

$CH_3SO_4^-$

(IX28)

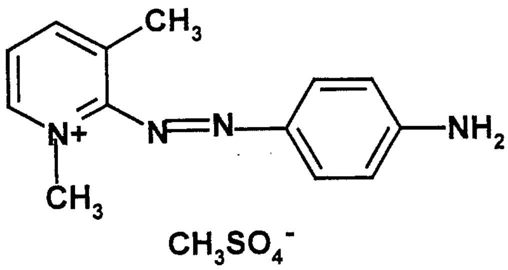

$CH_3SO_4^-$

(IX29)

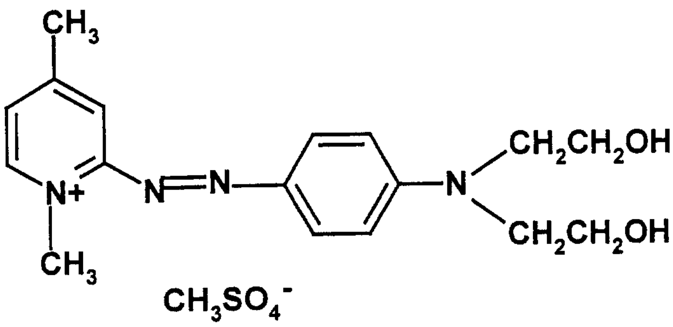

$CH_3SO_4^-$

(IX30)

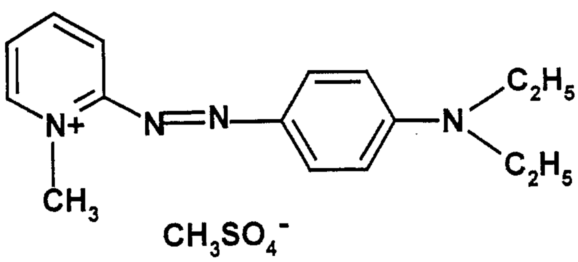

$CH_3SO_4^-$

(IX31)

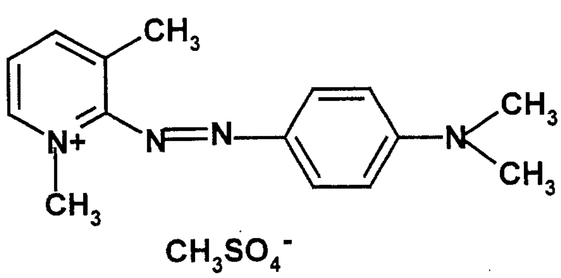

$CH_3SO_4^-$

(IX32)

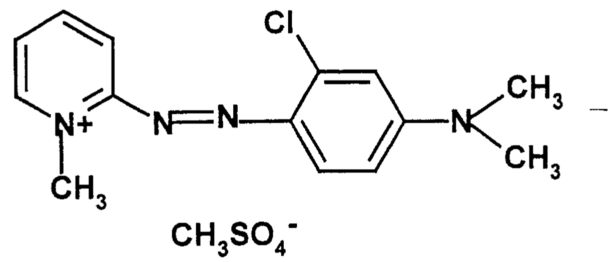

$(IX_{33})$

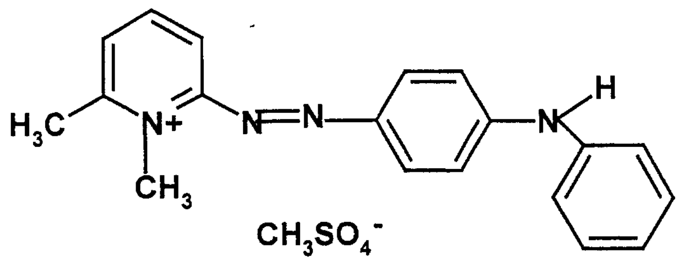

$(IX_{34})$

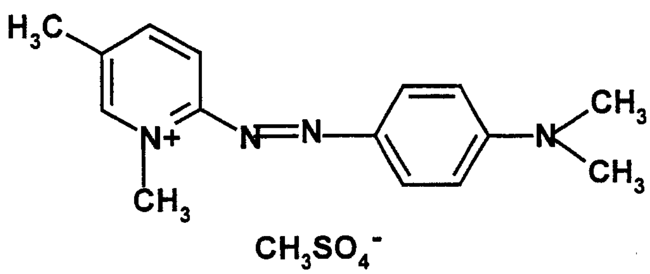

$(IX_{35})$

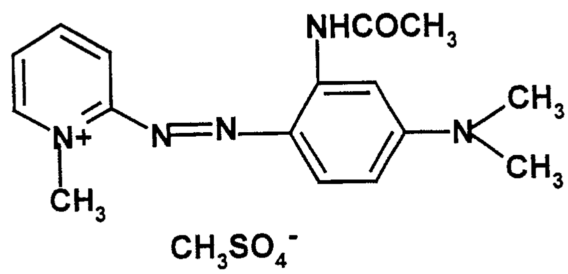

$(IX_{36})$

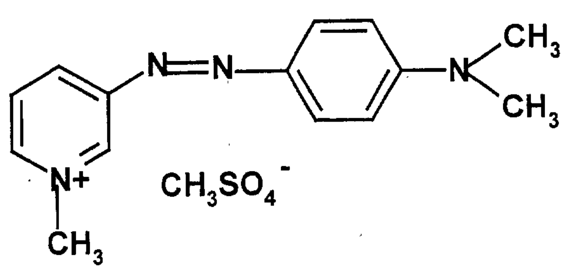

$(IX_{37})$

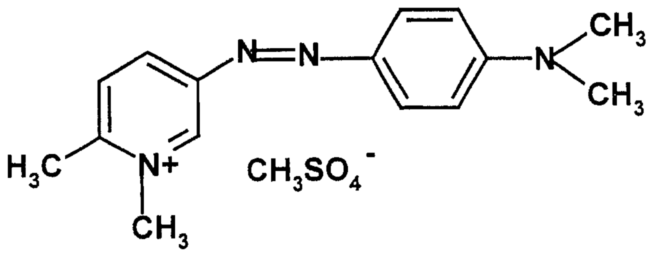

(IX$_{38}$)

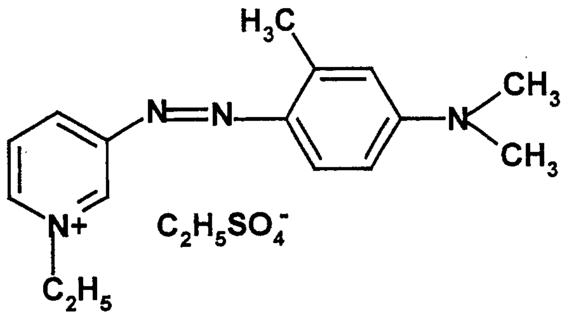

(IX$_{39}$)

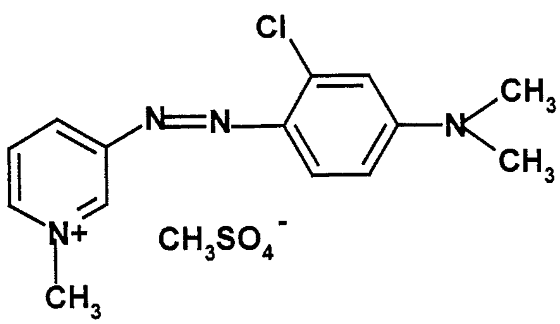

(IX$_{40}$)

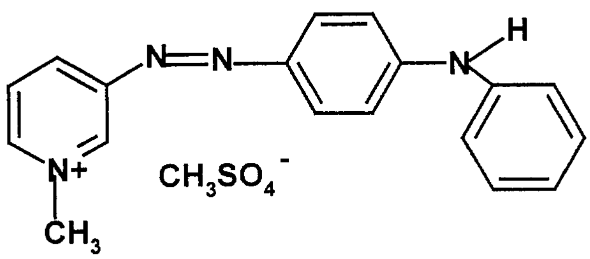

(IX$_{41}$)

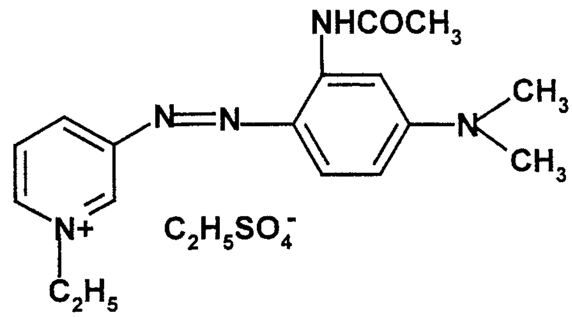

(IX$_{42}$)

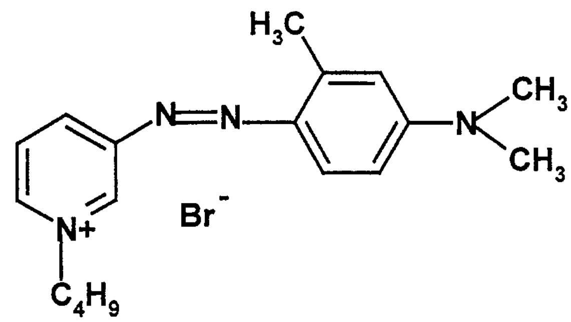

(IX$_{43}$)

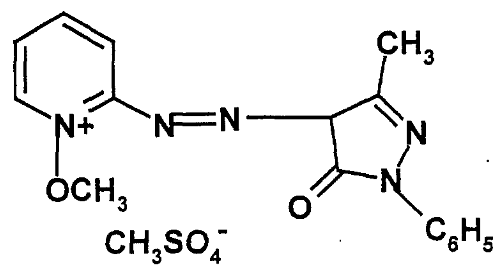

(IX$_{44}$)

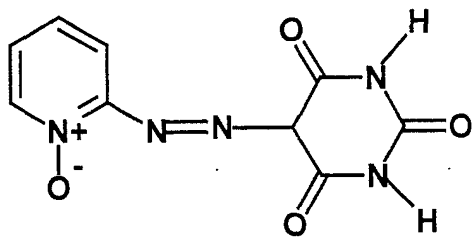

(IX$_{45}$)

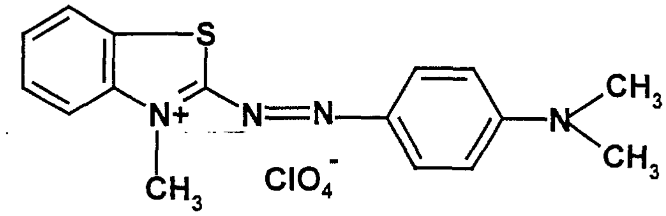

(IX$_{46}$)

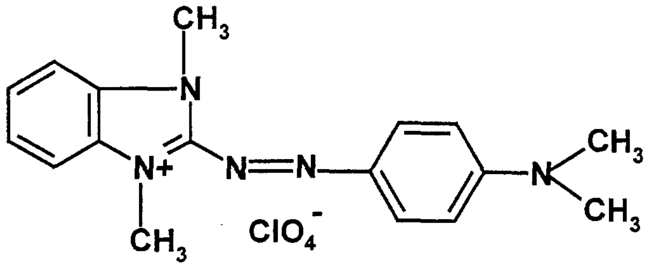

(IX$_{47}$)

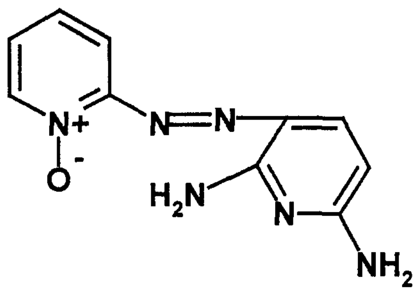

(IX$_{48}$)

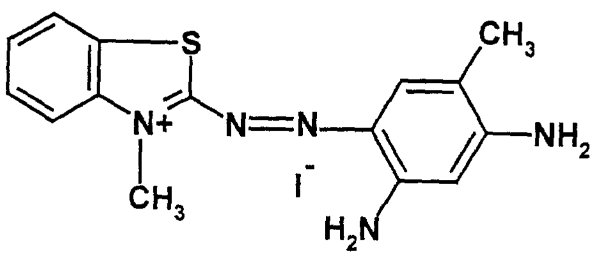

(IX$_{49}$)

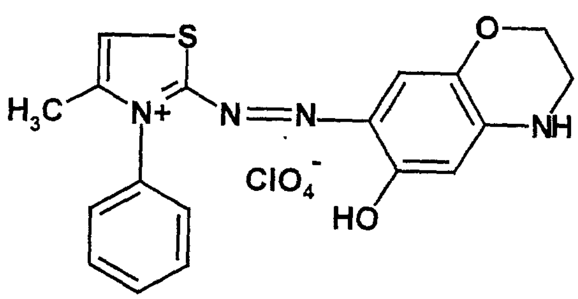

(IX$_{50}$)

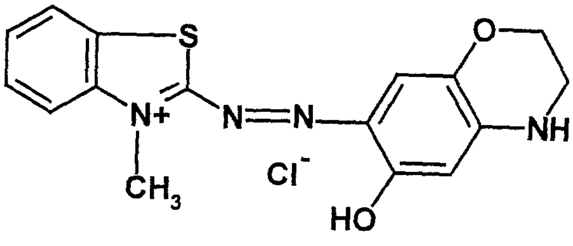

(IX$_{51}$)

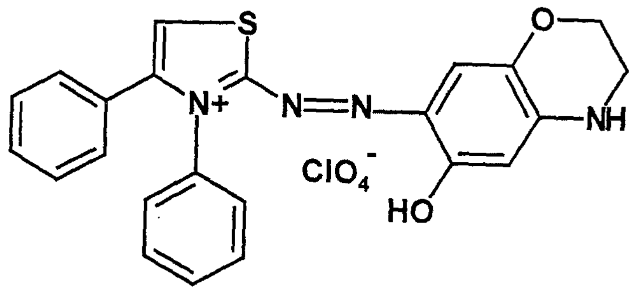

(IX52)

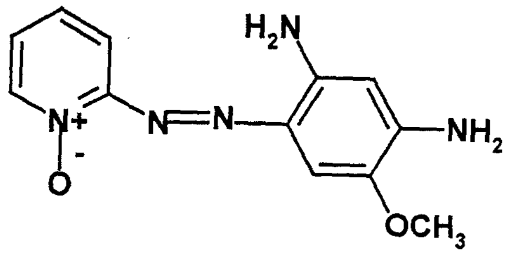

(IX53)

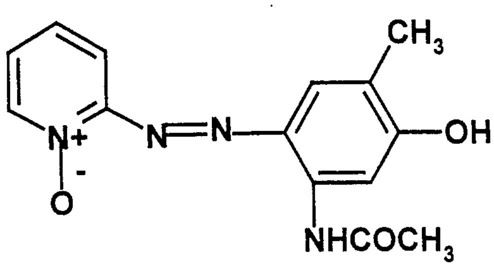

(IX54)

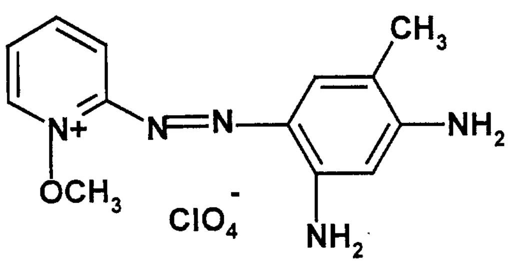

(IX55)

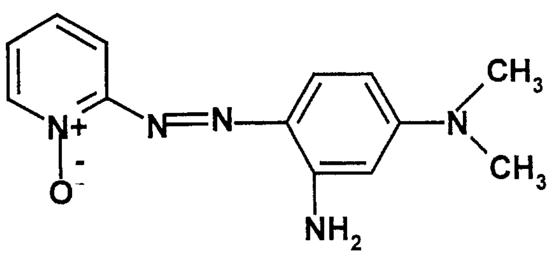

(IX56)

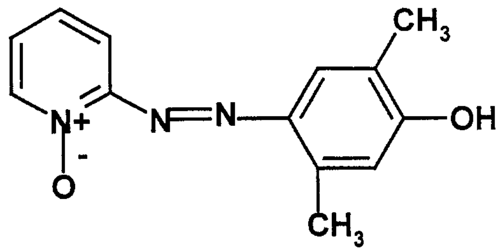

(IX$_{57}$)

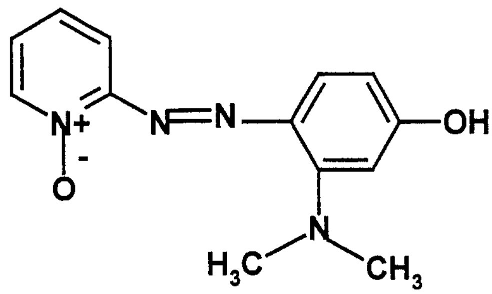

(IX$_{58}$)

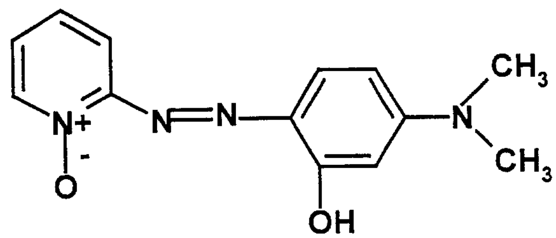

(IX$_{59}$)

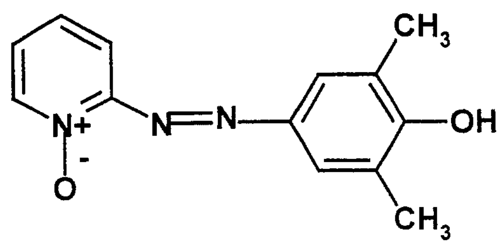

(IX$_{60}$)

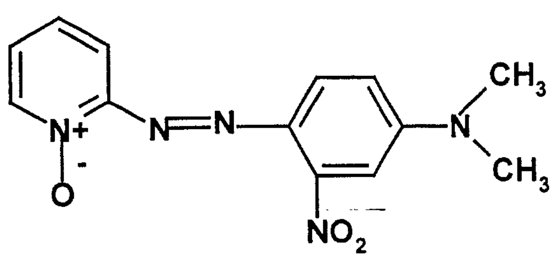

(IX$_{61}$)

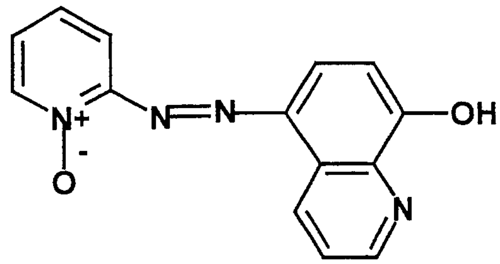

(IX62)

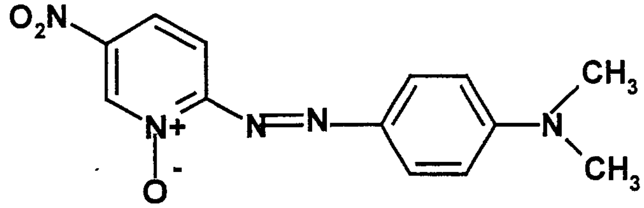

(IX63)

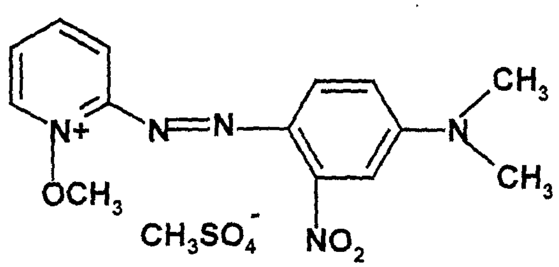

(IX64)

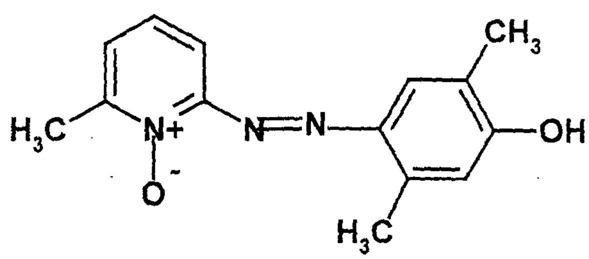

(IX65)

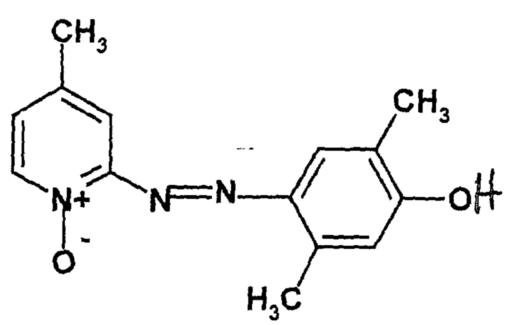

(IX66)

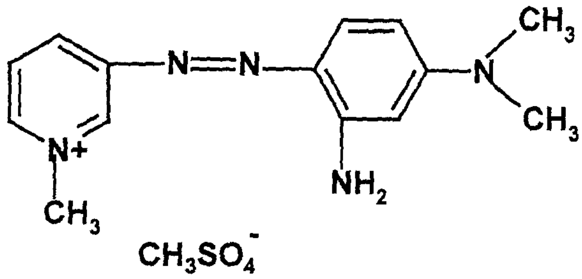

$CH_3SO_4^-$

(IX$_{67}$)

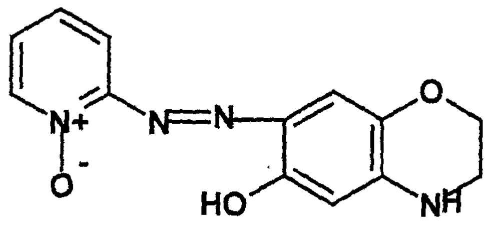

(IX$_{68}$)

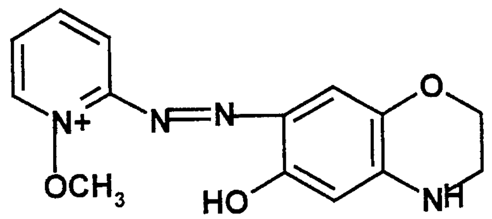

$CH_3SO_4^-$

(IX$_{69}$)

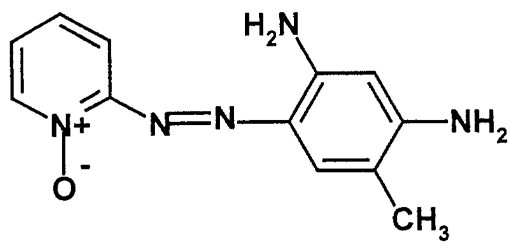

(IX$_{70}$)

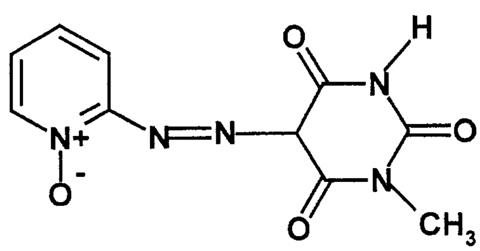

(IX$_{71}$)

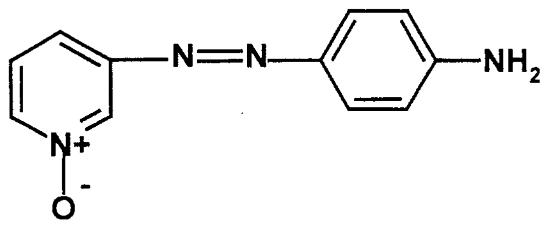

(IX72)

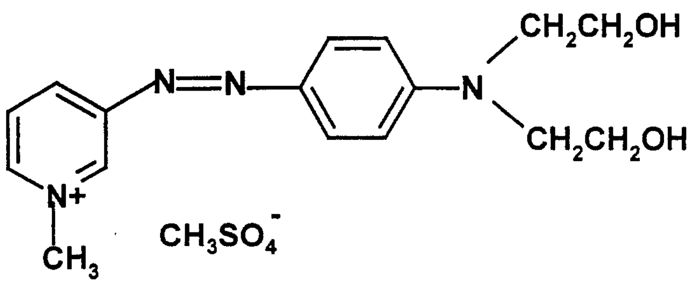

(IX73)

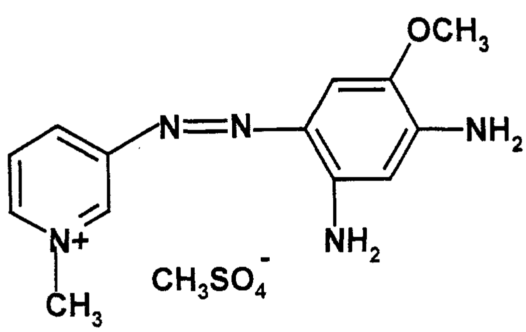

(IX74)

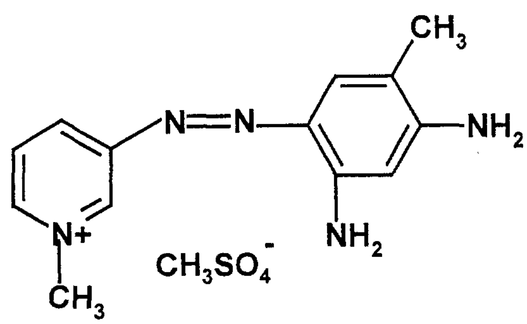

(IX75)

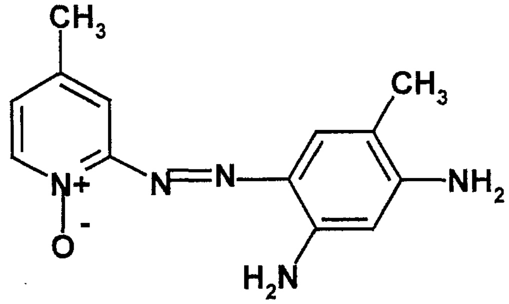

(IX76)

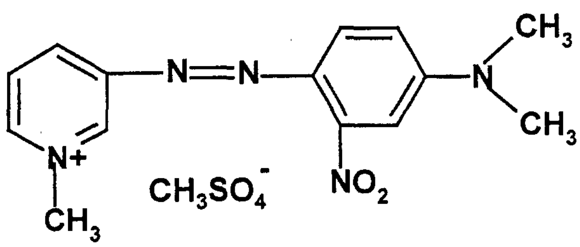

(IX77)

**20.** Composition selon l'une quelconque des revendications précédentes, **caractérisée par le fait que** le ou les colorants directs nitrés benzéniques et/ou le ou les colorants cationiques selon l'invention représentent de 0,001 à 20% en poids du poids total de la composition.

**21.** Composition selon la revendication 20, **caractérisée par le fait que** le ou lesdits colorants représentent de 0,005 à 10% en poids du poids total de la composition.

**22.** Composition selon l'une quelconque des revendications précédentes, **caractérisée par le fait qu'**elle contient au moins un coupleur.

**23.** Composition selon la revendication 22, **caractérisée par le fait que** le ou les coupleurs sont choisis parmi les métaphénylènediamines, les méta-aminophénols, les métadiphénols, les naphtols et les coupleurs hétérocycliques, et leurs sels d'addition avec un acide.

**24.** Composition selon les revendications 22 ou 23, **caractérisée par le fait que** le ou les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

**25.** Composition selon l'une quelconque des revendications 22 à 24, **caractérisée par le fait que** le ou les coupleurs sont présents à une concentration comprise entre 0,0001 et 15% en poids par rapport au poids total de la composition.

**26.** Composition selon l'une quelconque des revendications précédentes, **caractérisée par le fait qu'**elle contient au moins une base d'oxydation additionnelle.

**27.** Composition selon la revendication 26, **caractérisée par le fait que** la ou les bases d'oxydation additionnelles sont choisies parmi la paraphénylènediamine, la paratoluylènediamine, la 2-hydroxyéthyl-paraphénylènediamine, la 1-N,N-bis(2-hydroxyéthyl)-paraphénylènediamine, les para-aminophénols, les orthophénylènes diamines, les orthoaminophénols, les bases doubles, les bases hétérocycliques.

**28.** Composition selon les revendications 26 ou 27, **caractérisée par le fait que** la ou les bases d'oxydation additionnelles sont présentes à une concentration comprise entre 0,0001 et 15% en poids par rapport au poids total de la composition.

**29.** Composition selon l'une quelconque des revendications précédentes, **caractérisée par le fait que** le milieu approprié pour la teinture est constitué par de l'eau ou par un mélange d'eau et d'au moins un solvant organique.

**30.** Composition selon la revendication 29, **caractérisée par le fait que** les solvants sont présents dans des proportions comprises entre 1 et 40 % en poids par rapport au poids total de la composition.

**31.** Composition selon l'une quelconque des revendications précédentes, **caractérisée par le fait qu'**elle contient des séquestrants, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines, des opacifiants, des agents épaississants, des agents réducteurs ou antioxydants, des alcools gras, des agents alcalinisants ou acidifiants.

**32.** Composition selon l'une quelconque des revendications précédentes, **caractérisée par le fait qu'**elle contient au moins un tensioactif anionique, nonionique, cationique ou amphotère et de préférence non ionique dans une proportion allant de 0,1 à 20% en poids par rapport au poids total de la composition.

**33.** Composition selon l'une quelconque des revendications précédentes, **caractérisée par le fait qu'**elle contient au moins 0,01% en poids d'un polymère cationique par rapport au poids total de la composition.

**34.** Composition prête à l'emploi pour la teinture d'oxydation des fibres kératiniques en particulier des fibres kératiniques humaines telles que les cheveux **caractérisée par le fait qu'**elle est obtenue par mélange d'une composition telle que définie à l'une quelconque des revendications 1 à 33 et d'une composition contenant au moins un agent oxydant.

**35.** Composition selon la revendication 34, **caractérisée par le fait que** le pH est compris entre les valeurs 3 et 12 et de préférence compris entre 6 et 11.

**36.** Composition selon l'une quelconque des revendications précédentes, **caractérisée par le fait qu'**elle se présente sous forme de liquides, de poudres, de crèmes, de gels, éventuellement pressurisés, ou sous toute autre forme appropriée pour réaliser une teinture des fibres kératiniques, et notamment des cheveux humains.

**37.** Composition selon la revendication 34, **caractérisée par le fait que** l'agent oxydant est choisi parmi le peroxyde d'hydrogène, le peroxyde d'urée, les bromates ou ferricyanures de métaux alcalins, les persels tels que les perborates et les persulfates, les enzymes d'oxydoréduction telles que les laccases, les peroxydases et les oxydoréductases à 2 électrons, le cas échéant en présence de leur donneur ou cofacteur respectif.

**38.** Composition selon la revendication 37, **caractérisée par le fait que** l'agent oxydant est constitué par une solution d'eau oxygénée dont le titre varie de 1 à 40 volumes, et de préférence de 5 à 40 volumes.

**39.** Procédé de teinture d'oxydation des fibres kératiniques, et en particulier des fibres kératiniques humaines telles que les cheveux, consistant à appliquer sur les fibres une composition consistant à appliquer sur les fibres une composition colorante contenant, dans un milieu approprié pour la teinture, au moins une 1-(4-aminophényl)-pyrrolidine de formule (I) définie à l'une quelconque des revendications 1 à 8, en association avec au moins un colorant direct choisi parmi les colorants nitrés benzéniques et les colorants cationiques définis à l'une quelconque des revendications 1 et 9 à 19, la couleur étant révélée à pH alcalin, neutre ou acide, à l'aide d'une composition contenant au moins un agent oxydant, qui est mélangée juste au moment de l'emploi à la composition colorante ou qui est appliquée séquentiellement sans rinçage intermédiaire.

**40.** Procédé de teinture d'oxydation des fibres kératiniques, et en particulier des fibres kératiniques humaines telles que les cheveux, **caractérisée par le fait qu'**il consiste à appliquer la composition prête à l'emploi définie à la revendication 34, réalisée extemporanément au moment de l'emploi à partir de la composition selon l'invention et de la composition oxydante, sur les fibres kératiniques sèches ou humides, et à la laisser agir pendant un temps de pause variant, de préférence, de 1 à 60 minutes environ, et plus préférentiellement de 10 à 45 minutes environ,

à rincer les fibres, puis éventuellement à les laver au shampooing, puis à les rincer à nouveau, et à les sécher.

**41.** Dispositif à plusieurs compartiments ou "kit" pour la teinture d'oxydation des fibres kératiniques, en particulier des fibres kératiniques humaines telles que les cheveux, qui comporte au moins un compartiment contenant au moins une 1-(4-aminophényl)-pyrrolidine de formule (I) définie à l'une quelconque des revendications 1 à 8 et au moins un colorant direct choisi parmi les colorants nitrés benzéniques et les colorants cationiques définis à l'une quelconque des revendications 1 et 9 à 19, et au moins un autre compartiment contenant au moins un agent oxydant.

**42.** Dispositif à plusieurs compartiments ou "kit" pour la teinture d'oxydation des fibres kératiniques, en particulier des fibres kératiniques humaines telles que les cheveux, qui comporte au moins un compartiment contenant au moins une 1-(4-aminophényl)-pyrrolidine de formule (I) définie à l'une quelconque des revendications 1 à 8, au moins un compartiment contenant au moins un colorant direct choisi parmi les colorants nitrés benzéniques et les colorants cationiques définis à l'une quelconque des revendications 1, et 9 à 19, et au moins un autre compartiment contenant au moins un agent oxydant.

Office européen des brevets

# RAPPORT DE RECHERCHE EUROPEENNE

Numéro de la demande
EP 01 40 0879

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | EP 0 962 452 A (SQUIBB BRISTOL MYERS CO) 8 décembre 1999 (1999-12-08) * revendications 5,17,24 * | 1,3,39 | A61K7/13 |
| D,A | US 5 876 464 A (PAN YUH-GUO ET AL) 2 mars 1999 (1999-03-02) * revendications 1,8 * | 1,5,39 | |
| A | EP 0 970 687 A (OREAL) 12 janvier 2000 (2000-01-12) * revendications 1,27 * | 1,14,39 | |
| A | EP 0 920 856 A (OREAL) 9 juin 1999 (1999-06-09) * page 50, ligne 22; revendication 1 * | 1,39 | |
| A | US 5 067 967 A (AKRAM MUSTAFA ET AL) 26 novembre 1991 (1991-11-26) * revendications 4,6 * | 1,9 | |
| A | PATENT ABSTRACTS OF JAPAN vol. 1999, no. 11, 30 septembre 1999 (1999-09-30) & JP 11 158048 A (FUJI PHOTO FILM CO LTD), 15 juin 1999 (1999-06-15) * abrégé * | 1,39 | DOMAINES TECHNIQUES RECHERCHES (Int.Cl.7) A61K |
| D,A | EP 0 891 765 A (SQUIBB BRISTOL MYERS CO) 20 janvier 1999 (1999-01-20) * revendications 1,16,30 * | 1,2,39 | |
| A | DE 197 28 336 A (SCHWARZKOPF GMBH HANS) 8 janvier 1998 (1998-01-08) * revendication 1 * | 1 | |

-/--

Le présent rapport a été établi pour toutes les revendications

| Lieu de la recherche | Date d'achèvement de la recherche | Examinateur |
|---|---|---|
| LA HAYE | 4 septembre 2001 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 01 40 0879

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | WO 98 01106 A (BITTNER ANDREAS JOACHIM ;SCHWARZKOPF GMBH HANS (DE); KLEEN ASTRID) 15 janvier 1998 (1998-01-15)<br>* revendication 1 *<br>--- | 1 | |
| A | US 3 701 769 A (PLUE ARNOLD F ET AL) 31 octobre 1972 (1972-10-31)<br>* colonne 1; revendication 1 *<br>----- | 1 | |
| | | | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| LA HAYE | 4 septembre 2001 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 01 40 0879

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-09-2001

| Document brevet cité au rapport de recherche | Date de publication | Membre(s) de la famille de brevet(s) | Date de publication |
|---|---|---|---|
| EP 0962452 A | 08-12-1999 | US 5993491 A | 30-11-1999 |
| | | JP 11349564 A | 21-12-1999 |
| US 5876464 A | 02-03-1999 | EP 0937713 A | 25-08-1999 |
| | | JP 11269143 A | 05-10-1999 |
| EP 0970687 A | 12-01-2000 | FR 2780881 A | 14-01-2000 |
| | | AU 722556 B | 03-08-2000 |
| | | AU 3680699 A | 03-02-2000 |
| | | BR 9903124 A | 30-05-2000 |
| | | CN 1246330 A | 08-03-2000 |
| | | HU 9902334 A | 28-03-2000 |
| | | JP 2000063246 A | 29-02-2000 |
| EP 0920856 A | 09-06-1999 | AU 711808 B | 21-10-1999 |
| | | AU 9420998 A | 24-06-1999 |
| | | BR 9805515 A | 11-04-2000 |
| | | CN 1227097 A | 01-09-1999 |
| | | HU 9802817 A | 28-10-1999 |
| | | JP 11236322 A | 31-08-1999 |
| | | PL 330107 A | 07-06-1999 |
| | | US 6045591 A | 04-04-2000 |
| US 5067967 A | 26-11-1991 | DE 3917114 A | 29-11-1990 |
| | | FR 2647443 A | 30-11-1990 |
| | | GB 2232681 A,B | 19-12-1990 |
| | | JP 2061666 C | 10-06-1996 |
| | | JP 3011043 A | 18-01-1991 |
| | | JP 7091243 B | 04-10-1995 |
| | | US 5163970 A | 17-11-1992 |
| JP 11158048 A | 15-06-1999 | AUCUN | |
| EP 0891765 A | 20-01-1999 | US 5851237 A | 22-12-1998 |
| DE 19728336 A | 08-01-1998 | AUCUN | |
| WO 9801106 A | 15-01-1998 | DE 19728147 A | 18-06-1998 |
| | | EP 0912160 A | 06-05-1999 |
| | | JP 2000514073 T | 24-10-2000 |
| | | US 2001005914 A | 05-07-2001 |
| US 3701769 A | 31-10-1972 | AUCUN | |

EPO FORM P0460

Pour tout renseignement concernant cette annexe : voir Journal Officiel de l'Office européen des brevets, No.12/82